(19) Europäisches Patentamt
European Patent Office
Office européen des brevets

(11) EP 1 294 877 B1

(12) EUROPEAN PATENT SPECIFICATION

(45) Date of publication and mention of the grant of the patent:
**21.09.2005 Bulletin 2005/38**

(21) Application number: **01946702.6**

(22) Date of filing: **22.06.2001**

(51) Int Cl.7: **C12N 15/12**, C07K 14/47, C12N 5/10, C12Q 1/68, C12N 15/62, C07K 16/18, G01N 33/50, G01N 33/53, A01K 67/027 // A61P3/00

(86) International application number:
**PCT/US2001/020117**

(87) International publication number:
**WO 2001/098355 (27.12.2001 Gazette 2001/52)**

(54) **CGI-69 COMPOSITIONS AND METHODS OF USE**

KOMPOSITIONEN UND VERWENDUNGEN VON CGI-69

COMPOSITIONS CGI-69 ET PROCEDES D'UTILISATION

(84) Designated Contracting States:
**AT BE CH CY DE DK ES FI FR GB GR IE IT LI LU MC NL PT SE TR**

(30) Priority: **22.06.2000 US 213307 P**

(43) Date of publication of application:
**26.03.2003 Bulletin 2003/13**

(73) Proprietors:
- **GENENTECH, INC.**
  **South San Francisco, CA 94080-4990 (US)**
- **Curagen Corporation**
  **New Haven, CT 06511 (US)**

(72) Inventors:
- **LEWIN, David**
  **New Haven, CT 06511 (US)**
- **ADAMS, Sean, H.**
  **Belmont, CA 94002 (US)**
- **YU, Xing Xian**
  **San Mateo, CA 94403 (US)**

(74) Representative: **MacLean, Martin Robert et al**
**Mathys & Squire**
**120 Holborn**
**London EC1N 2SQ (GB)**

(56) References cited:
**WO-A-00/61614** **US-A- 6 132 973**

- **DATABASE EMBL [Online] EBI, Hinxton, UK; 1 June 2000 (2000-06-01) LAI ET AL.: "Homo sapiens CGI-69 protein mRNA, complete cds." Database accession no. AF151827 XP002201534 -& LAI CHUN-HUNG ET AL: "Identification of novel human genes evolutionarily conserved in Caenorhabditis elegans by comparative proteomics." GENOME RESEARCH, vol. 10, no. 5, May 2000 (2000-05), pages 703-713, XP002201533 ISSN: 1088-9051**
- **DATABASE EMBL [Online] EBI, Hinxton, UK; 16 December 1999 (1999-12-16) POUSTKA ET AL.: "Homo sapiens mRNA; cDNA DKFZp434C119 (from clone DKFZp434C119); complete" Database accession no. AL133584 XP002201535**
- **WALKER J E ET AL: "The mitochondrial transport protein superfamily" JOURNAL OF BIOENERGETICS AND BIOMEMBRANES, PLENUM PUBLISHING, NEW YORK, NY, US, vol. 25, no. 5, 1993, pages 435-446, XP002089542 ISSN: 0145-479X**
- **DAS K ET AL: "PREDOMINANT EXPRESSION OF THE MITOCHONDRIAL DICARBOXYLATE CARRIER IN WHITE ADIPOSE TISSUE" BIOCHEMICAL JOURNAL, PORTLAND PRESS, LONDON, GB, vol. 2, no. 344, 1 December 1999 (1999-12-01), pages 313-320, XP008003598 ISSN: 0264-6021**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 294 877 B1

- XING XIAN YU ET AL: "OVEREXPRESSION OF THE HUMAN 2-OXOGLUTARATE CARRIER LOWERS MITOCHONDRIAL MEMBRANE POTENTIAL IN HEK-293 CELLS: CONTRAST WITH THE UNIQUE COLD-INDUCED MITOCHONDRIAL CARRIER CGI-69" BIOCHEMICAL JOURNAL, THE BIOCHEMICAL SOCIETY, LONDON, GB, vol. 2, no. 353, 15 January 2001 (2001-01-15), pages 369-375, XP001059156 ISSN: 0264-6021

SEQUENCE LISTING

<110> LEWIN, DAVID
ADAMS, SEAN H.
YU, XING XIAN

<120> CGI-69 COMPOSITIONS AND METHODS OF USE

<130> 10716/67

<140> PCT/US 01/20117
<141> 22/06/2001

<150> 60/213,307
<151> 22/06/2000

<160> 18

<170> Patent In Ver. 2.1

<210> 1
<211> 1114
<212> DNA
<213> Homo sapiens

<400> 1

```
ctgaagcttc aagatggctg accaggaccc tgcgggcatc agccccctcc agcaaatggt 60
ggcctcaggc accggggctg tggttacctc tctcttcatg acacccctgg acgtggtgaa 120
ggttcgcctg cagtctcagc ggccctccat ggccagcgag ctgatgcctt cctccagact 180
gtggagcctc tcctatacca aattgccctc ctctctccaa tccacaggga agtgcctcct 240
gtattgcaat ggtgtcctgg agcctctgta cctgtgccca aatggtgccc gctgtgccac 300
ctggtttcaa gaccctaccc gcttcactgg caccatggat gccttcgtga agatcgtgag 360
gcacgagggc accaggaccc tctggagcgg cctcccggcc accctggtga tgactgtgcc 420
agctaccgcc atctacttca ctgcctatga ccaactgaag gccttcctgt gtggtcgagc 480
cctgacctct gacctctacg cacccatggt ggctggcgcg ctggcccgcc tgggcaccgt 540
gactgtgatc agccccctgg agcttatgcg gacaaagctg caggctcagc atgtgtcgta 600
ccgggagctg ggtgcctgtg ttcgaactgc agtggctcag ggtggctggc gctcactgtg 660
gctgggctgg ggccccactg cccttcgaga tgtgcccttc tcagccctgt actggttcaa 720
ctatgagctg gtgaagagct ggctcaatgg gctcaggccg aaggaccaga cttctgtggg 780
catgagcttt gtggctggtg gcatctcagg gacggtggct gcagtgctga ctctaccctt 840
tgacgtggta aagacccaac gccaggtcgc tctgggagcg atggaggctg tgagagtgaa 900
cccccctgcat gtggactcca cctggctgct gctgcggagg atccgggccg agtcgggcac 960
caagggactc tttgcaggct tccttcctcg gatcatcaag gctgccccct cctgtgccat 1020
catgatcagc acctatgagt tcggcaaaag cttcttccag aggctgaacc aggaccggct 1080
tctgggcggc tgaaaggggc aaggaggcaa ggac                            1114
```

<210> 2
<211> 1546
<212> DNA
<213> Homo sapiens

<400> 2

```
ggctaggtgc gctgcgagcg cgcggagcca cgagggcgga cggacgtaat gggcccgcct 60
ggccctgggc gccgcgccgc acgagcacca gcctagagcc aggactgaag cttcaagatg 120
gctgaccagg accctgcggg catcagcccc ctccagcaaa tggtggcctc aggcaccggg 180
gctgtggtta cctctctctt catgacaccc ctggacgtgg tgaaggttcg cctgcagtct 240
```

```
cagcggccct ccatggccag cgagctgatg ccttcctcca gactgtggag cctctcctat 300
accaaatgga agtgcctcct gtattgcaat ggtgtcctgg agcctctgta cctgtgccca 360
aatggtgccc gctgtgccac ctggtttcaa gaccctaccc gcttcactgg caccatggat 420
gccttcgtga agatcgtgag gcacgagggc accaggaccc tctggagcgg cctccccgcc 480
accctggtga tgactgtgcc agctaccgcc atctacttca ctgcctatga ccaactgaag 540
gccttcctgt gtggtcgagc cctgacctct gacctctacg cacccatggt ggctggcgcg 600
ctggcccgcc tgggcaccgt gactgtgatc agccccctgg agcttatgcg gacaaagctg 660
caggctcagc atgtgtcgta ccgggagctg ggtgcctgtg ttcgaactgc agtggctcag 720
ggtggctggc gctcactgtg gctgggctgg ggccccactg cccttcgaga tgtgcccttc 780
tcagccctgt actggttcaa ctatgagctg gtgaagagct ggctcaatgg gttcaggccg 840
aaggaccaga cttctgtggg catgagcttt gtggctggtg gcatctcagg gacggtggct 900
gcagtgctga ctctaccctt tgacgtggta aagacccaac gccaggtcgc tctgggagcg 960
atggaggctg tgagagtgaa cccccctgcat gtggactcca cctggctgct gctgcggagg 1020
atccgggccg agtcgggcac caagggactc tttgcaggct tccttcctcg gatcatcaag 1080
gctgccccct cctgtgccat catgatcagc acctatgagt tcggcaaaag cttcttccag 1140
aggctgaacc aggaccggct tctgggcggc tgaaaggggc aaggaggcaa ggaccccgtc 1200
tctcccacgg atggggagag ggcaggagga gacccagcca agtgcctttt cctcagcact 1260
gagggagggg gcttgtttcc cttccctccc ggcgacaagc tccagggcag ggctgtccct 1320
ctgggcggcc cagcacttcc tcagacacaa cttcttcctg ctgctccagt cgtggggatc 1380
atcacttacc caccccccaa gttcaagacc aaatcttcca gctgccccct tcgtgtttcc 1440
ctgtgtttgc tgtagctggg catgtctcca ggaaccaaga agccctcagc ctggtgtagt 1500
ctccctgacc cttgttaatt ccttaagtct aaagatgatg aacttc                1546
```

<210> 3
<211> 359
<212> PRT
<213> Homo sapiens

<400> 3

```
Met Ala Asp Gln Asp Pro Ala Gly Ile Ser Pro Leu Gln Gln Met Val
1               5                   10                  15

Ala Ser Gly Thr Gly Ala Val Val Thr Ser Leu Phe Met Thr Pro Leu
            20                  25                  30

Asp Val Val Lys Val Arg Leu Gln Ser Gln Arg Pro Ser Met Ala Ser
        35                  40                  45

Glu Leu Met Pro Ser Ser Arg Leu Trp Ser Leu Ser Tyr Thr Lys Leu
    50                  55                  60

Pro Ser Ser Leu Gln Ser Thr Gly Lys Cys Leu Leu Tyr Cys Asn Gly
65                  70                  75                  80

Val Leu Glu Pro Leu Tyr Leu Cys Pro Asn Gly Ala Arg Cys Ala Thr
                85                  90                  95

Trp Phe Gln Asp Pro Thr Arg Phe Thr Gly Thr Met Asp Ala Phe Val
            100                 105                 110

Lys Ile Val Arg His Glu Gly Thr Arg Thr Leu Trp Ser Gly Leu Pro
        115                 120                 125

Ala Thr Leu Val Met Thr Val Pro Ala Thr Ala Ile Tyr Phe Thr Ala
    130                 135                 140
```

Tyr Asp Gln Leu Lys Ala Phe Leu Cys Gly Arg Ala Leu Thr Ser Asp
145 150 155 160

Leu Tyr Ala Pro Met Val Ala Gly Ala Leu Ala Arg Leu Gly Thr Val
165 170 175

Thr Val Ile Ser Pro Leu Glu Leu Met Arg Thr Lys Leu Gln Ala Gln
180 185 190

His Val Ser Tyr Arg Glu Leu Gly Ala Cys Val Arg Thr Ala Val Ala
195 200 205

Gln Gly Gly Trp Arg Ser Leu Trp Leu Gly Trp Gly Pro Thr Ala Leu
210 215 220

Arg Asp Val Pro Phe Ser Ala Leu Tyr Trp Phe Asn Tyr Glu Leu Val
225 230 235 240

Lys Ser Trp Leu Asn Gly Leu Arg Pro Lys Asp Gln Thr Ser Val Gly
245 250 255

Met Ser Phe Val Ala Gly Gly Ile Ser Gly Thr Val Ala Ala Val Leu
260 265 270

Thr Leu Pro Phe Asp Val Val Lys Thr Gln Arg Gln Val Ala Leu Gly
275 280 285

Ala Met Glu Ala Val Arg Val Asn Pro Leu His Val Asp Ser Thr Trp
290 295 300

Leu Leu Leu Arg Arg Ile Arg Ala Glu Ser Gly Thr Lys Gly Leu Phe
305 310 315 320

Ala Gly Phe Leu Pro Arg Ile Ile Lys Ala Ala Pro Ser Cys Ala Ile
325 330 335

Met Ile Ser Thr Tyr Glu Phe Gly Lys Ser Phe Phe Gln Arg Leu Asn
340 345 350

Gln Asp Arg Leu Leu Gly Gly
355

<210> 4
<211> 351
<212> PRT
<213> Homo sapiens

<400> 4

```
Met Ala Asp Gln Asp Pro Ala Gly Ile Ser Pro Leu Gln Gln Met Val
 1               5                  10                  15

Ala Ser Gly Thr Gly Ala Val Val Thr Ser Leu Phe Met Thr Pro Leu
            20                  25                  30

Asp Val Val Lys Val Arg Leu Gln Ser Gln Arg Pro Ser Met Ala Ser
        35                  40                  45
```

Glu Leu Met Pro Ser Ser Arg Leu Trp Ser Leu Ser Tyr Thr Lys Trp
50 55 60

Lys Cys Leu Leu Tyr Cys Asn Gly Val Leu Glu Pro Leu Tyr Leu Cys
65 70 75 80

Pro Asn Gly Ala Arg Cys Ala Thr Trp Phe Gln Asp Pro Thr Arg Phe
85 90 95

Thr Gly Thr Met Asp Ala Phe Val Lys Ile Val Arg His Glu Gly Thr
100 105 110

Arg Thr Leu Trp Ser Gly Leu Pro Ala Thr Leu Val Met Thr Val Pro
115 120 125

Ala Thr Ala Ile Tyr Phe Thr Ala Tyr Asp Gln Leu Lys Ala Phe Leu
130 135 140

Cys Gly Arg Ala Leu Thr Ser Asp Leu Tyr Ala Pro Met Val Ala Gly
145 150 155 160

Ala Leu Ala Arg Leu Gly Thr Val Thr Val Ile Ser Pro Leu Glu Leu
165 170 175

Met Arg Thr Lys Leu Gln Ala Gln His Val Ser Tyr Arg Glu Leu Gly
180 185 190

Ala Cys Val Arg Thr Ala Val Ala Gln Gly Gly Trp Arg Ser Leu Trp
195 200 205

Leu Gly Trp Gly Pro Thr Ala Leu Arg Asp Val Pro Phe Ser Ala Leu
210 215 220

Tyr Trp Phe Asn Tyr Glu Leu Val Lys Ser Trp Leu Asn Gly Phe Arg
225 230 235 240

Pro Lys Asp Gln Thr Ser Val Gly Met Ser Phe Val Ala Gly Gly Ile
245 250 255

Ser Gly Thr Val Ala Ala Val Leu Thr Leu Pro Phe Asp Val Val Lys
260 265 270

Thr Gln Arg Gln Val Ala Leu Gly Ala Met Glu Ala Val Arg Val Asn
275 280 285

Pro Leu His Val Asp Ser Thr Trp Leu Leu Leu Arg Arg Ile Arg Ala
290 295 300

Glu Ser Gly Thr Lys Gly Leu Phe Ala Gly Phe Leu Pro Arg Ile Ile
305 310 315 320

Lys Ala Ala Pro Ser Cys Ala Ile Met Ile Ser Thr Tyr Glu Phe Gly
325 330 335

Lys Ser Phe Phe Gln Arg Leu Asn Gln Asp Arg Leu Leu Gly Gly
340 345 350

<210> 5

<211> 25
<212> DNA
<213> Artificial Sequence

<220>
<223> Description of Artificial Sequence: Primer

<400> 5

ctgaagcttc aagatggctg accag 25

<210> 6
<211> 25
<212> DNA
<213> Artificial Sequence

<220>
<223> Description of Artificial Sequence: Primer

<400> 6

gtccttgcct ccttgcccct ttcag 25

<210> 7
<211> 42
<212> DNA
<213> Artificial Sequence

<220>
<223> Description of Artificial Sequence: Primer

<400> 7

cttgtcatcg tcgtccttgt agtcgccgcc cagaagccgg tc 42

<210> 8
<211> 22
<212> DNA
<213> Artificial Sequence

<220>
<223> Description of Artificial Sequence: Primer

<400> 8

ccacctggtt tcaagaccct ac 22

<210> 9
<211> 23
<212> DNA
<213> Artificial Sequence

<220>
<223> Description of Artificial Sequence: Probe

<400> 9

cgcttcactg gcaccatgga tgc 23

<210> 10
<211> 20
<212> DNA
<213> Artificial Sequence

<220>
<223> Description of Artificial Sequence: Primer

<400> 10

tgcctcacga tcttcacgaa 20

<210> 11
<211> 19
<212> DNA
<213> Artificial Sequence

<220>
<223> Description of Artificial Sequence: Primer

<400> 11

agcgagctga tgccttcct 19

<210> 12
<211> 33
<212> DNA
<213> Artificial Sequence

<220>
<223> Description of Artificial Sequence: Probe

<400> 12

cagactgtgg agcttctcct ataccaaatt gcc 33

<210> 13
<211> 20
<212> DNA
<213> Artificial Sequence

<220>
<223> Description of Artificial Sequence: Primer

<400> 13

ccctgtggat tggagagagg 20

<210> 14

<211> 17
<212> DNA
<213> Artificial Sequence

<220>
<223> Description of Artificial Sequence: Primer

<400> 14

ctggctcctg cttcgca 17

<210> 15
<211> 21
<212> DNA
<213> Artificial Sequence

<220>
<223> Description of Artificial Sequence: Probe

<400> 15

tccgggctga atctggcacc a 21

<210> 16
<211> 20
<212> DNA
<213> Artificial Sequence

<220>
<223> Description of Artificial Sequence: Primer

<400> 16

ggaagcctgc aaagagtccc 20

<210> 17
<211> 8
<212> PRT
<213> Artificial Sequence

<220>
<223> Description of Artificial Sequence: FLAG tag

<400> 17

Asp Tyr Lys Asp Asp Asp Asp Lys
1               5

<210> 18
<211> 357
<212> PRT
<213> Mus sp.

<400> 18

Met Ile Arg Thr Leu Gly Ala Leu Ala Val Gln Gln Met Val Ala Ser
1 5 10 15

Gly Ala Gly Ala Val Val Thr Ser Leu Phe Met Thr Pro Leu Asp Val
20 25 30

```
Val Lys Val Arg Leu Gln Ser Gln Arg Pro Ser Ala Thr Ser Glu Leu
        35                  40                  45

Thr Thr Pro Ser Arg Phe Trp Ser Leu Ser Tyr Thr Lys Ser Ser Ser
    50                  55                  60

Ala Leu Gln Ser Pro Gly Lys Cys Leu Leu Tyr Cys Asn Gly Val Leu
65                  70                  75                  80

Glu Pro Leu Tyr Leu Cys Pro Asn Gly Thr Arg Cys Ala Thr Trp Phe
                85                  90                  95

Gln Asp Pro Thr Arg Phe Thr Gly Thr Leu Asp Ala Phe Val Lys Ile
            100                 105                 110

Val Arg His Glu Gly Thr Arg Thr Leu Trp Ser Gly Leu Pro Ala Thr
        115                 120                 125

Leu Val Met Thr Val Pro Ala Thr Ala Ile Tyr Phe Thr Ala Tyr Asp
    130                 135                 140

Gln Leu Lys Ala Phe Leu Cys Gly Gln Ser Leu Thr Ser Asp Leu Tyr
145                 150                 155                 160

Ala Pro Met Val Ala Gly Ala Leu Ala Arg Met Gly Thr Val Thr Val
                165                 170                 175

Val Ser Pro Leu Glu Leu Val Arg Thr Lys Leu Gln Ala Gln His Val
            180                 185                 190

Ser Tyr Arg Glu Leu Ala Ser Ser Val Gln Ala Ala Val Thr Gln Gly
        195                 200                 205

Gly Trp Arg Ser Leu Trp Leu Gly Trp Gly Pro Thr Ala Leu Arg Asp
    210                 215                 220

Val Pro Phe Ser Ala Leu Tyr Trp Phe Asn Tyr Glu Leu Val Lys Ser
225                 230                 235                 240

Trp Leu Ser Gly Leu Arg Pro Lys Asp Gln Thr Ser Val Gly Ile Ser
                245                 250                 255

Phe Val Ala Gly Gly Ile Ser Gly Met Val Ala Ala Thr Leu Thr Leu
            260                 265                 270

Pro Phe Asp Val Val Lys Thr Gln Arg Gln Met Ser Leu Gly Ala Val
        275                 280                 285

Glu Ala Val Arg Val Lys Pro Pro Arg Val Asp Ser Thr Trp Leu Leu
    290                 295                 300

Leu Arg Arg Ile Arg Ala Glu Ser Gly Thr Arg Gly Leu Phe Ala Gly
305                 310                 315                 320

Phe Leu Pro Arg Ile Ile Lys Ala Ala Pro Ser Cys Ala Ile Met Ile
                325                 330                 335
```

```
Ser Thr Tyr Glu Phe Gly Lys Ser Phe Phe Gln Arg Leu Asn Gln Glu
            340                 345                 350

Gln Pro Leu Gly Arg
        355
```

## Description

Metabolic diseases represent a serious public health concern worldwide. Obese and overweight individuals account for up to half of the population of the United States, and the incidence of obesity has risen at an alarming rate over the last decade. Compared to lean individuals, overweight persons are particularly susceptible to an array of disorders, including heart disease, high blood pressure, Type II diabetes/insulin resistance, stroke, and others (Must et al., 1999). The etiology of excess weight gain is complex and incompletely understood, but in general, approximately equal contribution of genetics and environmental/social factors occurs with respect to weight gain or loss (Bray, 1997; Hill and Peters, 1998).

In persons developing overweight or obese conditions, these factors underlie an imbalance between appetite/caloric intake and energy expenditure. Thus, novel strategies that improve energy balance through modulation of appetite and/or metabolic rate are useful to correct relevant diseases including obesity-related disorders such as insulin resistance/diabetes, circulatory system anomalies, *etc.* Strategies to thwart excessive adipose tissue growth and accretion may also help in the treatment of obesity, or in the treatment of other diseases associated with the presence of inappropriately high amounts of this tissue globally or locally (*i.e.*, lipomas, phaeochromocytomas, hibernomas). Innovative clinical treatments, including improving the circulating or tissue levels of triglycerides, cholesterol, glucose, insulin, leptin or other metabolically-relevant molecules, that help normalize one or more of the altered factors concomitant with metabolic derangement would also have tremendous value.

Increased risks of mortality and morbidity associated with perturbations of metabolism are not confined to the obese, overweight, or diabetic states, however. Cachexia, the loss of appetite, leading to fewer calories taken in compared to caloric requirements, is a feature of numerous disease states, including certain cancers, some viral infections (*i.e.,* acquired immunodeficiency syndrome, AIDS), or bacterial infections (*i.e.*, during some stages of sepsis). The clinical prognosis is poor for patients who drift into negative energy balance (Tisdale, 1997), and thus there is a need for new treatments that counteract cachexia. Furthermore, conditions in which energy expenditure is abnormally elevated can benefit from novel modalities that modulate metabolism. In severe bums, for instance, the metabolic rate can rise almost two-fold, making administration of appropriate nutrition a tremendous challenge (Goldstein and Elwyn, 1989; Kinney et al., 1970). Finally, poor outcomes from diseases characterized by excessive adipose tissue loss (*i.e.*, lipoatrophic disorders) are common.

Treatment of metabolic disease may be in part effected through the innovative use of certain molecules as drugs or as targets of pharmaceutical intervention. However, there is also a pressing need to discover molecules that may be used in creative diagnostic and/or predictive strategies associated with metabolic disease. For instance, alterations in the expression of certain genes and proteins may underlie or mark the progression of metabolic diseases such as obesity. Thus, analysis of the expression of certain genes and proteins in afflicted patients compared to a normal population will assist in unraveling the etiology of their disease, thereby helping in the design of effective therapeutic strategies. Normal patients may be screened for expression in cases in which expression is altered prior to the onset of disease, thus allowing for pre-emptive treatments that can limit metabolic or other disease progression. Furthermore, changes in the gene or protein sequences in certain populations may be associated with disease, hence illustrating the need to discover genes/proteins relevant to metabolic disorders and whose sequences lead to biological changes that predispose to metabolic disease, or are in fact predictive of the progression of disease. Finally, knowledge of such unique genes/proteins will enable tracking of the efficacy of therapeutic modalities designed to treat metabolic diseases. The same case may be made for the use of expression and sequence analysis to monitor or predict the outcome of other diseases associated with excessive fat accretion, or those associated with abnormal fat loss. Discovery of fat-specific genes and proteins are especially attractive in this regard.

The bulk of animal tissue oxygen consumption is driven by a finely-balanced system in which the rate of mitochondrial catabolism of fuels is regulated largely by the flow of electrons along the electron-transport chain. Concomitant pumping of protons outward across the mitochondrial inner membrane establishes a proton electrochemical gradient or protonmotive force ($\Delta p$), which drives ATP synthesis via inward flow of protons through $F_1F_0$ ATP synthase. Thus, fuel combustion, electron transport, proton flux, and ATP turnover are intimately coupled. However, a portion of the $\Delta p$ is dissipated as protons flow inward independent of ATP synthase, a phenomenon termed "proton leak" or "uncoupling." Fuel combustion and electron transport/outward proton pumping increase in response to dissipation of Ap; thus, innate mitochondrial proton leak may account for a significant amount of daily energy expenditure (estimated at between 20-40% of tissue metabolic rate)(Brand, et al., 1994; Rolfe, et al., 1999). Clarifying the molecular basis of proton leak is an active area of research, and holds promise in uncovering target pathways for pharmaceutical intervention to treat obesity and other diseases arising from perturbations of energy balance.

The first clue that specific proteins may underlie mammalian mitochondrial proton leak emerged from studies of brown adipose tissue (BAT), a specialized tissue in which a large proportion of mitochondrial oxygen consumption is uncoupled from ATP synthesis under conditions in which adaptational thermogenesis is triggered (i.e. cold-exposure in rodents)(Nichols, et al., 1984).

Mitochondrial carrier proteins (MCPs) are proteins localized to the mitochondrial membrane. MCPs facilitate the transport of molecules across the membrane, such as citrate, protons, and other ions. The level of activity of the MCPs can affect the level of animal tissue oxygen consumption. Forexample, transportation of protons inward across the membrane would decrease the proton electrochemical gradient. Although dependent on additional factors, this decrease would tend to have the effect of decreasing the net efficiency of ATP synthesis (fuel consumed/ATP yield). An increase in the activity of a MCP with this functionality would result in a further decrease in the net efficiency of ATP synthesis. In a similar fashion, a decrease in the activity of such a MCP would tend to result in an increase in $\Delta p$, and a corresponding increase in ATP synthesis. Additionally, an increase in the level of such a MCP within the mitochondria, for example due to an up-regulation of mRNA level, would also tend to decrease energetic efficiency.

Brown adipose tissue (BAT) is a specialized tissue in which a large proportion of mitochondrial oxygen consumption is uncoupled from ATP synthesis under conditions in which adaptational thermogenesis is triggered (i.e. cold-exposure in rodents) (Nichols, et al., 1984). The heat-generating futile cycling of the BAT mitochondrial proton circuit was found to be associated with a specific protein termed uncoupling protein (UCP, subsequently named UCP1) (Nichols, et al., 1984; Ricquier, et al., 1991). Uncoupling proteins (UCPs) are one class of MCPs. Despite confinement of UCP1 to BAT under most conditions, significant proton leak occurs in all tissues in which it has been measured (Rolfe, et al., 1994), leading to the possibility that UCPs are present body-wide and impact whole-animal metabolic rate. To date, four putative UCP homologs have been identified, with homolog-specific tissue expression patterns (see Adams, 2000).

J. Bioenergetics and Biomembranes, Vol.25, No.5, (1993) pages 435-446 describes the mitochondrial transport superfamily of proteins.

DATABASE EMBL (Online) Acc No. AF151827 (1 June 2000), and Genome Research, Vol.10, No.5, (May 2000), pages 703-713 describes the identification of novel human genes evolutionarily conserved in *C. elegans* by comparative proteomics.

WO 00/61614 describes novel uncoupling proteins, and diagnostic/therapeutic methods using these proteins, screening methods, and related agonists and antagonists. WO 00/61614 meets the conditions of Article 158(1) and (2) EPC and is therefore to be regarded as state of the art according to Articles 54(3) and (4) EPC but not for Article 54(2) EPC.

Biochemical Journal, Vol.2, No.353, (15 January 2001), pages 369-375 describes the lowering of mitochondrial membrane potential in HEK-293 cells caused by overexpression of the human 2-oxoglutarate carrier, and contrasts this with the cold-induced mitochondrial carrier CGI-69.

The present invention provides an isolated polynucleotide comprising:- (a) a nucleic acid sequence having at least 80% sequence identity to a sequence of SEQ ID NO:1, which nucleic acid sequence encodes a polypeptide, and (b) a nucleic acid sequence encoding a heterologous polypeptide expressed at the C-terminus of the polypeptide of (a); wherein said isolated polynucleotide encodes a polypeptide having uncoupling activity; or a complement of said polynucleotide.

The present invention also provides an isolated polypeptide comprising:- (a) an amino acid sequence having at least 80% sequence identity to a sequence of SEQ ID NO:3, and (b) a heterologous polypeptide fused to the carboxy-terminus of the amino acid sequence of (a); wherein the polypeptide has uncoupling activity.

The present invention further provides a transgenic non-human animal, comprising a transgene comprising:- (a) a nucleic acid sequence having at least 80% sequence identity to a sequence of SEQ ID NO:1, which nucleic acid sequence encodes a polypeptide, wherein the polypeptide has uncoupling activity; and (b) a nucleic acid sequence encoding a heterologous polypeptide expressed at the C-terminus of the polypeptide of (a).

The present invention yet further provides an *in vitro* method of measuring agonist or antagonist activity of a compound on an activity of a polypeptide of the invention, comprising: (a) contacting a composition activity of the polypeptide of the invention with the compound; and (b) determining a change in uncoupling activity.

The present invention also provides an *in vitro* method for detecting the presence or absence of CGI-$69_L$ in a biological sample, comprising: (a) contacting the biological sample with a CGI-$69_L$ primer or probe capable of detecting a polynucleotide encoding a polypeptide comprising an amino acid sequence having at least 98% sequence identity to SEQ ID NO:3, wherein said primer or probe is capable of detecting CGI-$69_L$ and is not capable of detecting CGI-69; and (b) comparing the presence or absence of the polynucleotide with a control biological sample, wherein a change in amount of polynucleotide is indicative of metabolic disease or risk of metabolic disease.

Using differential display mRNA expression analysis, a previously-uncharacterized gene was found to be upregulated 2-fold in brown adipose tissue (BAT) of mice exposed to cold (4°C) for 48hr. Contig and homology analysis revealed that the gene represents the murine ortholog to a public database sequence encoding a putative human protein (CGI-69). Isolation of CGI-69 cDNA from human liver revealed variants of CGI-69, including a previously undescribed nucleic acid encoding a "long version" of CGI-69 (CGI-$69_L$). CGI-69 and CGI-$69_L$ are shown herein to be localized in the mitochondrial membrane. Furthermore, a carboxy-terminal tagged CGI-69 is shown herein to act as an uncoupling protein (UCP).

In one embodiment, the invention provides an isolated nucleic acid encoding human CGI-69, a CGI-69 variant, a CGI-69 fusion protein, or fragments thereof.

In one aspect, the invention provides isolated polypeptides comprising CGI-69, a CGI-69 variant, a CGI-69 fusion protein, or fragments thereof.

In another aspect of the invention, isolated CGI-69 nucleic acids or polypeptides are used in in vitro methods to alter respiration in a cell containing such isolated nucleic acid or polypeptide.

In yet another aspect of the invention, cells expressing CGI-69 nucleic acids or polypeptides are used in in vitro methods of screening compounds for the ability to alter CGI-69 acitvity. Such compounds may be useful in altering respiration in a cell.

In another embodiment, CGI-69 nucleic acids or polypeptides are used for the manufacture of a medicament for altering the metabolism of a patient. Such methods may be useful for the manufacture of a medicament for treating metabolic disorders.

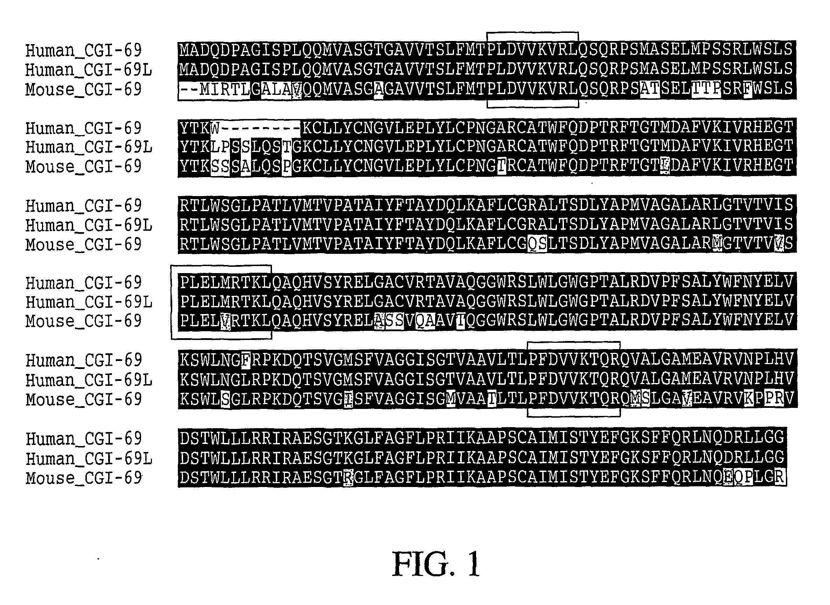

FIG. 1 depicts an alignment of human CGI-69 (SEQ ID NO:4), CGI-$69_L$(SEQ ID NO:3), and the mouse CGI-69 (SEQ ID NO:18). Boxes correspond to regions of mitochondrial energy transfer signature motifs found in UCP homologs that are reasonably preserved in CGI-69. Mitochodrial carrier domains (Pfam) in humanCGI-69 correspond to residues 10-48, 97-144, 152-276, and 298-340. Subsequent to the filing of the priority document, the sequence of human CGI-$69_L$ has been assigned GenBank accession numbers AF317711 (cDNA) and AF317711_1 (protein).

The present invention relates to a novel characterization of the putative protein CGI-69 as a mitochondrial carrier protein, the discovery of the novel splice variant, CGI-$69_L$, and the discovery of the alteration of the mitochondrial membrane potential ($\Delta\psi_m$) through the overexpression of the carboxy-FLAG-tagged CGI-69. Also discussed is the use of the protein, CGI-69 and CGI-69-encoding nucleic acids in diagnosing or treating metabolic diseases in humans. The term "CGI-69" includes the splice variant CGI-$69_L$. Novel evidence for an important function for CGI-69 in modifying the $\Delta\psi_m$ in BAT is presented. The white and brown adipose tissue of mammals are known to play a central role in energy storage and metabolic signaling, thus impacting appetite, metabolic rate, and energy balance.

The mouse ortholog to CGI-69 is up-regulated in mouse BAT upon exposure to cold ambient temperature ($T_a$). Based on this finding, the localization of CGI-69 to the mitochondrial membrane, and the domains shared with other mitochondrial proteins, it has now been discovered that CGI-69 is involved in regulating cellular metabolism. While not being bound by any particular theory, CGI-69 may be involved in cellular thermogenic uncoupling and, therefore, may be utilized to diagnose and treat specific perturbations in metabolic pathways underlying obesity and other metabolic disorders.

Altering the expression of *CGI-69* in a mammal, such as a human, through gene therapy is useful for treating metabolic diseases, such as obesity or cachexia, or for increasing or decreasing body weight. The term "metabolic disease" means a condition in which a disruption of normal energy homeostasis is a causative or exacerbating factor underlying disease. For example, metabolic disease includes obesity, wasting disorders such cachexia (associated with, for example, HIV infection, sepsis and trauma, and cancer), and diabetes (including Type n diabetes and insulin resistance).

Because of the metabolic role of CGI-69 in fat tissues and metabolism, compounds that have the property of increasing or decreasing CGI-69 activity are useful. This increase in activity may be produced in a variety of ways, for example: (1) by increasing or decreasing the copies of the gene in the cell (amplifiers and deamplifiers); (2) by increasing or decreasing transcription of the *CGI-69* gene (transcription up-regulators and down-regulators); (3) by increasing or decreasing the translation of *CGI-69* mRNA into protein (translation up-regulators and down-regulators); or (4) by increasing or decreasing the activity of CGI-69 itself (agonists and antagonists).

Compounds that are amplifiers and deamplifiers of *CGI-69* can be identified by contacting cells or organisms with the compound in vitro; and, then measuring the amount of DNA present that encodes CGI-69 (Ausubel et al., 1987). Compounds that are transcription up-regulators and down-regulators are identified by contacting cells or organisms with the compound in vitro, and then measuring the amount of mRNA produced that encodes CGI-69 (Ausubel et al., 1987). Compounds that are translation up-regulators and down-regulators are identified by contacting cells or organisms with the compound in vitro, and then measuring the amount of CGI-69 polypeptide produced (Ausubel et al., 1987).

The present invention also provides compounds that are amplifiers, transcription up-regulators, translation up-regulators or agonists, for manufacture of a medicament for combating metabolic diseases that can be ameliorated by increasing CGI-69 or CGI-69 activity, such as obesity or for decreasing weight gain. Conversely, the present invention provides compounds that are deamplifiers, transcription down-regulators, translation down-regulators or antagonists, for manufacture of a medicament for combating metabolic diseases that can be ameliorated by decreasing CGI-69 or CGI-69 activity, such as cachexia, or for increasing weight gain. Gene therapy is another way to up-regulate or down-regulate transcription and/or translation.

Both CGI-69 polypeptides and the polynucleotides can be used in clinical screens to test for metabolic disease etiology, or to assess the level of risk for these disorders. Tissue samples of a patient can be examined for the amount of CGI-69 polypeptide or *CGI-69* mRNA. When amounts significantly smaller or larger than normal are found, they are indicative of metabolic disease or risk of metabolic disease. A mutated *CGI-69* can yield altered activity, expression/protein levels, and a patient with such a mutation may have a metabolic disease or be at risk for a metabolic disease. Finally, determining the amount of expression of CGI-69 in a tissue sample from a mammal, or in a tissue culture, can be used to discover inducers or repressors of the gene.

High expression of *CGI-69* in mouse BAT indicates an important role for this protein in regulating adipose tissue physiological function and hence metabolic homeostasis. Agonists or antagonists that modulate the protein's activity are therefore useful for the manufacture of a medicament for treating metabolic disorders and disorders associated with changes in adipose tissue physiological function or mass. A determination of the abundance of *CGI-69* mRNA, CGI-69 protein or CGI-69 biological activity is useful to detect the progression of diseases characterized by perturbations in cellular metabolic function. The efficacy of drugs designed to modulate metabolism, may also be monitored by assessing CGI-69 mRNA/protein/activity levels in clinical samples, which would be expected to change relative to alterations in metabolism. Finally, detection of polymorphisms or mutations in the gene or protein in a patient sample can be used to predict: (1) the propensity of a person to develop metabolic disease, (2) disease progression rate/prognosis for said disorders, or (3) responses to therapeutic modalities designed to combat metabolic disease.

Determination of *CGI-69* mRNA, proteins or CGI-69 activity levels in clinical samples has predictive value for tracking progression of metabolic disorders or in cases in which therapeutic modalities are applied to correct said disorders. Such determinations of CGI-69 are useful in screening assays designed to discover drugs or other therapeutic modalites that modulate metabolic function.

Variation in the 3'UTR of gene transcripts can modify mRNA stability and hence translation. It is possible that patients suffering from metabolic disorders, or patients destined to have such afflictions, may display disease-specific patterns of variant expression. Thus, determination of the CGI-69 variant profile in clinical samples is of value in diagnosis or prediction of disease progression, or in assessment of the efficacy of therapeutic modalites to treat said disorders.

These embodiments are accomplished by known methods including those described below.

***Definitions***

Unless defined otherwise, all technical and scientific terms have the same meaning as is commonly understood by one of skill in the art to which this invention belongs. The definitions below are presented for clarity.

The recommendations of (Demerec et al., 1966) where these are relevant to genetics are adapted herein. To distinguish between genes (and related nucleic acids) and the proteins that they encode, the abbreviations for genes are indicated by *italicized* (or underlined) text while abbreviations for the proteins are not italicized. Thus, *CGI-69* or CGI-69 refers to the nucleotide sequence that encodes CGI-69.

"Isolated," when referred to a molecule, refers to a molecule that has been identified and separated and/or recovered from a component of its natural environment. Contaminant components of its natural environment are materials that interfere with diagnostic or therapeutic use.

1. *Nucleic acid-related definitions*

(a) *control sequences*

Control sequences are DNA sequences that enable the expression of an operably-linked coding sequence in a particular host organism. Prokaryotic control sequences include promoters, operator sequences, and ribosome binding sites. Eukaryotic cells utilize promoters, polyadenylation signals, and enhancers.

(b) *operably-linked*

Nucleic acid is operably-linked when it is placed into a functional relationship with another nucleic acid sequence. For example, a promoter or enhancer is operably-linked to a coding sequence if it affects the transcription of the sequence, or a ribosome-binding site is operably-linked to a coding sequence if positioned to facilitate translation. Generally, "operably-linked" means that the DNA sequences being linked are contiguous, and, in the case of a secretory leader, contiguous and in reading phase. However, enhancers do not have to be contiguous. Linking can be accomplished by conventional recombinant DNA methods.

(c) *isolated nucleic acids*

An isolated nucleic acid molecule is purified from the setting in which it is found in nature and is separated from at least one contaminant nucleic acid molecule. Isolated *CGI-69* molecules are distinguished from the specific *CGI-69* molecule, as it exists in cells. However, an isolated *CGI-69* molecule includes *CGI-69* molecules contained in cells that ordinarily express CGI-69 where, for example, the nucleic acid molecule is in a chromosomal location different from that of natural cells.

CGI-69 nucleic acids include that provided in Table 1 (CGI-69L) or a fragment thereof. The start and stop codons for the encoded protein is bolded and underlined.

**Table 1** *CGI-69$_L$* **nucleotide sequence (SEQ ID NO:1)**

| | | | | | | |
|---|---|---|---|---|---|---|
| CTGAAGCTTC | AAG**ATG**GCTG | ACCAGGACCC | TGCGGGCATC | AGCCCCCTCC | AGCAAATGGT | 60 |
| GGCCTCAGGC | ACCGGGGCTG | TGGTTACCTC | TCTCTTCATG | ACACCCCTGG | ACGTGGTGAA | 120 |
| GGTTCGCCTG | CAGTCTCAGC | GGCCCTCCAT | GGCCAGCGAG | CTGATGCCTT | CCTCCAGACT | 180 |
| GTGGAGCCTC | TCCTATACCA | AATTGCCCTC | CTCTCTCCAA | TCCACAGGGA | AGTGCCTCCT | 240 |
| GTATTGCAAT | GGTGTCCTGG | AGCCTCTGTA | CCTGTGCCCA | AATGGTGCCC | GCTGTGCCAC | 300 |
| CTGGTTTCAA | GACCCTACCC | GCTTCACTGG | CACCATGGAT | GCCTTCGTGA | AGATCGTGAG | 360 |
| GCACGAGGGC | ACCAGGACCC | TCTGGAGCGG | CCTCCCCGCC | ACCCTGGTGA | TGACTGTGCC | 420 |
| AGCTACCGCC | ATCTACTTCA | CTGCCTATGA | CCAACTGAAG | GCCTTCCTGT | GTGGTCGAGC | 480 |

CCTGACCTCT GACCTCTACG CACCCATGGT GGCTGGCGCG CTGGCCCGCC TGGGCACCGT
540

GACTGTGATC AGCCCCCTGG AGCTTATGCG GACAAAGCTG CAGGCTCAGC ATGTGTCGTA
600

CCGGGAGCTG GGTGCCTGTG TTCGAACTGC AGTGGCTCAG GGTGGCTGGC GCTCACTGTG
660

GCTGGGCTGG GGCCCCACTG CCCTTCGAGA TGTGCCCTTC TCAGCCCTGT ACTGGTTCAA
720

CTATGAGCTG GTGAAGAGCT GGCTCAATGG GCTCAGGCCG AAGGACCAGA CTTCTGTGGG
780

CATGAGCTTT GTGGCTGGTG GCATCTCAGG GACGGTGGCT GCAGTGCTGA CTCTACCCTT
840

TGACGTGGTA AAGACCCAAC GCCAGGTCGC TCTGGGAGCG ATGGAGGCTG TGAGAGTGAA
900

CCCCCTGCAT GTGGACTCCA CCTGGCTGCT GCTGCGGAGG ATCCGGGCCG AGTCGGGCAC
960

CAAGGGACTC TTTGCAGGCT TCCTTCCTCG GATCATCAAG GCTGCCCCCT CCTGTGCCAT
1020

CATGATCAGC ACCTATGAGT TCGGCAAAAG CTTCTTCCAG AGGCTGAACC AGGACCGGCT
1080

TCTGGGCGGC TGAAAGGGGC AAGGAGGCAA GGAC
1114

2. *Protein-related definitions*

(a) *purified polypeptide*

When the molecule is a purified polypeptide, the polypeptide will be purified (1) to obtain at least 15 residues of N-terminal or internal amino acid sequence using a sequenator, or (2) to homogeneity by SDS-PAGE under non-reducing or reducing conditions using Coomassie blue or silver stain. Isolated polypeptides include those expressed heterologously in genetically-engineered cells or expressed *in vitro,* since at least one component of the CGI-69 natural environment will not be present. Ordinarily, isolated polypeptides are prepared by at least one purification step.

(b) *active polypeptide*

An active CGI-69 fragment retains a biological activity of native or naturally-occurring CGI-69. Biological activity or refers to a function, either inhibitory or stimulatory, caused by a native CGI-69. A biological activity of CGI-69 includes, for example, mitochondrial localization.

CGI-69 polypeptides include the amino acid whose sequence is provided in Table 3 (CGI-$69_L$) or a fragment thereof.

A polypeptide encoded by SEQ ID NO:1 is presented in Table 3.

**Table 3** CGI-69$_L$ polypeptide sequence (SEQ ID NO:3)

```
MADQDPAGISPLQQMVASGTGAVVTSLFMTPLDVVKVRLQSQRPSMASELMPSSR
LWSLSYTKLPSSLQSTGKCLLYCNGVLEPLYLCPNGARCATWFQDPTRFTGTMDA
FVKIVRHEGTRTLWSGLPATLVMTVPATAIYFTAYDQLKAFLCGRALTSDLYAPM
VAGALARLGTVTVISPLELMRTKLQAQHVSYRELGACVRTAVAQGGWRSLWLGWG
PTALRDVPFSALYWFNYELVKSWLNGLRPKDQTSVGMSFVAGGISGTVAAVLTLP
FDVVKTQRQVALGAMEAVRVNPLHVDSTWLLLRRIRAESGTKGLFAGFLPRIIKA
```

```
APSCAIMISTYEFGKSFFQRLNQDRLLGG
```

1. *probes*

Probes are nucleic acid sequences of variable length, preferably between at least about 10 nucleotides (nt), 100 nt, or many (*e.g.*, 6,000 nt) depending on the specific use. Probes are used to detect identical, similar, or complementary nucleic acid sequences. Longer length probes can be obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies. Probes are substantially purified oligonucleotides that will hybridize under stringent conditions to at least optimally 12, 25, 50, 100, 150, 200, 250, 300, 350 or 400 consecutive sense strand nucleotide sequence of SEQ ID NO:1, or an anti-sense strand nucleotide sequence of SEQ ID NO:1; or of a naturally occurring mutant of SEQ ID NO:1.

The full- or partial length native sequence *CGI-69* may be used to "pull out" similar (homologous) sequences (Ausubel et al.,1987; Sambrook, 1989), such as: (1) full-length or fragments of CGI-69 cDNA from a cDNA library from any species *(e.g.* human, murine, feline, canine, bacterial, viral, retroviral, yeast), (2) from cells or tissues, (3) variants within a species, and (4) homologues and variants from other species. To find related sequences that may encode related genes, the probe may be designed to encode unique sequences or degenerate sequences. Sequences may also be genomic sequences including promoters, enhancer elements and introns of native sequence *CGI-69*.

For example, a *CGI-69* coding region in another species may be isolated using such probes. A probe of about 40 bases is designed, based on *CGI-69*, and made. To detect hybridizations, probes are labeled using, for example, radionucleotides such as $^{32}P$ or $^{35}S$, or enzymatic labels such as alkaline phosphatase coupled to the probe via avidin-biotin systems. Labeled probes are used to detect nucleic acids having a complementary sequence to that of *CGI-69* in libraries of cDNA, genomic DNA or mRNA of a desired species.

Such probes can be used as a part of a diagnostic test kit for identifying cells or tissues which mis-express a CGI-69, such as by measuring a level of CGI-69 in a sample of cells from a subject *e.g.,* detecting *CGI-69* mRNA levels or determining whether a genomic *CGI-69* has been mutated or deleted.

2. *isolated nucleic acid*

An isolated nucleic acid molecule is separated from other nucleic acid molecules that are present in the natural source of the nucleic acid. Preferably, an isolated nucleic acid is free of sequences that naturally flank the nucleic acid (*i.e.*, sequences located at the 5'- and 3'-termini of the nucleic acid) in the genomic DNA of the organism from which the nucleic acid is derived. For example, in various embodiments, isolated *CGI-69* molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived *(e.g.,* brain, heart, liver, spleen, *etc.*). Moreover, an isolated nucleic acid molecule, such as a cDNA molecule, can be substantially free of other cellular material or culture medium when produced by recombinant techniques, or of chemical precursors or other chemicals when chemically synthesized.

A nucleic acid molecule of the CGI-69, *e.g.,* a nucleic acid molecule having the nucleotide sequence of SEQ ID NO:1, or a complement, can be isolated using standard molecular biology techniques and the provided sequence information. Using all or a portion of the nucleic acid sequence of SEQ ID NO:1 as a hybridization probe, *CGI-69* molecules can be isolated using standard hybridization and cloning techniques (Ausubel et al., 1987; Sambrook, 1989).

PCR amplification techniques can be used to amplify *CGI-69* using cDNA, mRNA or alternatively, genomic DNA, as a template and appropriate oligonucleotide primers. Such nucleic acids can be cloned into an appropriate vector and characterized by DNA sequence analysis. Furthermore, oligonucleotides corresponding to *CGI-69* sequences can be prepared by standard synthetic techniques, *e.g.,* an automated DNA synthesizer.

3. *oligonucleotide*

An oligonucleotide comprises a series of linked nucleotide residues, which oligonucleotide has a sufficient number of nucleotide bases to be used in a PCR reaction or other application. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides of SEQ ID NO:1, or a complement thereof. Oligonucleotides may be chemically synthesized and may also be used as probes.

*4. complementary nucleic acid sequences; binding*

In another embodiment, an isolated nucleic acid molecule of *CGI-69* comprises a nucleic acid molecule that is a complement of the nucleotide sequence shown in SEQ ID NO:1, or a portion of this nucleotide sequence *(e.g.,* a fragment that can be used as a probe or primer or a fragment encoding a biologically-active portion of a CGI-69). A nucleic acid molecule that is complementary to the nucleotide sequence shown in SEQ ID NO:1, is one that is sufficiently complementary to the nucleotide sequence shown in SEQ ID NO:1, that it can hydrogen bond with little or no mismatches to the nucleotide sequence shown in SEQ ID NO:1, thereby forming a stable duplex.

"Complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

*5. Fragments*

Nucleic acid fragments are at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full-length sequence. Of course, any length in between is contemplated. Fragments may be derived from any contiguous portion of a nucleic acid or amino acid sequence of choice.

6. *derivatives and analogs*

Derivatives are nucleic acid sequences or amino acid sequences formed from the native compounds either directly or by modification or partial substitution. Analogs are nucleic acid sequences or amino acid sequences that have a structure similar to, but not identical to, the native compound but differ from it in respect to certain components orside chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid. Derivatives or analogs of the nucleic acids or proteins of *CGI-69* include, but are not limited to, molecules comprising regions that are substantially homologous to the nucleic acids or proteins of *CGI-69,* in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions (Ausubel et al., 1987).

7. *homology*

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of CGI-69. Isoforms can be expressed in different tissues of the same organism as a result of, for example, alternative splicing of RNA. For example, CGI-$69_L$ is an isoform of CGI-69. Alternatively, different genes can encode isoforms. For *CGI-69*, homologous nucleotide sequences include nucleotide sequences encoding for an CGI-69 of species other than humans, including, but not limited to: vertebrates, *e.g.,* frog, mouse, (see Fig. 2), rat, rabbit, dog, cat, cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human CGI-69. Homologous nucleic acid sequences include those nucleic acid sequences that encode conservative amino acid substitutions (see below) in SEQ ID NO:3, as well as a polypeptide possessing CGI-69 biological activity. Various biological activities of the CGI-69 are described below.

*8. open reading frames*

The open reading frame (ORF) of a *CGI-69* gene encodes CGI-69 or CGI-$69_L$. An ORF is a nucleotide sequence that has a start codon (ATG) and terminates with one of the three "stop" codons (TAA, TAG, or TGA). In this invention, however, an ORF may be any part of a coding sequence that may or may not comprise a start codon and a stop codon. To achieve a unique sequence, preferable *CGI-69* ORFs encode at least 50 amino acids. *CGI-69 polypeptides*

1. *mature*

A *CGI-69* can encode a mature CGI-69. A "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product, encoded by the corresponding gene. Alternatively, it may be defined as the polypeptide, precursor or proprotein encoded by an open reading frame described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an open reading frame, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

**2. *active***

An active CGI-69 polypeptide or CGI-69 polypeptide fragment retains a biological activity similar, but not necessarily identical, to an activity of a naturally-occuring (wild-type) CGI-69 polypeptide, including mature forms. A particular biological assay, with or without dose dependency, can be used to determine CGI-69 activity. A nucleic acid fragment encoding a biologically-active portion of CGI-69 can be prepared by isolating a portion of SEQ ID NO:1 that encodes a polypeptide having an CGI-69 biological activity (the biological activities of the CGI-69 are described below), expressing the encoded portion of CGI-69 (*e.g.,* by recombinant expression *in vitro*) and assessing the activity of the encoded portion of CGI-69. Biological activity refers to a function, either inhibitory or stimulatory, caused by a native CGI-69, for example, mitochondrial localization.

CGI-69 *nucleic acid variants and hybridization*

**1. *variant polynucleotides, genes and recombinant genes***

In addition to the *CGI-69* sequence shown in SEQ ID NO:1, DNA sequence polymorphisms that change the amino acid sequences of the CGI-69 may exist within a population. For example, allelic variation among individuals will exhibit genetic polymorphism in *CGI-69*. The terms "gene" and "recombinant gene" refer to nucleic acid molecules comprising an open reading frame (ORF) encoding CGI-69, preferably a vertebrate CGI-69. Such natural allelic variations can typically result in 1-5% variance in *CGI-69*. *"CGI-69* variant polynucleotide" or "*CGI-69* variant nucleic acid sequence" means a nucleic acid molecule which encodes an active CGI-69 that (1) has at least about 80% nucleic acid sequence identity with a nucleotide acid sequence encoding a full-length native CGI-69, (2) a full-length native CGI-69 lacking the signal peptide, or (3) any other fragment of a full-length CGI-69, Ordinarily, an *CGI-69* variant polynucleotide will have at least about 80% nucleic acid sequence identity, more preferably at least about 81%, 82%, 83%, 84%, 85%, 86%, 87%, 88%, 89%, 90%, 91%, 92%, 93%, 94%, 95%, 96%, 97%, 98% nucleic acid sequence identity and yet more preferably at least about 99% nucleic acid sequence identity with the nucleic acid sequence encoding a full-length native CGI-69. A *CGI-69* variant polynucleotide may encode full-length native CGI-69 lacking the signal peptide, or any other fragment of a full-length CGI-69. Variants do not encompass the native nucleotide sequence.

Ordinarily, *CGI-69* variant polynucleotides are at least about 30 nucleotides in length, often at least about 60, 90, 120, 150, 180, 210, 240, 270, 300, 450, 600 nucleotides in length, more often at least about 900 nucleotides in length, or more.

"Percent (%) nucleic acid sequence identity" with respect to CGI-69-encoding nucleic acid sequences identified herein is defined as the percentage of nucleotides in CGI-69 that are identical with the nucleotides in a candidate sequence of interest, after aligning the sequences and introducing gaps, if necessary, to achieve the maximum percent sequence identity. Alignment for purposes of determining % nucleic acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

When nucleotide sequences are aligned, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) can be calculated as follows:

$$\% \text{ nucleic acid sequence identity} = W/Z \cdot 100$$

where

W is the number of nucleotides scored as identical matches by the sequence alignment program's or algorithm's alignment of C and D

and

Z is the total number of nucleotides in D.

When the length of nucleic acid sequence C is not equal to the length of nucleic acid sequence D, the % nucleic acid sequence identity of C to D will not equal the % nucleic acid sequence identity of D to C.

2. *Stringency*

Homologs (*i.e.,* nucleic acids encoding CGI-69 derived from species other than human) or other related sequences (*e.g*., paralogs) can be obtained by low, moderate or high stringency hybridization with all or a portion of the particular human sequence as a probe using methods well known in the art for nucleic acid hybridization and cloning.

The specificity of single stranded DNA to hybridize complementary fragments is determined by the "stringency" of the reaction conditions. Hybridization stringency increases as the propensity to form DNA duplexes decreases. In nucleic acid hybridization reactions, the stringency can be chosen to either favor specific hybridizations (high stringency), which can be used to identify, for example, full-length clones from a library. Less-specific hybridizations (low stringency) can be used to identify related, but not exact, DNA molecules (homologous, but not identical) or segments.

DNA duplexes are stabilized by: (1) the number of complementary base pairs, (2) the type of base pairs, (3) salt concentration (ionic strength) of the reaction mixture, (4) the temperature of the reaction, and (5) the presence of certain organic solvents, such as formamide which decreases DNA duplex stability. In general, the longer the probe, the higher the temperature required for proper annealing. A common approach is to vary the temperature: higher relative temperatures result in more stringent reaction conditions. (Ausubel et al., 1987) provide an excellent explanation of stringency of hybridization reactions.

To hybridize under "stringent conditions" describes hybridization protocols in which nucleotide sequences at least 60% homologous to each other remain hybridized. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium.

(a) *high stringency*

"Stringent hybridization conditions" conditions enable a probe, primer or oligonucleotide to hybridize only to its target sequence. Stringent conditions are sequence-dependent and will differ. Stringent conditions comprise: (1) low ionic strength and high temperature washes *(e.g.* 15 mM sodium chloride, 1.5 mM sodium citrate, 0.1 % sodium dodecyl sulfate at 50°C); (2) a denaturing agent during hybridization (*e.g.* 50% (v/v) formamide, 0.1% bovine serum albumin, 0.1% Ficoll, 0.1% polyvinylpyrrolidone, 50mM sodium phosphate buffer (pH 6.5; 750 mM sodium chloride, 75 mM sodium citrate at 42°C); or (3) 50% formamide. Washes typically also comprise 5X SSC (0.75 M NaCl, 75 mM sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C. Preferably, the conditions are such that sequences at least about 65%, 70%, 75%, 85%, 90%, 95%, 98%, or 99% homologous to each other typically remain hybridized to each other. These conditions are presented as examples and are not meant to be limiting.

(b) *moderate stringency*

"Moderately stringent conditions" use washing solutions and hybridization conditions that are less stringent (Sambrook, 1989), such that a polynucleotide will hybridize to the entire, fragments, derivatives or analogs of SEQ ID NO:1. One example comprises hybridization in 6X SSC, 5X Denhardt's solution, 0.5% SDS and 100 mg/ml denatured salmon sperm DNA at 55°C, followed by one or more washes in 1X SSC, 0.1% SDS at 37°C. The temperature, ionic strength, *etc.,* can be adjusted to accommodate experimental factors such as probe length. Other moderate stringency conditions have been described (Ausubel et al., 1987; Kriegler, 1990).

(c) *low stringency*

"Low stringent conditions" use washing solutions and hybridization conditions that are less stringent than those for moderate stringency (Sambrook, 1989), such that a polynucleotide will hybridize to the entire, fragments, derivatives or analogs of SEQ ID NO:1. A non-limiting example of low stringency hybridization conditions are hybridization in 35% formamide, 5X SSC, 50 mM Tas-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency, such as those for cross-species hybridizations are well-described (Ausubel et al., 1987; Kriegler, 1990; Shilo and Weinberg, 1981).

3. *Conservative mutations*

In addition to naturally-occurring allelic variants of *CGI-69,* changes can be introduced by mutation into SEQ ID NO:1 that incur alterations in the amino acid sequences of the encoded CGI-69 that do not alter CGI-69 function. For example, nucleotide substitutions leading to amino acid substitutions at "non-essential" amino acid residues can be made in the sequence of SEQ ID NO:3.

A "non-essential" amino acid residue is a residue that can be altered from the wild-type sequences of the CGI-69 without altering biological activity, whereas an "essential" amino acid residue is required for such biological activity. For example, amino acid residues that are conserved among the CGI-69 of the invention are predicted to be particularly non-amenable to alteration. Amino acids for which conservative substitutions can be made are well known in the art.

Useful conservative substitutions are shown in Table A, "Preferred substitutions." Conservative substitutions whereby an amino acid of one class is replaced with another amino acid of the same type fall within the scope of the invention so long as the substitution does not materially alter the biological activity of the compound. If such substitutions result in a change in biological activity, then more substantial changes, indicated in Table B as exemplary, are introduced and the products screened for CGI-69 polypeptide biological activity.

Table A

| Preferred substitutions | | |
|---|---|---|
| Original residue | Exemplary substitutions | Preferred substitutions |
| Ala (A) | Val, Leu, Ile | Val |
| Arg (R) | Lys, Gln, Asn | Lys |
| Asn(N) | Gln, His, Lys, Arg | Gln |
| Asp (D) | Glu | Glu |
| Cys (C) | Ser | Ser |
| Gln (Q) | Asn | Asn |
| Glu (E) | Asp | Asp |
| Gly (G) | Pro, Ala | Ala |
| His (H) | Asn, Gln, Lys, Arg | Arg |
| Ile (I) | Leu, Val, Met, Ala, Phe, Norleucine | Leu |
| Leu (L) | Norleucine, Ile, Val, Met, Ala, Phe | Ile |
| Lys (K) | Arg, Gln, Asn | Arg |
| Met (M) | Leu, Phe, Ile | Leu |
| Phe (F) | Leu, Val, Ile, Ala, Tyr | Leu |
| Pro (P) | Ala | Ala |
| Ser (S) | Thr | Thr |
| Thr (T) | Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Norleucine | Leu |

Non-conservative substitutions that effect (1) the structure of the polypeptide backbone, such as a β-sheet or α-helical conformation, (2) the charge (3) hydrophobicity, or (4) the bulk of the side chain of the target site can modify CGI-69 polypeptide function. Residues are divided into groups based on common side-chain properties as denoted in Table B. Non-conservative substitutions entail exchanging a member of one of these classes for another class. Substitutions may be introduced into conservative substitution sites or more preferably into non-conserved sites.

Table B

| Amino acid classes | |
|---|---|
| Class | Amino acids |
| hydrophobic | Norleucine, Met, Ala, Val, Leu, Ile |
| neutral hydrophilic | Cys, Ser, Thr |
| acidic | Asp, Glu |
| basic | Asn, Gln, His, Lys, Arg |
| disrupt chain conformation | Gly, Pro |
| aromatic | Trp, Tyr, Phe |

The variant polypeptides can be made using methods known in the art such as oligonucleotide-mediated (site-directed) mutagenesis, alanine scanning, and PCR mutagenesis. Site-directed mutagenesis (Carter, 1986; Zoller and Smith, 1987), cassette mutagenesis, restriction selection mutagenesis (Wells et al., 1985) or other known techniques can be performed on the cloned DNA to produce the *CGI-69* variant DNA (Ausubel et al., 1987; Sambrook, 1989).

In one embodiment, the isolated nucleic acid molecule comprises a nucleotide sequence encoding a protein, wherein the protein comprises an amino acid sequence at least about 45%, preferably 60%, more preferably 70%, 80%, 90%, and most preferably about 95% homologous to SEQ ID NO:3.

4. *Anti-sense nucleic acids*

Using antisense and sense CGI-69 oligonucleotides can prevent CGI-69 polypeptide expression. These oligonucleotides bind to target nucleic acid sequences, forming duplexes that block transcription or translation of the target sequence by enhancing degradation of the duplexes, terminating prematurely transcription or translation, or by other means.

Antisense or sense oligonucleotides are singe-stranded nucleic acids, either RNA or DNA, which can bind target *CGI-69* mRNA (sense) or *CGI-69* DNA (antisense) sequences. Anti-sense nucleic acids can be designed according to Watson and Crick or Hoogsteen base pairing rules. The anti-sense nucleic acid molecule can be complementary to the entire coding region of *CGI-69* mRNA, but more preferably, to only a portion of the coding or noncoding region of *CGI-69* mRNA. For example, the anti-sense oligonucleotide can be complementary to the region surrounding the translation start site of *CGI-69* mRNA. Antisense or sense oligonucleotides may comprise a fragment of the *CGI-69* DNA coding region of at least about 14 nucleotides, preferably from about 14 to 30 nucleotides. In general, antisense RNA or DNA molecules can comprise at least 5, 10, 15, 20, 25, 30, 35, 40, 45, 50, 55, 60, 65, 70, 75, 80, 85, 90, 95, 100 bases in length or more. Among others, (Stein and Cohen, 1988; van der Krol et al., 1988b) describe methods to derive antisense or a sense oligonucleotides from a given cDNA sequence.

Examples of modified nucleotides that can be used to generate the anti-sense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the anti-sense nucleic acid can be produced biologically using an expression vector into which a nucleic acid has been sub-cloned in an anti-sense orientation such that the transcribed RNA will be complementary to a target nucleic acid of interest.

To introduce antisense or sense oligonucleotides into target cells (cells containing the target nucleic acid sequence), any gene transfer method may be used. Examples of gene transfer methods include (1) biological, such as gene transfer vectors like Epstein-Barr virus or conjugating the exogenous DNA to a ligand-binding molecule, (2) physical, such as electroporation and injection, and (3) chemical, such as $CaPO_4$ precipitation and oligonucleotide-lipid complexes.

An antisense or sense oligonucleotide is inserted into a suitable gene transfer retroviral vector. A cell containing the target nucleic acid sequence is contacted with the recombinant retroviral vector, either in vivo or ex vivo. Examples of suitable retroviral vectors include those derived from the murine retrovirus M-MuLV, N2 (a retrovirus derived from M-MuLV), or the double copy vectors designated DCT5A, DCT5B and DCT5C (WO 90/13641, 1990). To achieve sufficient nucleic acid molecule transcription, vector constructs in which the transcription of the anti-sense nucleic acid molecule is controlled by a strong pol II or pol III promoter are preferred. Alternatively, inducible promoters may be preferred when it is desired to control the expression of the construct.

To specify target cells in a mixed population of cells, cell surface receptors that are specific to the target cells can be exploited. Antisense and sense oligonucleotides are conjugated to a ligand-binding molecule, as described in (WO 91/04753, 1991). Ligands are chosen for receptors that are specific to the target cells. Examples of suitable ligand-binding molecules include cell surface receptors, growth factors, cytokines, or other ligands that bind to cell surface receptors or molecules. Preferably, conjugation of the ligand-binding molecule does not substantially interfere with the ability of the receptors or molecule to bind the ligand-binding molecule conjugate, or block entry of the sense or antisense oligonucleotide or its conjugated version into the cell.

Liposomes efficiently transfer sense or an antisense oligonucleotide to cells (WO 90/10448, 1990). The sense or antisense oligonucleotide-lipid complex is preferably dissociated within the cell by an endogenous lipase.

The anti-sense nucleic acid molecule of *CGI-69* may be an $\alpha$-anomeric nucleic acid molecule. An $\alpha$-anomeric nucleic acid molecule forms specific double-stranded hybrids with complementary RNA in which, contrary to the usual $\alpha$-units, the strands run parallel to each other (Gautier et al., 1987). The anti-sense nucleic acid molecule can also comprise a 2'-o-methylribonucleotide (Inoue et al., 1987a) or a chimeric RNA-DNA analogue (Inoue et al., 1987b).

In one embodiment, an anti-sense nucleic acid of *CGI-69* is a ribozyme. Ribozymes are catalytic RNA molecules with ribonuclease activity that are capable of cleaving a single-stranded nucleic acid, such as an mRNA, to which they have a complementary region. Thus, ribozymes, such as hammerhead ribozymes (Haseloff and Gerlach, 1988) can be used to catalytically cleave *CGI-69* mRNA transcripts and thus inhibit translation. A ribozyme specific for an CGI-69-encoding nucleic acid can be designed based on the nucleotide sequence of an CGI-69 cDNA (*i.e.,* SEQ ID NOS:1or 2). For example, a derivative of a *Tetrahymena* L-19 IVS RNA can be constructed in which the nucleotide sequence of the active site is complementary to the nucleotide sequence to be cleaved in an CGI-69-encoding mRNA (Cech et al., U.S. Patent No. 5,116,742, 1992; Cech et al., U.S. Patent No. 4,987,071,1991). *CGI-69* mRNA can also be used to select a catalytic RNA having a specific ribonuclease activity from a pool of RNA molecules (Bartel and Szostak, 1993).

Alternatively, *CGI-69* expression can be inhibited by targeting nucleotide sequences complementary to the regulatory region of the *CGI-69* (*e.g*., the *CGI-69* promoter and/or enhancers) to form triple helical structures that prevent transcription of the *CGI-69* in target cells (Helene, 1991; Helene et al., 1992; Maher, 1992).

Modifications of antisense and sense oligonucleotides can augment their effectiveness. Modified sugar-phosphodiester bonds or other sugar linkages (WO 91/06629, 1991), increase *in vivo* stability by conferring resistance to endogenous nucleases without disrupting binding specificity to target sequences. Other modifications can increase the affinities of the oligonucleotides for their targets, such as covalently linked organic moieties (WO 90/10448, 1990) or poly-(L)-lysine. Other attachments modify binding specificities of the oligonucleotides for their targets, including metal complexes or intercalating (*e.g.* ellipticine) and alkylating agents.

For example, the deoxyribose phosphate backbone of the nucleic acids can be modified to generate peptide nucleic acids (Hyrup and Nielsen, 1996). "Peptide nucleic acids" or "PNAs" refer to nucleic acid mimics (*e.g.,* DNA mimics) in that the deoxyribose phosphate backbone is replaced by a pseudopeptide backbone and only the four natural nucleobases are retained. The neutral backbone of PNAs allows for specific hybridization to DNA and RNA under conditions of low ionic strength. The synthesis of PNA oligomers can be performed using standard solid phase peptide synthesis protocols (Hyrup and Nielsen, 1996; Perry-O'Kecfe et al., 1996).

PNAs of *CGI-69* can be used in therapeutic and diagnostic applications. For example, PNAs can be used as anti-sense or antigene agents for sequence-specific modulation of gene expression by inducing transcription or translation arrest or inhibiting replication. *CGI-69* PNAs may also be used in the analysis of single base pair mutations (*e. g*., PNA directed PCR clamping; as artificial restriction enzymes when used in combination with other enzymes, *e.g.,* $S_1$ nucleases (Hyrup and Nielsen, 1996); or as probes or primers for DNA sequence and hybridization (Hyrup and Nielsen, 1996; Peny-O'Keefe et al.,1996).

PNAs of *CGI-69* can be modified to enhance their stability or cellular uptake. Lipophilic or other helper groups may be attached to PNAs, PNA-DNA dimmers formed, or the use of liposomes or other drug delivery techniques. For example, PNA-DNA chimeras can be generated that may combine the advantageous properties of PNA and DNA. Such chimeras allow DNA recognition enzymes (*e.g.*, RNase H and DNA polymerases) to interact with the DNA portion while the PNA portion provides high binding affinity and specificity. PNA-DNA chimeras can be linked using linkers of appropriate lengths selected in terms of base stacking, number of bonds between the nucleobases, and orientation (Hyrup and Nielsen, 1996). The synthesis of PNA-DNA chimeras can be performed (Finn et al., 1996; Hyrup and Nielsen, 1996). For example, a DNA chain can be synthesized on a solid support using standard phosphoramidite coupling chemistry, and modified nucleoside analogs, *e.g.*, 5'-(4-methoxytrityl)amino-5'-deoxy-thymidine phosphoramidite, can be used between the PNA and the 5' end of DNA (Finn et al., 1996; Hyrup and Nielsen, 1996). PNA monomers are then coupled in a stepwise manner to produce a chimeric molecule with a 5' PNA segment and a 3' DNA segment (Finn et al., 1996). Alternatively, chimeric molecules can be synthesized with a 5' DNA segment and a 3' PNA segment (Petersen et al., 1976).

The oligonucleotide may include other appended groups such as peptides (*e.g.,* for targeting host cell receptors *in vivo*), or agents facilitating transport across the cell membrane (Lemaitre et al., 1987; Letsinger et al., 1989; Tullis, US Patent No. 4904582, 1988) or the blood-brain barrier *(e.g.,* (Pardridge and Schimmel, WO89/10134, 1989)). In addition, oligonucleotides can be modified with hybridization-triggered cleavage agents (van der Krol et al., 1988a) or intercalating agents (Zon, 1988). The oligonucleotide may be conjugated to another molecule, *e.g.,* a peptide, a hybridization triggered cross-linking agent, a transport agent, a hybridization-triggered cleavage agent, and the like.

*CGI-69 polypeptides*

One aspect of the invention pertains to isolated CGI-69, and biologically-active portions derivatives, fragments, analogs or homologs thereof. In one embodiment, native CGI-69 can be isolated from cells or tissue sources by an appropriate purification scheme using standard protein purification techniques. In another embodiment, CGI-69 molecules are produced by recombinant DNA techniques. Alternative to recombinant expression, a CGI-69 or polypeptide can be synthesized chemically using standard peptide synthesis techniques.

1. *Polypeptides*

A CGI-69 polypeptide includes the amino acid sequence of CGI-69 which sequence is provided in SEQ ID NO:3. The invention also includes a mutant or variant protein any of which residues may be changed from the corresponding residues shown in SEQ ID NO:3, while still encoding a protein that maintains CGI-69 activities and physiological functions, or a functional fragment thereof.

2. *Variant CGI-69 polypeptides*

In general, an CGI-69 variant preserves CGI-69 -like function and includes any variant in which residues at a particular position in the sequence have been substituted by other amino acids, and further includes the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

"CGI-69 polypeptide variant" means an active CGI-69 polypeptide having at least: (1) about 80% amino acid sequence identity with a full-length native sequence CGI-69 polypeptide sequence or (2) any fragment of a full-length CGI-69 polypeptide sequence. For example, CGI-69 polypeptide variants include CGI-69 polypeptides wherein one or more amino acid residues are added or deleted at the N- or C-terminus of the full-length native amino acid sequence. An CGI-69 polypeptide variant will have at least about 80% amino acid sequence identity, preferably at least about 81 % amino acid sequence identity, more preferably at least about 82%, 83%, 84%, 85%, 86%, 87%, 88%, 89%, 90%, 91%, 92%, 93%, 94%, 95%, 96%, 97%, 98% amino acid sequence identity and most preferably at least about 99% amino acid sequence identity with a full-length native sequence CGI-69 polypeptide sequence. Ordinarily, CGI-69 variant polypeptides are at least about 10 amino acids in length, often at least about 20 amino acids in length, more often at least about 30, 40, 50, 60, 70, 80, 90, 100, 150, 200, or 300 amino acids in length, or more.

"Percent (%) amino acid sequence identity" is defined as the percentage of amino acid residues in CGI-69 that are identical with amino acid residues in a candidate sequence when the two sequences are aligned. To determine % amino acid identity, sequences are aligned and if necessary, gaps are introduced to achieve the maximum % sequence identity; conservative substitutions are not considered as part of the sequence identity. Amino acid sequence alignment procedures to determine percent identity are well known to those of skill in the art. Often publicly available computer software such as BLAST, BLAST2, ALIGN2 or Megalign (DNASTAR) software is used to align peptide sequences. Those skilled in the art can determine appropriate parameters for measuring alignment, including any algorithms needed to achieve maximal alignment over the full length of the sequences being compared.

When amino acid sequences are aligned, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) can be calculated as:

$$\% \text{ amino acid sequence identity} = X/Y \cdot 100$$

where

X is the number of amino acid residues scored as identical matches by the sequence alignment program's or algorithm's alignment of A and B

and

Y is the total number of amino acid residues in B.

If the length of amino acid sequence A is not equal to the length of amino acid sequence B, the % amino acid sequence identity of A to B will not equal the % amino acid sequence identity of B to A.

3. *Isolated/purified polypeptides*

An "isolated" or "purified" polypeptide, protein or biologically active fragment is separated and/or recovered from a component of its natural environment. Contaminant components include materials that would typically interfere with diagnostic or therapeutic uses for the polypeptide, and may include enzymes, hormones, and other proteinaceous or non-proteinaceous materials. Preferably, the polypeptide is purified to a sufficient degree to obtain at least 15 residues of N-terminal or internal amino acid sequence. To be substantially isolated, preparations having less than 30% by dry weight of non-CGI-69 contaminating material (contaminants), more preferably less than 20%, 10% and most preferably less than 5% contaminants. An isolated, recombinantly-produced CGI-69 or biologically active portion is preferably substantially free of culture medium, i.e., culture medium represents less than 20%, more preferably less than about 10%, and most preferably less than about 5% of the volume of the CGI-69 preparation. Examples of contaminants include cell debris, culture media, and substances used and produced during *in vitro* synthesis of CGI-69.

*4. Biologically active*

Biologically active portions of CGI-69 include peptides comprising amino acid sequences sufficiently homologous to or derived from the amino acid sequences of CGI-69 (SEQ ID NO:3) that include fewer amino acids than the full-length CGI-69, and exhibit at least one activity of an CGI-69. Biologically active portions comprise a domain or motif with at least one activity of native CGI-69. A biologically active portion of a CGI-69 can be a polypeptide that is, for example, 10, 25, 50, 100 or more amino acid residues in length. Other biologically active portions, in which other regions of the protein are deleted, can be prepared by recombinant techniques and evaluated for one or more of the functional activities of a native CGI-69.

Biologically active portions of CGI-69 may have an amino acid sequence shown in SEQ ID NO:3, or substantially homologous to SEQ ID NO:3, and retains the functional activity of the protein of SEQ ID NO:3, yet differs in amino acid sequence due to natural allelic variation or mutagenesis. Other biologically active CGI-69 may comprise an amino acid sequence at least 45% homologous to the amino acid sequence of SEQ ID NO:3, and retains the functional activity of native CGI-69.

5. *Chimeric and fusion proteins*

Fusion polypeptides are useful in expression studies, cell-localization, bioassays, and CGI-69 purification. A CGI-69 "chimeric protein" or "fusion protein" comprises CGI-69 fused to a non-CGI-69 polypeptide. A non-CGI-69 polypeptide is not substantially homologous to CGI-69 (SEQ ID NO:3). A CGI-69 fusion protein may include any portion to the entire CGI-69, including any number of the biologically active portions. CGI-69 may be fused to the C-terminus of the GST (glutathione S-transferase) sequences. Such fusion proteins facilitate the purification of recombinant CGI-69. In certain host cells, *(e.g.* mammalian), heterologous signal sequences fusions may ameliorate CGI-69 expression and/or secretion. Additional exemplary fusions are presented in Table C.

Fusion proteins can be easily created using recombinant methods. A nucleic acid encoding CGI-69 can be fused in-frame with a non-CGI-69 encoding nucleic acid, to the CGI-69 $NH_2$- or COO- -terminus, or internally. Fusion genes may also be synthesized by conventional techniques, including automated DNA synthesizers. PCR amplification using anchor primers that give rise to complementary overhangs between two consecutive gene fragments that can subsequently be annealed and reamplified to generate a chimeric gene sequence (Ausubel et al., 1987) is also useful. Many vectors are commercially available that facilitate sub-cloning *CGI-69* in-frame to a fusion moiety.

Fusion proteins can be used to give CGI-69 compositions a novel characteristic not present in native CGI-69. For example, *amino*-FLAG-tagged CGI-69 and native CGI-69 have not been shown to exhibit uncoupling activity. In contrast, overexpression of *carboxy*-FLAG-tagged CGI-69 in 293 cells diminished the mitochondrial membrane potential to a similar magnitude as an uncoupling protein (UCP3). Other negatively charged sequences may have a similar biological function.

Table C

| **Useful non-CGI-69 fusion polypeptides** | | | | |
|---|---|---|---|---|
| Reporter | *in vitro* | *in vivo* | Notes | Reference |
| Human growth hormone (hGH) | Radioimmuno-assay | none | Expensive, insensitive, narrow linear range. | (Selden et al., 1986) |
| β-glucuronidase (GUS) | Colorimetric, fluorescent, or chemiluminescent | colorimetric (histo-chemical staining with X-gluc) | sensitive, broad linear range, non-iostopic. | (Gallagher, 1992) |
| Green fluorescent protein (GFP) and related molecules (RFP, BFP, CGI-69, *etc.*) | Fluorescent | fluorescent | can be used in live cells; resists photo-bleaching | (Chalfie et al., 1994) |

Table C (continued)

| Useful non-CGI-69 fusion polypeptides | | | | |
|---|---|---|---|---|
| Reporter | *in vitro* | *in vivo* | Notes | Reference |
| Luciferase (firefly) | bioluminsecent | Bio-luminescent | protein is unstable, difficult to reproduce, signal is brief | (de Wet et al., 1987) |
| Chloramphenico al acetyltransferas e (CAT) | Chromatography, differential extraction, fluorescent, or immunoassay | none | Expensive radioactive substrates, time-consuming, insensitive, narrow linear range | (Gorman et al., 1982) |
| β-galacto-sidase | colorimetric, fluorescence, chemiluminscence | colorimetric (histochemical staining with X-gal), bioluminescent in live cells | sensitive, broad linear range; some cells have high endogenous activity | (Alam and Cook, 1990) |
| Secrete alkaline phosphatase (SEAP) | colorimetric, bioluminescent, chemiluminescent | none | Chemiluminscence assay is sensitive and broad linear range; some cells have endogenouse alkaline phosphatase activity | (Berger et al., 1988) |
| Tat from HIV | Mediates delivery into cytoplasm and nuclei | Mediates delivery into cytoplasm and nuclei | Exploits amino acid residues of HIV tat protein. | (Frankel et al., US Patent No. 5,804,604, 1998) |

*Therapeutic applications of CGI-69*

1. *Agonists and antagonists*

"Antagonist" includes any molecule that partially or fully blocks, inhibits, or neutralizes a biological activity of endogenous CGI-69. Similarly, "agonist" includes any molecule that mimics a biological activity of endogenous CGI-69. Biologic activity biologic and/or immunologic activities of native or naturally-occurring human OGC. A preferred activity is mitochondrial localization. Molecules that can act as agonists or antagonists include fragments or variants of endogenous CGI-69, peptides, antisense oligonucleotides, small organic molecules, *etc.*

2. *Identifying antagonists and agonists*

To assay for antagonists, CGI-69 is added to, or expressed in, a cell in vitro along with the compound to be screened for a particular activity. If the compound inhibits the activity of interest in the presence of the CGI-69, that compound is an antagonist to the CGI-69; if CGI-69 activity is enhanced, the compound is an agonist.

(a) *Specific examples of potential antagonists and agonist*

Any molecule that alters CGI-69 cellular effects is a candidate antagonist or agonist. Screening techniques well known to those skilled in the art can identify these molecules. Examples of antagonists and agonists include: (1) small organic and inorganic compounds, (2) small peptides, (3) Abs and derivatives, (4) polypeptides closely related to CGI-69, (5) antisense DNA and RNA, (6) ribozymes, (7) triple DNA helices and (8) nucleic acid aptamers.

Small molecules that bind to the CGI-69 active site or other relevant part of the polypeptide and inhibit the biological activity of the CGI-69 are antagonists. Examples of small molecule antagonists include small peptides, peptide-like molecules, preferably soluble, and synthetic non-peptidyl organic or inorganic compounds. These same molecules, if they enhance CGI-69 activity, are examples of agonists.

Alternatively, a potential antagonist or agonist may be a closely related protein, for example, a mutated form of the CGI-69 that recognizes an CGI-69-interacting protein but imparts no effect, thereby competitively inhibiting CGI-69 action. Alternatively, a mutated CGI-69 may be constitutively activated and may act as an agonist.

Antisense RNA or DNA constructs can be effective antagonists. Antisense RNA or DNA molecules block function by inhibiting translation by hybridizing to targeted mRNA. Antisense technology can be used to control gene expression through triple-helix formation or antisense DNA or RNA, both of which depend on polynucleotide binding to DNA or RNA. For example, the 5' coding portion of the *CGI-69* sequence is used to design an antisense RNA oligonucleotide of from about 10 to 40 base pairs in length. A DNA oligonucleotide is designed to be complementary to a region of the gene involved in transcription (triple helix) (Beal and Dervan, 1991; Cooney et al., 1988; Lee et al., 1979), thereby preventing transcription and the production of the CGI-69. The antisense RNA oligonucleotide hybridizes to the mRNA *in vivo* and blocks translation of the mRNA molecule into the CGI-69 (antisense) (Cohen, 1989; Okano et al., 1991). These oligonucleotides can also be delivered to cells such that the antisense RNA or DNA may be expressed *in vivo* to inhibit production of the CGI-69. When antisense DNA is used, oligodeoxyribonucleotides derived from the translation-initiation site, *e.g.,* between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques (WO 97/33551,1997; Rossi, 1994).

To inhibit transcription, triple-helix nucleic acids that are single-stranded and comprise deoxynucleotides are useful antagonists. These oligonucleotides are designed such that triple-helix formation via Hoogsteen base-pairing rules is promoted, generally requiring stretches of purines or pyrimidines (WO 97/33551, 1997).

Aptamers are short oligonucleotide sequences that can be used to recognize and specifically bind almost any molecule. The systematic evolution of ligands by exponential enrichment (SELEX) process (Ausubel et al., 1987; Ellington and Szostak, 1990; Tuerk and Gold, 1990) is powerful and can be used to find such aptamers. Aptamers have many diagnostic and clinical uses; almost any use in which an antibody has been used clinically or diagnostically, aptamers too may be used. In addition, aptamers are cheaper to make once they have been identified, and can be easily applied in a variety of formats, including administration in pharmaceutical compositions, bioassays and diagnostic tests (Jayasena, 1999).

*CGI-69 recombinant expression vectors and host cells*

Vectors are tools used to shuttle nucleic acids between host cells or as a means to express a nucleotide sequence. Some vectors function only in prokaryotes, while others function in both prokaryotes and eukaryotes, enabling large-scale DNA preparation from prokaryotes for expression in eukaryotes. Inserting a DNA of interest, such as *CGI-69* nucleotide sequence or a fragment, is accomplished by ligation techniques and/or mating protocols well known to the skilled artisan. Such DNA is inserted such that its integration does not disrupt any necessary components of the vector. In the case of vectors that are used to express the inserted DNA protein, the introduced DNA is operably-linked to the vector elements that govern its transcription and translation.

Vectors can be divided into two general classes: Cloning vectors are replicating plasmid or phage with regions that are non-essential for propagation in an appropriate host cell, and into which foreign DNA can be inserted; the foreign DNA is replicated and propagated as if it were a component of the vector. An expression vector (such as a plasmid, yeast, or animal virus genome) is used to introduce foreign genetic material into a host cell or tissue in order to transcribe and translate the foreign DNA. In expression vectors, the introduced DNA is operably-linked to elements, such as promoters, that signal to the host cell to transcribe the inserted DNA. Some promoters are exceptionally useful, such as inducible promoters that control gene transcription in response to specific factors. Operably-linking *CGI-69* or anti-sense construct to an inducible promoter can control the expression of *CGI-69* or fragments, or anti-sense constructs. Examples of classic inducible promoters include those that are responsive to $\alpha$-interferon, heat-shock, heavy metal ions, and steroids such as glucocorticoids (Kaufman, 1990) and tetracycline. Other desirable inducible promoters include those that are not endogenous to the cells in which the construct is being introduced, but, however, is responsive in those cells when the induction agent is exogenously supplied.

Vectors have many difference manifestations. A "plasmid" is a circular double stranded DNA molecule into which additional DNA segments can be introduced. Viral vectors can accept additional DNA segments into the viral genome. Certain vectors are capable of autonomous replication in a host cell (*e.g.,* episomal mammalian vectors or bacterial vectors having a bacterial origin of replication). Other vectors (*e.g.,* non-episomal mammalian vectors) are integrated into the genome of a host cell upon introduction into the host cell, and thereby are replicated along with the host genome. In general, useful expression vectors are often plasmids. However, other forms of expression vectors, such as viral vectors (*e.g.*, replication defective retroviruses, adenoviruses and adeno-associated viruses) are contemplated.

Recombinant expression vectors that comprise *CGI-69* (or fragments) regulate *CGI-69* transcription by exploiting one or more host cell-responsive (or that can be manipulated *in vitro)* regulatory sequences that is operably-linked to *CGI-69*. "Operably-linked" indicates that a nucleotide sequence of interest is linked to regulatory sequences such that expression of the nucleotide sequence is achieved.

Vectors can be introduced in a variety of organisms and/or cells (Table D). Alternatively, the vectors can be transcribed and translated *in vitro,* for example using T7 promoter regulatory sequences and T7 polymerase.

Table D

| Examples of hosts for cloning or expression | | |
|---|---|---|
| Organisms | Examples | Sources and References* |
| Prokaryotes | | |
| Enterobacteriaceae | *E. coli* | |
| | K 12 strain MM294 | ATCC 31,446 |
| | X1776 | ATCC 31,537 |
| | W3110 | ATCC 27,325 |
| | K5 772 | ATCC 53,635 |
| | *Enterobacter* | |
| | *Erwinia* | |
| | *Klebsiella* | |
| | *Proteus* | |
| | *Salmonella (S. tyhpimurium*) | |
| | *Serratia (S. marcescans*) | |
| | *Shigella* | |
| | *Bacilli* (B. *subtilis* and *B. licheniformis*) | |
| | *Pseudomonas* (P. *aeruginosa*) | |
| | *Streptomyces* | |

*Unreferenced cells are generally available from American Type Culture Collection (Manassas, VA).

Table D (continued)

| Examples of hosts for cloning or expression | | |
|---|---|---|
| Organisms | Examples | Sources and References* |
| Eukaryotes | | |
| Yeasts | *Saccharomyces cerevisiae* | |
| | *Schizosaccharomyces pombe* | |
| | *Kluyveromyces* | (Fleer et al., 1991) |
| | *K. lactis* MW98-8C, CBS683, CBS4574 | (de Louvencourt et al., 1983) |
| | *K. fragilis* | ATCC 12,424 |
| | *K. bulgaricus* | ATCC 16,045 |
| | *K. wickeramii* | ATCC 24,178 |
| | *K. waltii* | ATCC 56,500 |
| | *K. drosophilarum* | ATCC 36,906 |
| | *K. thermotolerans* | |
| | *K. marxianus; yarrowia* | (EPO 402226, 1990) |
| | *Pichia pastoris* | (Sreelaishna et al., 1988) |
| | *Candida* | |
| | *Trichoderma reesia* | |
| | *Neurospora crassa* | (Case et al., 1979) |
| | *Torulopsis* | |
| | *Rhodotorula* | |
| | *Schwanniomyces (S. occidentalis*) | |
| Filamentous Fungi | *Neurospora* | |
| | *Penicillium* | |
| | *Tolypocladium* | (WO 91/00357, 1991) |
| | *Aspergillus (A. nidulans* and *A. niger)* | (Kelly and Hynes, 1985; Tilbum et al., 1983; Yelton et al., 1984) |
| Invertebrate cells | *Drosophila S2* | |
| | *Spodoptera* Sf9 | |
| Vertebrate cells | Chinese Hamster Ovary (CHO) | |
| | simian COS COS-7 | ATCC CRL 1651 |
| | HEK 293 | |

*Unreferenced cells are generally available from American Type Culture Collection (Manassas, VA).

Vector choice is dictated by the organism or cells being used and the desired fate of the vector. Vectors may replicate once in the target cells, or may be "suicide" vectors. In general, vectors comprise signal sequences, origins of replication, marker genes, enhancer elements, promoters, and transcription termination sequences. The choice of these elements depends on the organisms in which the vector will be used and are easily determined. Some of these elements may be conditional, such as an inducible or conditional promoter that is turned "on" when conditions are appropriate. Examples of inducible promoters include those that are tissue-specific, which relegate expression to certain cell types, steroid-responsive, or heat-shock reactive. Some bacterial repression systems, such as the *lac* operon, have been exploited in mammalian cells and transgenic animals (Fieck et al., 1992; Wyborski et al., 1996; Wyborski and Short, 1991). Vectors often use a selectable marker to facilitate identifying those cells that have incorporated the vector. Many selectable markers are well known in the art for the use with prokaryotes, usually antibiotic-resistance genes or the use of autotrophy and auxotrophy mutants.

Using antisense and sense CGI-69 oligonucleotides can prevent CGI-69 polypeptide expression. These oligonucleotides bind to target nucleic acid sequences, forming duplexes that block transcription or translation of the target sequence by enhancing degradation of the duplexes, terminating prematurely transcription or translation, or by other means.

Antisense or sense oligonucleotides are single-stranded nucleic acids, either RNA or DNA, which can bind target *CGI-69* mRNA (sense) or *CGI-69* DNA (antisense) sequences. According to the present invention, antisense or sense oligonucleotides comprise a fragment of the *CGI-69* DNA coding region of at least about 14 nucleotides, preferably from about 14 to 30 nucleotides. In general, antisense RNA or DNA molecules can comprise at least 5, 10, 15, 20, 25, 30, 35, 40, 45, 50, 55, 60, 65, 70, 75, 80, 85, 90, 95, 100 bases in length or more. Among others, (Stein and Cohen, 1988; van der Krol et al., 1988b) describe methods to derive antisense or a sense oligonucleotides from a given cDNA sequence.

Modifications of antisense and sense oligonucleotides can augment their effectiveness. Modified sugar-phosphodiester bonds or other sugar linkages (WO 91/06629, 1991), increase in vivo stability by conferring resistance to endogenous nucleases without disrupting binding specificity to target sequences. Other modifications can increase the affinities of the oligonucleotides for their targets, such as covalently linked organic moieties (WO 90/10448, 1990) or poly-(L)-lysine. Other attachments modify binding specificities of the oligonucleotides for their targets, including metal complexes or intercalating *(e.g.* ellipticine) and alkylating agents.

To introduce antisense or sense oligonucleotides into target cells (cells containing the target nucleic acid sequence), any gene transfer method may be used and are well known to those of skill in the art. Examples of gene transfer methods include 1) biological, such as gene transfer vectors like Epstein-Barr virus or conjugating the exogenous DNA to a ligand-binding molecule (WO 91/04753, 1991), 2) physical, such as electroporation, and 3) chemical, such as $CaPO_4$ precipitation and oligonucleotide-lipid complexes (WO 90/10448,1990).

The terms "host cell" and "recombinant host cell" are used interchangeably. Such terms refer not only to a particular subject cell but also to the progeny or potential progeny of such a cell. Because certain modifications may occur in succeeding generations due to either mutation or environmental influences, such progeny may not, in fact, be identical to the parent cell, but are still included within the scope of the term.

Methods of eukaryotic cell transfection and prokaryotic cell transformation are well known in the art. The choice of host cell will dictate the preferred technique for introducing the nucleic acid of interest. Table E, which is not meant to be limiting, summarizes many of the known techniques in the art. Introduction of nucleic acids into an organism may also be done with *ex vivo* techniques that use an *in vitro* method of transfection, as well as established genetic techniques, if any, for that particular organism.

Table E:

| Methods to introduce nucleic acid into cells | | | |
|---|---|---|---|
| Cells | Methods | References | Notes |
| Prokaryotes (bacteria) | Calcium chloride | (Cohen et al., 1972; Hanahan, 1983; Mandel and Higa, 1970) | |
| | Electroporation | (Shigekawa and Dower, 1988) | |
| Eukaryotes | | | |

Table E: (continued)

| Methods to introduce nucleic acid into cells | | | |
|---|---|---|---|
| Cells | Methods | References | Notes |
| Mammalian cells | Calcium phosphate phosphate transfection | *N*-(2-Hydroxyethyl) piperazine-*N'*-(2-ethanesulfonic acid (HEPES) buffered saline solution (Chen and Okayama, 1988; Graham and van der Eb, 1973; Wigler et al., 1978)<br>BES (*N,N*-bis(2-hydroxyethyl)-2-aminoethanesulfonic acid) buffered solution (Ishiura et al., 1982) | Cells may be "shocked" with glycerol or dimethylsulfoxide (DMSO) to increase transfection efficiency (Ausubel et al., 1987). |
| | Diethylaminoethyl (DEAE)-Dextran transfection | (Fujita et al., 1986; Lopata et al., 1984; Selden et al., 1986) | Most useful for transient, but not stable, transfections. Chloroquine can be used to increase efficiency. |
| | Electroporation | (Neumann et al., 1982; Potter, 1988; Potter et al., 1984; Wong and Neumann, 1982) | Especially useful for hard-to-transfect lymphocytes. |
| | Cationic lipid reagent transfection | (Elroy-Stein and Moss, 1990; Felgner et al., 1987; Rose et al., 1991; Whitt et al., 1990) | Applicable to both in vivo and in *vitro* transfection. |
| | Retroviral | Production exemplified by (Cepko et al., 1984; Miller and Buttimore, 1986; Pear et al., 1993)<br>Infection *in vitro* and in vivo: (Austin and Cepko, 1990; Bodine et al., 1991; Fekete and Cepko, 1993; Lemischka et al., 1986; Turner et al., 1990; Williams et al., 1984) | Lengthy process, many packaging lines available at ATCC. Applicable to both *in vivo* and *in vitro* transfection. |
| | Polybrene | (Chaney et al., 1986; Kawai and Nishizawa, 1984) | |
| | Microinjection | (Capecchi, 1980) | Can be used to establish cell lines carrying integrated copies of CGI-69 DNA sequences. |
| | Protoplast fusion | (Rassoulzadegan et al., 1982; Sandri-Goldin et al., 1981; Schaffner, 1980) | |
| Insect cells *(in vitro)* | Baculovirus systems | (Luckow, 1991; Miller, 1988; O'Reilly et al., 1992) | Useful for *in vitro* production of proteins with eukaryotic modifications. |

Table E: (continued)

| Methods to introduce nucleic acid into cells | | | |
|---|---|---|---|
| Cells | Methods | References | Notes |
| Yeast | Electroporation | (Becker and Guarente, 1991) | |
| | Lithium acetate | (Gietz et al., 1998; Ito et al., 1983) | |
| | Spheroplast fusion | (Beggs, 1978; Hinnen et al., 1978) | Laborious, can produce aneuploids. |
| Plant cells [general reference: (Hansen and Wright, 1999)] | Agrobacterium transformation | (Bechtold and Pelletier, 1998; Escudero and Hohn, 1997; Hansen and Chilton, 1999; Touraev and al., 1997) | |
| | Biolistics (microprojectiles) | (Finer et al., 1999; Hansen and Chilton, 1999; Shillito, 1999) | |
| | Electroporation (protoplasts) | (Fromm et al., 1985; Ou-Lee et al., 1986; Rhodes et al., 1988; Saunders et al., 1989)<br>May be combined with liposomes (Trick and al., 1997) | |
| | Polyethylene glycol (PEG) treatment | (Shillito, 1999) | |
| | Liposomes | May be combined with electroporation (Trick and al., 1997) | |
| | *in planta* microinjection | (Leduc and al., 1996; Zhou and al., 1983) | |
| | Seed imbibition | (Trick and al., 1997) | |
| | Laser beam | (Hoffman, 1996) | |
| | Silicon carbide whiskers | (Thompson and al., 1995) | |

Vectors often use a selectable marker to facilitate identifying those cells that have incorporated the vector. Many selectable markers are well known in the art for the use with prokaryotes, usually antibiotic-resistance genes or the use of autotrophy and auxotrophy mutants. Table F lists often-used selectable markers for mammalian cell transfection.

Table F

| Useful selectable markers for eukaryote cell transfection | | | |
|---|---|---|---|
| **Selectable Marker** | **Selection** | **Action** | **Reference** |
| Adenosine deaminase (ADA) | Media includes 9-β-D-xylofuranosyl adenine (Xyl-A) | Conversion of Xyl-A to Xyl-ATP, which incorporates into nucleic acids, killing cells. ADA detoxifies | (Kaufman et al., 1986) |
| Dihydrofolate reductase (DHFR) | Methotrexate (MTX) and dialyzed serum (purine-free media) | MTX competitive inhibitor of DHFR. In absence of exogenous purines, cells require DHFR, a necessary enzyme in purine biosynthesis. | (Simonsen and Levinson, 1983) |

Table F (continued)

| Useful selectable markers for eukaryote cell transfection | | | |
|---|---|---|---|
| **Selectable Marker** | **Selection** | **Action** | **Reference** |
| Aminoglycoside phosphotransferase ("APH", "neo", "G418") | G418 | G418, an aminoglycoside detoxified by APH, interferes with ribosomal function and consequently, translation. | (Southern and Berg, 1982) |
| Hygromycin-B-phosphotransferase (HPH) | hygromycin-B | Hygromycin-B, an aminocyclitol detoxified by HPH, disrupts protein translocation and promotes mistranslation. | (Palmer et al., 1987) |
| Thymidine kinase (TK) | Forward selection (TK+): Media (HAT) incorporates aminopterin. Reverse selection (TK-): Media incorporates 5-bromodeoxyuridine (BrdU). | Forward: Aminopterin forces cells to synthesze dTTP from thymidine, a pathway requiring TK. Reverse: TK phosphorylates BrdU, which incorporates into nucleic acids, killing cells. | (Littlefield, 1964) |

A prokaryotic or eukaryotic host cell in culture can be used to produce CGI-69. Accordingly, *CGI-69* provides methods for producing CGI-69 using the host cells. In one embodiment, the method comprises culturing the host cell (into which a recombinant expression vector encoding CGI-69 has been introduced) in a suitable medium, such that CGI-69 is produced. In another embodiment, the method further comprises isolating CGI-69 from the medium or the host cell.

*Transgenic CGI-69 animals*

Transgenic animals are useful for studying the function and/or activity of *CGI-69* and for identifying and/or evaluating modulators of CGI-69 activity. "Transgenic animals" are non-human animals, preferably mammals, more preferably rodents such as rats or mice, in which one or more of the cells include a transgene. Other transgenic animals include primates, sheep, dogs, cows, goats, chickens, amphibians, *etc.* A "transgene" is exogenous DNA that is integrated into the genome of a cell from which a transgenic animal develops and remains in the genome of the mature animal. Transgenes preferably direct the expression of an encoded gene product in one or more cell types or tissues of the transgenic animal with the purpose of preventing expression of a naturally encoded gene product in one or more cell types or tissues (a "knockout" transgenic animal), or serving as a marker or indicator of an integration, chromosomal location, or region of recombination (*e.g. cre*/*loxP* mice). A "homologous recombinant animal" is a non-human animal, such as a rodent, in which endogenous *CGI-69* has been altered by an exogenous DNA molecule that recombines homologously with endogenous *CGI-69* in a *(e.g.* embryonic) cell prior to development the animal. Host cells with exogenous *CGI-69* can be used to produce non-human transgenic animals, such as fertilized oocytes or embryonic stem cells into which *CGI-69*-coding sequences have been introduced. Such host cells can then be used to create non-human transgenic animals or homologous recombinant animals.

**1. *Approaches to transgenic animal production***

A transgenic animal can be created by introducing *CGI-69* into the male pronuclei of a fertilized oocyte (*e.g.,* by microinjection, retroviral infection) and allowing the oocyte to develop in a pseudopregnant female foster animal (pffa). The *CGI-69* cDNA sequences (SEQ ID NO:1) can be introduced as a transgene into the genome of a non-human animal. Alternatively, a homologue of *CGI-69,* such as the naturally-occuring variant of *CGI-69*, can be used as a transgene. Intronic sequences and polyadenylation signals can also be included in the transgene to increase transgene expression. Tissue-specific regulatory sequences can be operably-linked to the *CGI-69* transgene to direct expression of *CGI-69* to particular cells. Methods for generating transgenic animals via embryo manipulation and microinjection, particularly animals such as mice, have become conventional in the art, *e.g.* (Evans et al., U.S. Patent No. 4,870,009, 1989; Hogan, 0879693843, 1994; Leder and Stewart, U.S. Patent No. 4,736,866,1988; Wagner and Hoppe, US Patent No. 4,873,191, 1989). Other non-mice transgenic animals may be made by similar methods. A transgenic founder animal, which can be used to breed additional transgenic animals, can be identified based upon the presence of the transgene in its genome and/or expression of the transgene mRNA in tissues or cells of the animals. Transgenic (*e.g. CGI-69*) animals can be bred to other transgenic animals carrying other transgenes.

A CGI-69 transgenic animal that is heterozyhous for the transgene may be bred with another heterozyhous CGI-69 transgenic animal to produce animals that are homozygous for the transgene. In certain instances, such homozygous transgenic animals may display different characteristics than the heterozygous parents. Thus, in situations where the heterozygous transgenic animal lacks a particular phenotype, such animals may still have substantial utility as sources of the homozyhous transgenic animals displaying a particular characteristic.

2. *Vectors for transgenic animal production*

To create a homologous recombinant animal, a vector containing at least a portion of *CGI-69* into which a deletion, addition or substitution has been introduced to thereby alter, *e.g.*, functionally disrupt, *CGI-69. CGI-69* can be a murine gene , or other *CGI-69* homologue, such as the human homolog (SEQ ID NO:1). In one approach, a knockout vector functionally disrupts the endogenous *CGI-69* gene upon homologous recombination, and thus a non-functional CGI-69 protein, if any, is expressed.

Alternatively, the vector can be designed such that, upon homologous recombination, the endogenous *CGI-69* is mutated or otherwise altered but still encodes functional protein (*e.g.,* the upstream regulatory region can be altered to thereby alter the expression of endogenous CGI-69). In this type of homologous recombination vector, the altered portion of the *CGI-69* is flanked at its 5'- and 3'-termini by additional nucleic acid of the *CGI-69* to allow for homologous recombination to occur between the exogenous *CGI-69* carried by the vector and an endogenous *CGI-69* in an embryonic stem cell. The additional flanking *CGI-69* nucleic acid is sufficient to engender homologous recombination with endogenous *CGI-69.* Typically, several kilobases of flanking DNA (both at the 5'- and 3'-termini) are included in the vector (Thomas and Capecchi, 1987). The vector is then introduced into an embryonic stem cell line *(e.g.,* by electroporation), and cells in which the introduced *CGI-69* has homologously-recombined with the endogenous *CGI-69* are selected (Li et al., 1992).

3. *Introduction of CGI-69 transgene cells during development*

Selected cells are then injected into a blastocyst of an animal *(e.g.,* a mouse) to form aggregation chimeras (Bradley, 1987). A chimeric embryo can then be implanted into a suitable pffa and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described (Berns et al., WO 93/04169, 1993; Bradley, 1991; Kucherlapati et al., WO 91/01140, 1991; Le Mouellic and Brullet, WO 90/11354, 1990).

Alternatively, transgenic animals that contain selected systems that allow for regulated expression of the transgene can be produced. An example of such a system is the *cre*/*loxP* recombinase system of bacteriophage P1 (Lakso et al., 1992). Another recombinase system is the FLP recombinase system of *Saccharomyces cerevisiae* (O'Gorman et al., 1991). If a *cre*/*loxP* recombinase system is used to regulate expression of the transgene, animals containing transgenes encoding both the *Cre* recombinase and a selected protein are required. Such animals can be produced as "double" transgenic animals, by mating an animal containing a transgene encoding a selected protein to another containing a transgene encoding a recombinase.

Clones of transgenic animals can also be produced (Wilmut et al., 1997). In brief, a cell from a transgenic animal can be isolated and induced to exit the growth cycle and enter $G_0$ phase. The quiescent cell can then be fused to an enucleated oocyte from an animal of the same species from which the quiescent cell is isolated. The reconstructed oocyte is then cultured to develop to a morula or blastocyte and then transferred to a pffa. The offspring borne of this female foster animal will be a clone of the "parent" transgenic animal.

*Pharmaceutical compositions*

Agonists or antagonists of CGI-69 can be incorporated into pharmaceutical compositions. Such compositions typically comprise the agonists or antagonists and a pharmaceutically acceptable carrier. A "pharmaceutically acceptable carrier" includes any and all solvents, dispersion media, coatings, antibacterial and antifungal agents, isotonic and absorption delaying agents, and the like, compatible with pharmaceutical administration (Gennaro, 2000). Preferred examples of such carriers or diluents include, but are not limited to, water, saline, Finger's solutions, dextrose solution, and 5% human serum albumin. Liposomes and non-aqueous vehicles such as fixed oils may also be used. Except when a conventional media or agent is incompatible with an active compound, use of these compositions is contemplated. Supplementary active compounds can also be incorporated into the compositions.

*1. General considerations*

A pharmaceutical composition of the agonist or antagonist is formulated to be compatible with its intended route of administration, including intravenous, intradermal, subcutaneous, oral (*e.g.;* inhalation), transdermal (*i.e*., topical), transmucosal, and rectal administration. Solutions or suspensions used for parenteral, intradermal, or subcutaneous application can include: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampules, disposable syringes or multiple dose vials made of glass or plastic.

*2. Injectable formulations*

Pharmaceutical compositions suitable for injection include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL™ (BASF, Parsippany, N.J.) or phosphate buffered saline (PBS). In all cases, the composition must be sterile and should be fluid so as to be administered using a syringe. Such compositions should be stable during manufacture and storage and must be preserved against contamination from microorganisms such as bacteria and fungi. The carrier can be a solvent or dispersion medium containing, for example, water, ethanol, polyol (such as glycerol, propylene glycol, and liquid polyethylene glycol), and suitable mixtures. Proper fluidity can be maintained, for example, by using a coating such as lecithin, by maintaining the required particle size in the case of dispersion and by using surfactants. Various antibacterial and antifungal agents, for example, parabens, chlorobutanol, phenol, ascorbic acid, and thimerosal, can contain microorganism contamination. Isotonic agents, for example, sugars, polyalcohols such as manitol, sorbitol, and sodium chloride can be included in the composition. Compositions that can delay absorption include agents such as aluminum monostearate and gelatin.

Sterile injectable solutions can be prepared by incorporating the active compound in the required amount in an appropriate solvent with one or a combination of ingredients as required, followed by sterilization. Generally, dispersions are prepared by incorporating the active compound into a sterile vehicle that contains a basic dispersion medium, and the other required ingredients. Sterile powders for the preparation of sterile injectable solutions, methods of preparation include vacuum drying and freeze-drying that yield a powder containing the active ingredient and any desired ingredient from a sterile solutions.

3. *Oral compositions*

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally. Pharmaceutically compatible binding agents, and/or adjuvant materials can be included. Tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, PRIMOGEL, or corn starch; a lubricant such as magnesium stearate or STEROTES; a glidant such as colloidal silicon dioxide; a sweetening agent such as sucrose or saccharin; or a flavoring agent such as peppermint, methyl salicylate, or orange flavoring.

4. *Compositions for inhalation*

For administration by inhalation, the compounds are delivered as an aerosol spray from a nebulizer or a pressurized container that contains a suitable propellant, *e.g.,* a gas such as carbon dioxide.

*5. Systemic administration*

Systemic administration can also be transmucosal or transdermal. For transmucosal or transdermal administration, penetrants that can permeate the target barrier(s) are selected. Transmucosal penetrants include, detergents, bile salts, and fusidic acid derivatives. Nasal sprays or suppositories can be used for transmucosal administration. For transdermal administration, the active compounds are formulated into ointments, salves, gels, or creams.

The compounds can also be prepared in the form of suppositories (*e.g.*, with bases such as cocoa butter and other glycerides) or retention enemas for rectal delivery.

*6. Carriers*

In one embodiment, the active compounds are prepared with carriers that protect the compound against rapid elimination from the body, such as a controlled release formulation, including implants and microencapsulated delivery systems. Biodegradable or biocompatible polymers can be used, such as ethylene vinyl acetate, polyanhydrides, polyglycolic acid, collagen, polyorthoesters, and polylactic acid. Such materials can be obtained commercially from ALZA Corporation (Mountain View, CA) and NOVA Pharmaceuticals, Inc. (Lake Elsinore, CA), or prepared by one of skill in the art. Liposomal suspensions can also be used as pharmaceutically acceptable carriers. These can be prepared according to methods known to those skilled in the art, such as in (Eppstein et al., US Patent No. 4,522,811,1985).

**7. *Unit dosage***

Oral formulations or parenteral compositions in unit dosage form can be created to facilitate administration and dosage uniformity. Unit dosage form refers to physically discrete units suited as single dosages for the subject to be treated, containing a therapeutically effective quantity of active compound in association with the required pharmaceutical carrier. The specification for the unit dosage forms of the invention are dictated by, and directly dependent on, the unique characteristics of the active compound and the particular desired therapeutic effect, and the inherent limitations of compounding the active compound.

8. *Gene therapy compositions*

The nucleic acid molecules of *CGI-69* can be inserted into vectors and used as gene therapy vectors. Gene therapy vectors are suitable for delivery to a subject by, for example, intravenous injection, local administration (Nabel and Nabel, US Patent No. 5,328,470, 1994), or by stereotactic injection (Chen et al., 1994). The pharmaceutical preparation of a gene therapy vector can include an acceptable diluent, or can comprise a slow release matrix in which the gene delivery vehicle is imbedded. Alternatively, where the complete gene delivery vector can be produced intact from recombinant cells, *e.g.*, retroviral vectors, the pharmaceutical preparation can include one or more cells that produce the gene delivery system.

9. *Dosage*

The pharmaceutical composition may further comprise other therapeutically active compounds as noted herein which are usually applied in the treatment of CGI-69 -related conditions.

An appropriate dosage level of an agonist or antagonist in a medicament for the treatment or prevention of conditions which reguire CGI-69 modulation will generally be about 0.01 to 500 mg per kg patient body weight per day which can be for administration in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0. 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day.

However, the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

10. *Kits for pharmaceutical compositions*

The pharmaceutical compositions can be included in a kit, container, pack, or dispenser together with instructions for administration to treat a metabolic disorder or disease. When the invention is supplied as a kit, the different components of the composition may be packaged in separate containers and admixed immediately before use. Such packaging of the components separately may permit long-term storage without losing the active components' functions.

Kits may also include reagents in separate containers that facilitate the execution of a specific test, such as diagnostic tests or tissue typing. For example, *CGI-69* DNA templates and suitable primers may be supplied for internal controls.

(a) *Containers or vessels*

The reagents included in kits can be supplied in containers of any sort such that the life of the different components are preserved, and are not adsorbed or altered by the materials of the container. For example, sealed glass ampules may contain lyophilized CGI-69 or buffer that have been packaged under a neutral, non-reacting gas, such as nitrogen. Ampules may consist of any suitable material, such as glass, organic polymers, such as polycarbonate, polystyrene, *etc.,* ceramic, metal or any other material typically employed to hold reagents. Other examples of suitable containers include simple bottles that may be fabricated from similar substances as ampules, and envelopes, that may consist of foil-lined interiors, such as aluminum or an alloy. Other containers include test tubes, vials, flasks, bottles, syringes, or the like. Containers may have a sterile access port, such as a bottle having a stopper that can be pierced by a hypodermic injection needle. Other containers may have two compartments that are separated by a readily removable membrane that upon removal permits the components to mix. Removable membranes may be glass, plastic, rubber, *etc.*

(b) *Instructional materials*

Kits may also be supplied with instructional materials. Instructions may be printed on paper or other substrate, and/or may be supplied as an electronic-readable medium, such as a floppy disc, CD-ROM, DVD-ROM, Zip disc, videotape, laserdisc, audio tape, *etc.* Detailed instructions may not be physically associated with the kit; instead, a user may be directed to an internet web site specified by the manufacturer or distributor of the kit, or supplied as electronic mail.

***Screening and detection methods***

The isolated nucleic acid molecules of *CGI-69* can be used to express CGI-69 (*e.g.*, via a recombinant expression vector in a host cell in gene therapy applications), to detect *CGI-69* mRNA (*e.g.*, in a biological sample) or a genetic lesion in a CGI-69, and to modulate CGI-69 activity, as described below. In addition, CGI-69 polypeptides can be used to screen drugs or compounds that modulate the CGI-69 activity or expression as well as for the manufacture of a medicament for treating disorders characterized by insufficient or excessive production of CGI-69 or production of CGI-69 forms that have decreased or aberrant activity compared to CGI-69 wild-type protein, or modulate biological function that involve CGI-69. These reagents may be provided in the form of a kit as described above for pharmaceutical administrations optionally including instructions for assaying or screening CGI-69 involvement in a metabolic disease or disorder.

1. *Screening assays*

The invention provides a method (screening assay) for identifying modalities, i.e., candidate or test compounds or agents (*e.g.,* peptides, peptidomimetics, small molecules or other drugs), foods, combinations thereof, *etc.,* that effect CGI-69, a stimulatory or inhibitory effect, including translation, transcription, activity or copies of the gene in cells. The invention also includes compounds identified in screening assays.

Testing for compounds that increase or decrease CGI-69 activity is desirable. A compound may modulate CGI-69 activity by affecting: (1) the number of copies of the gene in the cell (amplifiers and deamplifiers); (2) increasing or decreasing transcription of the *CGI-69* (transcription up-regulators and down-regulators); (3) by increasing or decreasing the translation of *CGI-69* mRNA into protein (translation up-regulators and down-regulators); or (4) by increasing or decreasing the activity of CGI-69 itself (agonists and antagonists).

(a) *effects of compounds*

To identify compounds that affect CGI-69 at the DNA, RNA and protein levels, cells or organisms are contacted in vitro with a candidate compound and the corresponding change in CGI-69 DNA, RNA or protein is assessed (Ausubel et al., 1987). For DNA amplifiers and deamplifiers, the amount of *CGI-69* DNA is measured, for those compounds that are transcription up-regulators and down-regulators, the amount of *CGI-69* mRNA is determined; for translational up- and down-regulators, the amount of CGI-69 polypeptides is measured. Compounds that are agonists or antagonists may be identified by contacting cells or organisms with the compound.

Many assays for screening candidate or test compounds that bind to or modulate the activity of CGI-69 or polypeptide or biologically active portion are available. Test compounds can be obtained using any of the numerous approaches in combinatorial library methods, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptides, while the other four approaches encompass peptide, non-peptide oligomer or small molecule libraries of compounds (Lam, 1997).

(b) *small molecules*

A "small molecule" refers to a composition that has a molecular weight of less than about 5 kD and more preferably less than about 4 kD, and most preferably less than 0.6 kD. Small molecules can be, nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention. Examples of methods for the synthesis of molecular libraries have been described (Carell et al., 1994a; Carell et al., 1994b; Cho et al., 1993; DeWitt et al., 1993; Gallop et al., 1994; Zuckermann et al., 1994).

Libraries of compounds may be presented in solution (Houghten et al., 1992) or on beads (Lam et al., 1991), on chips (Fodor et al., 1993), bacteria, spores (Ladner et al., US Patent No. 5,223,409, 1993), plasmids (Cull et al., 1992) or on phage (Cwirla et al.; 1990; Devlin et al., 1990; Felici et al., 1991; Ladner et al., US Patent No. 5,223,409, 1993; Scott and Smith, 1990). A cell-free assay comprises contacting CGI-69 or biologically-active fragment with a known compound that binds CGI-69 to form an assay mixture, contacting the assay mixture with a test compound, and determining the ability of the test compound to interact with CGI-69, where determining the ability of the test compound to interact with CGI-69 comprises determining the ability of the CGI-69 to preferentially bind to or modulate the activity of an CGI-69 target molecule.

(c) *cell-free assays*

The cell-free assays of the invention may be used with the membrane-bound forms of CGI-69. Cell-free assays comprising the membrane-bound form, a solubilizing agent may be used to maintain CGI-69 in solution. Examples of such solubilizing agents include non-ionic detergents such as n-octylglucoside, n-dodecylglucoside, n-dodecylmaltoside, octanoyl-N-methylglucamide, decanoyl-N-methylglucamide, polyoxyethylene ethers (*e.g.*, t-Octylphenoxypolyethoxyethanol (TRITON® X-100) and others from the TRITON® series), polyoxyethylene 9 lauryl ether (THESIT®), Isotridecypoly(ethylene glycol ether)$_n$, N-dodecyl-N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

(d) *immobilization of target molecules to facilitate screening*

In more than one embodiment of the assay methods, immobilizing either CGI-69 or its partner molecules can facilitate separation of complexed from uncomplexed forms of one or both of the proteins, as well as to accommodate high throughput assays. Binding of a test compound to CGI-69, or interaction of CGI-69 with a target molecule in the presence and absence of a candidate compound, can be accomplished in any vessel suitable for containing the reactants, such as microtiter plates, test tubes, and micro-centrifuge tubes. A fusion protein can be provided that adds a domain that allows one or both of the proteins to be bound to a matrix. For example, GST (glutathione S-transferase) -CGI-69 fusion proteins or GST-target fusion proteins can be adsorbed onto glutathione sepharose beads (SIGMA Chemical, St. Louis, MO) or glutathione derivatized microtiter plates that are then combined with the test compound or the test compound and either the non-adsorbed target protein or CGI-69, and the mixture is incubated under conditions conducive to complex formation (*e.g.,* at physiological conditions for salt and pH). Following incubation, the beads or microtiter plate wells are washed to remove any unbound components, the matrix immobilized in the case of beads, complex determined either directly or indirectly. Alternatively, the complexes can be dissociated from the matrix, and the level of CGI-69 binding or activity determined using standard techniques.

Other techniques for immobilizing proteins on matrices can also be used in screening assays. Either CGI-69 or its target molecule can be immobilized using biotin-avidin or biotin-streptavidin systems. Biotinylation can be accomplished using many reagents, such as biotin-NHS (N-hydroxy-succinimide; PIERCE Chemicals, Rockford, IL), and immobilized in wells of streptavidin-coated 96 well plates (PIERCE Chemical). Alternatively, Abs reactive with CGI-69 or target molecules but do not interfere with binding of the CGI-69 to its target molecule can be derivatized to the wells of the plate, and unbound target or CGI-69 trapped in the wells by antibody conjugation. Methods for detecting such complexes, in addition to those described for the GST-immobilized complexes, include immunodetection of complexes using Abs reactive with CGI-69 or its target, as well as enzyme-linked assays that rely on detecting an enzymatic activity associated with the CGI-69 or target molecule.

(e) *screens to identify modulators*

Modulators of CGI-69 expression can be identified in a method where a cell is contacted with a candidate compound and the expression of CGI-69 mRNA or protein in the cell is determined. The expression level of CGI-69 mRNA or protein in the presence of the candidate compound is compared to CGI-69 mRNA or protein levels in the absence of the candidate compound. The candidate compound can then be identified as a modulator of CGI-69 mRNA or protein expression based upon this comparison. For example, when expression of CGI-69 mRNA or protein is greater (*i.e*., statistically significant) in the presence of the candidate compound than in its absence, the candidate compound is identified as a stimulator of CGI-69 mRNA or protein expression. Alternatively, when expression of CGI-69 mRNA or protein is less (statistically significant) in the presence of the candidate compound than in its absence, the candidate compound is identified as an inhibitor of CGI-69 mRNA or protein expression. The level of CGI-69 mRNA or protein expression in the cells can be determined by methods described for detecting CGI-69 mRNA or protein.

(f) *hybrid assays*

In yet another aspect of the invention, CGI-69 can be used as "bait" in two-hybrid or three hybrid assays (Bartel et al., 1993; Brent et al., WO94/10300, 1994; Iwabuchi et al., 1993; Madura et al., 1993; Saifer et al., US Patent No. 5,283,317, 1994; Zervos et al., 1993) to identify other proteins that bind or interact with CGI-69 and modulate CGI-69 activity. Such CGI-69-interacting partner proteins are also likely to be involved in the propagation of signals by the CGI-69 as, for example, upstream or downstream elements of a CGI-69 pathway.

The two-hybrid system is based on the modular nature of most transcription factors, which consists of separable DNA-binding and activation domains. The assay utilizes two different DNA constructs. In one construct, the gene that codes for CGI-69 is fused to a gene encoding the DNA binding domain of a known transcription factor (*e.g*., GAL4). The other construct, a DNA sequence from a library of DNA sequences that encodes an unidentified protein ("prey" or "sample") is fused to a gene that codes for the activation domain of the known transcription factor. If the "bait" and the "prey" proteins are able to interact in vivo, forming a CGI-69 -dependent complex, the DNA-binding and activation domains of the transcription factor are brought into close proximity. This proximity allows transcription of a reporter gene (*e.g.,* LacZ) that is operably-linked to a transcriptional regulatory site responsive to the transcription factor. Expression of the reporter gene can be detected, and cell colonies containing the functional transcription factor can be isolated and used to obtain the cloned gene that encodes the CGI-69 -interacting protein. The invention further pertains to novel agents identified by the aforementioned screening assays and uses thereof for treatments as described herein.

*Predictive medicine*

The invention also pertains to the field of predictive medicine in which diagnostic assays, prognostic assays, pharmacogenomics, and monitoring clinical trials are used for prognostic (predictive) purposes.

Accordingly, one aspect of the invention relates to diagnostic assays for determining CGI-69 and/or nucleic acid expression as well as CGI-69 activity, in the context of a biological sample (*e.g*., blood, serum, cells, tissue) to determine whether an individual is afflicted with a disease or disorder, or is at risk of developing a disorder, associated with aberrant CGI-69 expression or activity, including cancer. The invention also provides for prognostic (or predictive) assays for determining whether an individual is at risk of developing a disorder associated with CGI-69, nucleic acid expression or activity. For example, mutations in *CGI-69* can be assayed in a biological sample. Such assays can be used for prognostic or predictive purpose to prophylactically treat an individual prior to the onset of a disorder characterized by or associated with CGI-69 nucleic acid expression, or biological activity.

Another aspect of the invention provides methods for determining CGI-69 activity, or nucleic acid expression, in an individual to select appropriate therapeutic or prophylactic agents for that individual (referred to herein as "pharmacogenomics"). Pharmacogenomics allows for the selection of modalities (*e.g.,* drugs, foods) for therapeutic or prophylactic treatment of an individual based on the individual's genotype (*e.g*., the individual's genotype to determine the individual's ability to respond to a particular agent). Another aspect of the invention pertains to monitoring the influence of modalities (*e.g.,* drugs, foods) on the expression or activity of CGI-69 in clinical trials.

### 1. *Diagnostic assays*

An exemplary method for detecting the presence or absence of CGI-69 in a biological sample involves obtaining a biological sample from a subject and contacting the biological sample with a compound or an agent capable of detecting CGI-69 or *CGI-69* (*e.g.,* mRNA, genomic DNA) such that the presence of CGI-69 is confirmed in the sample. An agent for detecting *CGI-69* mRNA or genomic DNA is a labeled nucleic acid probe that can hybridize to *CGI-69* mRNA or genomic DNA. The nucleic acid probe can be, for example, a full-length *CGI-69* nucleic acid, such as the nucleic acid of SEQ ID NO:1, or a portion thereof, such as an oligonucleotide of at least 15, 30, 50, 100, 250 or 500 nucleotides in length and sufficient to specifically hybridize under stringent conditions to *CGI-69* mRNA or genomic DNA.

The term "biological sample" includes tissues, cells and biological fluids isolated from a subject. The detection method of the invention can be used to detect *CGI-69* mRNA, protein, or genomic DNA in a biological sample *in vitro*. For example, *in vitro* techniques for detection of *CGI-69* mRNA include Northern hybridizations and *in situ* hybridizations. *In vitro* techniques for detection of CGI-69 polypeptide include enzyme linked immunosorbent assays (ELISAs), Western blots, immunoprecipitations, and immunofluorescence. *In vitro* techniques for detection of *CGI-69* genomic DNA include Southern hybridizations and fluorescent *in situ* hybridization (FISH).

In one embodiment, the biological sample from the subject contains protein molecules, and/or mRNA molecules, and/or genomic DNA molecules. Preferred biological samples are blood and adipose tissue.

In another embodiment, the methods further involve contacting a control sample from a subject with a compound or agent to detect CGI-69, *CGI-69* mRNA, or genomic DNA, and comparing the presence of CGI-69, *CGI-69* mRNA or genomic DNA in the control sample with the presence of CGI-69, *CGI-69* mRNA or genomic DNA in the test sample.

The invention also encompasses kits for detecting CGI-69 in a biological sample. For example, the kit can comprise: a labeled compound or agent capable of detecting CGI-69 or *CGI-69* mRNA in a sample; reagent and/or equipment for determining the amount of CGI-69 in the sample; and reagent and/or equipment for comparing the amount of CGI-69 in the sample with a standard. The compound or agent can be packaged in a suitable container. The kit can further comprise instructions for using the kit to detect CGI-69 or nucleic acid.

### 2. *Prognostic assays*

The diagnostic methods described herein can furthermore be utilized to identify subjects having or at risk of developing a disease or disorder associated with aberrant CGI-69 expression or activity. For example, the described assays can be used to identify a subject having or at risk of developing a disorder associated with CGI-69, nucleic acid expression or activity. Alternatively, the prognostic assays can be used to identify a subject having or at risk for developing a disease or disorder. The invention provides a method for identifying a disease or disorder associated with aberrant CGI-69 expression or activity in which CGI-69 or nucleic acid (*e.g.,* mRNA, genomic DNA) is detected in a test sample from a subject. A test sample is a biological sample from a subject. For example, a test sample can be a biological fluid (*e.g.,* serum), cell sample, or tissue.

Prognostic assays can be used to determine whether a subject can be administered a modality (*e.g.,* an agonist, antagonist, peptidomimetic, protein, peptide, nucleic acid, small molecule, food, *etc.*) to treat a disease or disorder associated with aberrant CGI-69 expression or activity. Such methods can be used to determine whether a subject can be effectively treated with an agent for a disorder. The invention provides methods for determining whether a subject can be effectively treated with an agent for a disorder associated with aberrant CGI-69 expression or activity in which CGI-69 or nucleic acid is detected in a test sample (*e.g.,* where the presence of CGI-69 or nucleic acid is diagnostic for a subject that can be administered the agent to treat a disorder associated with aberrant CGI-69 expression or activity).

The methods of the invention can also be used to detect genetic lesions in a *CGI-69* to determine if a subject with the genetic lesion is at risk for a disorder. Methods include detecting, in a sample from the subject, the presence or absence of a genetic lesion characterized by at an alteration affecting the integrity of a gene encoding a CGI-69 polypeptide, or the mis-expression of *CGI-69.* Such genetic lesions can be detected by ascertaining: (1) a deletion of one or more nucleotides from *CGI-69;* (2) an addition of one or more nucleotides to *CGI-69;* (3) a substitution of one or more nucleotides in *CGI-69*, (4) a chromosomal rearrangement of an *CGI-69* gene; (5) an alteration in the level of an *CGI-69* mRNA transcripts, (6) aberrant modification of an *CGI-69,* such as a change genomic DNA methylation, (7) the presence of a non-wild-type splicing pattern of an *CGI-69* mRNA transcript, (8) a non-wild-type level of *CGI-69,* (9) allelic loss of *CGI-69*, and/or (10) inappropriate post-translational modification of CGI-69 polypeptide. There are a large number of known assay techniques that can be used to detect lesions in *CGI-69.* Any biological sample containing nucleated cells may be used.

In certain embodiments, lesion detection may use a probe/primer in a polymerase chain reaction (PCR) (*e. g.,* (Mullis, US Patent No. 4,683,202, 1987; Mullis et al., US Patent No. 4,683,195, 1987), such as anchor PCR or rapid amplification of cDNA ends (RACE) PCR, or, alternatively, in a ligation chain reaction (LCR) (*e.g.,* (Landegren et al., 1988; Nakazawa et al., 1994), the latter is particularly useful for detecting point mutations in *CGI-69*-genes (Abravaya et al., 1995). This method may include isolating nucleic acids from a sample from a subject, contacting the nucleic acids with one or more primers that specifically hybridize to *CGI-69* under conditions such that hybridization and amplification of the *CGI-69* (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al., 1990), transcriptional amplification system (Kwoh et al., 1989); Qβ Replicase (Lizardi et al., 1988), or any other nucleic acid amplification method, followed by the detection of the amplified molecules using techniques well known to those of skill in the art. These detection schemes are especially useful for the detection of nucleic acid molecules present in low abundance.

Mutations in *CGI-69* from a sample can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes can be used to score for the presence of specific mutations by development or loss of a ribozyme cleavage site.

Hybridizing a sample and control nucleic acids, *e.g.,* DNA or RNA, to high-density arrays containing hundreds or thousands of oligonucleotides probes, can identify genetic mutations in *CGI-69* (Cronin et al., 1996; Kozal et al., 1996). For example, genetic mutations in *CGI-69* can be identified in two-dimensional arrays containing light-generated DNA probes as described (Cronin et al., 1996). Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the *CGI-69* and detect mutations by comparing the sequence of the sample *CGI-69* with the corresponding wild-type (control) sequence. Examples of sequencing reactions include those based on classic techniques (Maxam and Gilbert, 1977; Sanger et al., 1977). Any of a variety of automated sequencing procedures can be used when performing diagnostic assays (Naeve et al., 1995) including sequencing by mass spectrometry (Cohen et al., 1996; Griffin and Griffin, 1993; Koster, WO94/16101, 1994).

Other methods for detecting mutations in the *CGI-69* include those in which protection from cleavage agents is used to detect mismatched bases in RNA/RNA or RNA/DNA heteroduplexes (Myers et al., 1985). In general, the technique of "mismatch cleavage" starts by providing heteroduplexes formed by hybridizing (labeled) RNA or DNA containing the wild-type *CGI-69* sequence with potentially mutant RNA or DNA obtained from a sample. The double-stranded duplexes are treated with an agent that cleaves single-stranded regions of the duplex such as those that arise from base pair mismatches between the control and sample strands. For instance, RNA/DNA duplexes can be treated with RNase and DNA/DNA hybrids treated with $S_1$ nuclease to enzymatically digest the mismatched regions. In other embodiments, either DNA/DNA or RNA/DNA duplexes can be treated with hydroxylamine or osmium tetroxide and with piperidine in order to digest mismatched regions. The digested material is then separated by size on denaturing polyacrylamide gels to determine the mutation site (Grompe et al., 1989; Saleeba and Cotton, 1993). The control DNA or RNA can be labeled for detection.

Mismatch cleavage reactions may employ one or more proteins that recognize mismatched base pairs in double-stranded DNA (DNA mismatch repair) in defined systems for detecting and mapping point mutations in *CGI-69* cDNAs obtained from samples of cells. For example, the mutY enzyme of *E. coli* cleaves A at G/A mismatches and the thymidine DNA glycosylase from HeLa cells cleaves T at G/T mismatches (Hsu et al., 1994). According to an exemplary embodiment, a probe based on a wild-type *CGI-69* sequence is hybridized to a cDNA or other DNA product from a test cell(s). The duplex is treated with a DNA mismatch repair enzyme, and the cleavage products, if any, can be detected from electrophoresis protocols or the like (Modrich et al., US Patent No. 5,459,039, 1995).

Electrophoretic mobility alterations can be used to identify mutations in *CGI-69.* For example, single strand conformation polymorphism (SSCP) may be used to detect differences in electrophoretic mobility between mutant and wild type nucleic acids (Cotton, 1993; Hayashi, 1992; Orita et al., 1989). Single-stranded DNA fragments of sample and control *CGI-69* nucleic acids are denatured and then renatured. The secondary structure of single-stranded nucleic acids varies according to sequence; the resulting alteration in electrophoretic mobility allows detection of even a single base change. The DNA fragments may be labeled or detected with labeled probes. The sensitivity of the assay may be enhanced by using RNA (rather than DNA), in which the secondary structure is more sensitive to a sequence changes. The method may use heteroduplex analysis to separate double stranded heteroduplex molecules on the basis of changes in electrophoretic mobility (Keen et al., 1991).

The migration of mutant or wild-type fragments can be assayed using denaturing gradient gel electrophoresis (DGGE; (Myerset al., 1985). In DGGE, DNA is modified to prevent complete denaturation, for example by adding a GC clamp of approximately 40 bp of high-melting GC-rich DNA by PCR. A temperature gradient may also be used in place of a denaturing gradient to identify differences in the mobility of control and sample DNA (Rossiter and Caskey, 1990).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found (Saiki et al., 1986; Saiki et al., 1989). Such allele-specific oligonucleotides are hybridized to PCR-amplified target DNA or a number of different mutations when the oligonucleotides are attached to the hybridizing membrane and hybridized with labeled target DNA.

Alternatively, allele specific amplification technology that depends on selective PCR amplification may be used. Oligonucleotide primers for specific amplifications may carry the mutation of interest in the center of the molecule (so that amplification depends on differential hybridization (Gibbs et al., 1989)) or at the extreme 3'-terminus of one primer where, under appropriate conditions, mismatch can prevent, or reduce polymerase extension (Prosser, 1993). Novel restriction site in the region of the mutation may be introduced to create cleavage-based detection (Gasparini et al., 1992). Certain amplification may also be performed using *Taq* ligase for amplification (Barany, 1991). In such cases, ligation occurs only if there is a perfect match at the 3'-terminus of the 5' sequence, allowing detection of a known mutation by scoring for amplification.

The described methods may be performed, for example, by using pre-packaged kits comprising at least one probe (nucleic acid or antibody) that may be conveniently used, for example, in clinical settings to diagnose patients exhibiting symptoms or family history of a disease or illness involving CGI-69.

Furthermore, any cell type or tissue in which CGI-69 is expressed may be utilized in vitro in the prognostic assays described herein.

3. *Pharmacogenomics*

Agents, or modulators that have a stimulatory or inhibitory effect on CGI-69 activity or expression, as identified by a screening assay, can be used for manufacture of a medicament for treating prophylactically or therapeutically disorders. In conjunction with such treatment, the pharmacogenomics (*i.e.*, the study of the relationship between a subject's genotype and the subject's response to a foreign modality, such as a food, compound or drug) may be considered Metabolic differences of therapeutics can lead to severe toxicity or therapeutic failure by altering the relation between dose and blood concentration of the pharmacologically active drug. Thus, the pharmacogenomics of the individual permits the selection of effective agents (*e.g.,* drugs) for prophylactic or therapeutic treatments based on a consideration of the individual's genotype. Pharmacogenomics can further be used to determine appropriate dosages and therapeutic regimens. Accordingly, the activity of CGI-69 , expression of *CGI-69,* or *CGI-69* mutation(s) in an individual can be determined to guide the selection of appropriate agent(s) for therapeutic or prophylactic treatment.

Pharmacogenomics deals with clinically significant hereditary variations in the response to modalities due to altered modality disposition and abnormal action in affected persons (Eichelbaum and Evert, 1996; Under et al., 1997). In general, two pharmacogenetic conditions can be differentiated: (1) genetic conditions transmitted as a single factor altering the interaction of a modality with the body (altered drug action) or (2) genetic conditions transmitted as single factors altering the way the body acts on a modality (altered drug metabolism). These pharmacogenetic conditions can occur either as rare defects or as nucleic acid polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g.,* N-acetyltransferase 2 (NAT 2) and cytochrome P450 enzymes CYP2D6 and CYP2C19) explains the phenomena of some patients who show exaggerated drug response and/or serious toxicity after taking the standard and safe dose of a drug. These polymorphisms are expressed in two phenotypes in the population, the extensive metabolizer (EM) and poor metabolizer (PM). The prevalence of PM is different among different populations. For example, the *CYP2D6* gene is highly polymorphic and several mutations have been identified in PM, which all lead to the absence of functional CYP2D6. Poor metabolizers due to mutant *CYP2D6* and *CYP2C19* frequently experience exaggerated drug responses and side effects when they receive standard doses. If a metabolite is the active therapeutic moiety, PM shows no therapeutic response, as demonstrated for the analgesic effect of codeine mediated by its CYP2D6-formed metabolite morphine. At the other extreme are the so-called ultra-rapid metabolizers who are unresponsive to standard doses. Recently, the molecular basis of ultra-rapid metabolism has been identified to be due to CYP2D6 gene amplification.

The activity of CGI-69 , expression of *CGI-69* nucleic acid, or mutation content of *CGI-69* in an individual can be determined to select appropriate agent(s) useful for manufacture of medicaments for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with an CGI-69 modulator, such as a modulator identified by one of the described exemplary screening assays.

**4. *Monitoring effects during clinical trials***

Monitoring the influence of agents (*e.g.*, drugs, compounds) on the expression or activity of CGI-69 can be applied not only in basic drug screening, but also in clinical trials. For example, the effectiveness of an agent determined by a screening assay to increase *CGI-69* expression, protein levels, or up-regulate CGI-69 activity can be monitored in clinical trails of subjects exhibiting decreased *CGI-69* expression, protein levels, or down-regulated CGI-69 activity. Alternatively, the effectiveness of an agent determined to decrease *CGI-69* expression, protein levels, or down-regulate CGI-69 activity, can be monitored in clinical trails of subjects exhibiting increased *CGI-69* expression, protein levels, or up-regulated CGI-69 activity. In such clinical trials, the expression or activity of CGI-69 and, preferably, other genes that have been implicated in, for example, cancer can be used as a "read out" or markers for a particular cell's responsiveness.

For example, genes, including *CGI-69*, that are modulated in cells by treatment with a modality (*e.g.,* food, compound, drug or small molecule) can be identified. To study the effect of agents on metabolic disorders or disorders associated with changes in adipose tissue physiological function or mass, for example, in a clinical trial, cells can be isolated and RNA prepared and analyzed for the levels of expression of *CGI-69* and other genes implicated in the disorder. The gene expression pattern can be quantified by Northern blot analysis, nuclear run-on or RT-PCR experiments, or by measuring the amount of protein, or by measuring the activity level of CGI-69 or other gene products. In this manner, the gene expression pattern itself can serve as a marker, indicative of the cellular physiological response to the agent. Accordingly, this response state may be determined before, and at various points during, treatment of the individual with the agent.

The invention provides a method for monitoring the effectiveness of treatment of a subject with an agent (*e. g.*, an agonist, antagonist, protein, peptide, peptidomimetic, nucleic acid, small molecule, food or other drug candidate identified by the screening assays described herein) comprising the steps of (1) detecting the level of expression of an CGI-69, *CGI-69* mRNA, or genomic DNA in a preadministration sample from a subject; (2) detecting the level of expression or activity of the CGI-69 . *CGI-69* mRNA, or genomic DNA in one or more post-administration samples from the subject; (3) comparing the level of expression or activity of the CGI-69, *CGI-69* mRNA, or genomic DNA in the pre-administration sample with the CGI-69, *CGI-69* mRNA, or genomic DNA in the post administration sample or samples; and (4) altering the administration of the agent to the subject accordingly. For example, increased administration of the agent may be desirable to increase the expression or activity of CGI-69 to higher levels than detected, *i.e.*, to increase the effectiveness of the agent. Alternatively, decreased administration of the agent may be desirable to decrease expression or activity of CGI-69 to lower levels than detected, *i.e.*, to decrease the effectiveness of the agent.

5. Therapeutic uses

The invention provides for both prophylactic and therapeutic uses for the treatment of a subject at risk of (or susceptible to) a disorder or having a disorder associated with aberrant CGI-69 expression or activity. Examples include disorders in which cell metabolic demands (and consequently, demands on mitochondria and endoplasmic reticulum) are high, such as during rapid cell growth. Examples of such disorders and diseases include metabolic disorders or disorders associated with changes in adipose tissue physiological function or mass.

### 6. *Disease and disorders*

The CGI-69 compositions of the present invention are useful for the manufacture of medicaments for treating, or for diagnosing diseases and disorders including obesity, cachexia, tumors, cancers, and fever associated with viral infections and bacterial infections.

Therapeutics that antagonize (*i.e.*, reduce or inhibit) activity may be useful for the manufacture of medicaments for treating diseases and disorders that are characterized by increased CGI-69 levels or biological activity. Antagonists may be for administration in a therapeutic or prophylactic manner. Therapeutics that may be used include: (1) CGI-69 peptides, or analogs, derivatives, fragments or homologs thereof; (2) *CGI-69*; (3) antisense nucleic acid and nucleic acids that are "dysfunctional" (*i.e.*, due to a heterologous insertion within the coding sequences) that are used to eliminate endogenous function of by homologous recombination (Capecchi, 1989); or (4) modulators (*i.e.*, inhibitors, agonists and antagonists, including additional peptide mimetic of the invention) that alter the interaction between CGI-69 and its binding partner.

Therapeutics that increase (*i.e.*, are agonists to) activity may be useful for the manufacture of medicaments for treating diseases and disorders that are characterized by decreased CGI-69 levels or biological activity, such as obesity. Therapeutics that upregulate activity may be for administration therapeutically or prophylactically. Therapeutics that may be used include peptides, or analogs, derivatives, fragments or homologs thereof; or an agonist that increases bioavailability.

Increased or decreased levels can be readily detected by quantifying peptide and/or RNA, by assaying *in vitro* for RNA or peptide levels, structure and/or activity of the expressed peptides (or *CGI-69* mRNAs)in a patient tissue sample (*e.g.*, from biopsy tissue) . Methods include, but are not limited to, immunoassays *(e.g.,* by Western blot analysis, immunoprecipitation followed by sodium dodecyl sulfate (SDS) polyacrylamide gel electrophoresis, immunocytochemistry, *etc.)* and/or hybridization assays to detect expression of mRNAs (*e.g.*, Northern assays, dot blots, *in situ* hybridization, and the like).

### 7. *Prophylactic methods*

The invention provides use of an agent that modulates CGI-69 expression or at least one CGI-69 activity, for manufacture of a medicament for preventing, in a subject, a disease or condition associated with an aberrant CGI-69 expression or activity. Subjects at risk for a disease that is caused or contributed to by aberrant CGI-69 expression or activity can be identified by, for example, any or a combination of diagnostic or prognostic assays. A prophylactic agent may be for administration prior to the manifestation of symptoms characteristic of the CGI-69 aberrancy, such that a disease or disorder is prevented or, alternatively, delayed in its progression. Depending on the type of CGI-69 aberrancy, for example, a CGI-69 agonist or CGI-69 antagonist can be used for manufacture of a medicament for treament of the subject. The appropriate agent can be determined based on screening assays.

### *8. Therapeutic uses*

Another aspect of the invention pertains to uses of modulators for modulating CGI-69 expression or activity for therapeutic purposes. The modulatory method of the invention involves contacting a cell in vitro with an agent that modulates one or more of the activities of CGI-69 activity associated with the cell. An agent that modulates CGI-69 activity can be a nucleic acid or a protein, a naturally occurring cognate ligand of CGI-69, a peptide, a CGI-69 peptidomimetic, or other small molecule. The agent may stimulate CGI-69 activity. Examples of such stimulatory agents include active CGI-69 and a *CGI-69* that has been introduced into the cell. In another embodiment, the agent inhibits CGI-69 activity. An example of an inhibitory agent includes antisense *CGI-69* nucleic acids. Modulatory methods can be performed *in vitro* (*e.g.*, by culturing the cell with the agent) The invention provides the use of agents for manufacture of medicaments for treating an individual afflicted with a disease or disorder characterized by aberrant expression or activity of a CGI-69 or nucleic acid molecule. In one embodiment, the invention involves use of an agent (*e.g.,* an agent identified by a screening assay), or combination of agents that modulates (*e.g.*, up-regulates or down-regulates) CGI-69 expression or activity. In another embodiment, the invention involves use of a CGI-69 or nucleic acid molecule for manufacture of a medicament to compensate for reduced or aberrant CGI-69 expression or activity.

Stimulation of CGI-69 activity is desirable in situations in which CGI-69 is abnormally down-regulated and/or in which increased CGI-69 activity is likely to have a beneficial effect; for example, in obesity. Conversely, diminished CGI-69 activity is desired in conditions in which CGI-69 activity is abnormally up-regulated and/or in which decreased CGI-69 activity is likely to to have a beneficial effect; for example, in cachexia.

**9. *Determination of the biological effect of the therapeutic***

Suitable *in vitro* assays can be performed to determine the effect of a specific therapeutic and whether it is useful for treatment of the affected tissue.

In various specific embodiments, *in vitro* assays may be performed with representative cells of the type(s) involved in the patient's disorder, to determine if a given therapeutic exerts the desired effect upon the cell type(s). Modalities for use in therapy may be tested in suitable animal model systems including, but not limited to rats, mice, chicken, cows, monkeys, rabbits, dogs and the like, prior to testing in human subjects.

***10. Prophylactic and therapeutic uses of the compositions of the invention***

CGI-69 nucleic acids and proteins are useful in potential prophylactic and therapeutic applications implicated in a variety of disorders including, but not limited to metabolic disorders or disorders associated with changes in adipose tissue physiological function or mass.

As an example, a cDNA encoding CGI-69 may be useful in gene therapy, and the protein may be useful when administered to a subject in need thereof. By way of non-limiting example, the compositions of the invention are useful for manufacture of a medicament for treatment of patients suffering from metabolic disorders or disorders associated with changes in adipose tissue physiological function or mass.

*CGI-69* nucleic acids, or fragments thereof, may also be useful in diagnostic applications, wherein the presence or amount of the nucleic acid or the protein is to be assessed. A further use could be as an anti-bacterial molecule (*i.e.*, some peptides have been found to possess anti-bacterial properties).

**EXAMPLES**

The following examples are included to demonstrate preferred embodiments of the present invention. It should be appreciated by those of skill in the art that the techniques disclosed in the examples that follow represent techniques discovered by the inventors to function well in the practice of the invention, and thus can be considered to constitute preferred modes for its practice. However, those of skill in the art should, in light of the present disclosure, appreciate that many changes can be made in the specific embodiments that are disclosed and still obtain a like or similar result without departing from the scope of the invention.

**Example 1 Characterization of *CGI-69***

In this example, *CGI-69* is characterized as a gene induced by cold in brown adipose tissue (BAT) of mice by using differential mRNA analysis. Through this result, along with the analysis of the human CGI-69 protein, it was determined that CGI-69 is a mitochondrial carrier protein (MCP).

All studies were done in accordance with guidelines set forth by the Institutional Animal Care and Use Committee at Genentech. Male FVB-N/J mice (Jackson Labs, Bar Harbor, ME, USA) were received at 3 wk of age and housed at 2 mice/cage until tissue harvest at 6 wk of age. All mice were fed rodent chow *ad libitum* (Chow 5010, Ralston Purina, St. Louis, MO, USA) and housed on a 12:12 light/dark cycle (lights on 06:00). Control and Cold-Challenged mice were housed at 22°C during this 3 wk period, whereas Warm-Acclimated mice were housed at 33°C, within their thermoneutral zone (TNZ). For Cold-Challenged mice, cages were transferred to a 4°C room for 48 hr prior to tissue harvest. Following $CO_2$-induced euthanasia in the afternoon, interscapular BAT was excised, carefully cleared of visible white adipose tissue (WAT), connective tissue, and blood vessels, and snap-frozen in liquid nitrogen for subsequent RNA preparation. For all treatment groups (Control, Cold-Challenged, and Warm-Acclimated), 3 independent BAT samples were generated for analysis; each sample was composed of BAT pooled from 10 mice.

Samples from each treatment group were transferred to CuraGen Corp. (New Haven, CT, USA), RNA prepared and reverse-transcribed, and subjected to Quantitative Expression Analysis (QEA), the details of which are presented elsewhere (Shimkets, et al., 1999). Analyses focused on identification of genes regulated at least 2-fold by changes in $T_a$.

Full-length cDNAs of human CGI-69 were generated by PCR, using primers [forward 5'CTGAAGCTTCAAGATGGCTGACCAG3' (SEQ ID NO:5) and reverse 5'GTCCTTGCCTCCTTGCCCCTTTCAG3' (SEQ ID NO:6)] based on the sequence deposited in the public database (GenBank accession AF151827) and using human liver cDNA as a template (Clontech, Palo Alto, CA, USA). For subcellular localization studies, a carboxy-terminus FLAG-tagged version (FLAG-huCGI-69) was generated by PCR using the forward primer and a FLAG-reverse primer [5'CTTGTCATCGTCGTCCTTGTAGTCGCCGCCCAGAAGCCGGTC 3' (SEQ ID NO:7)]. CGI-69 PCR products were subcloned from pCR2.1 (Invitrogen, Carlsbad, CA, USA) into pRK7 (Genentech, Inc.) for expression analyses. Also for subcellular localization, MCF7 cells were transfected with pcDNA3-Flag-UCP3 or pRK7-FLAG-huCGI-69, and fixed in 3% formaldehyde as previously-described (Yu, et al., in press). Incubations with anti-FLAG and anti-cytochrome c oxidase antibodies, Cy3 and FITC-conjugated secondary antibodies, and visualization via confocal microscopy were performed as detailed elsewhere (Yu, et al., in press).

Analysis of BAT genes upregulated by cold identified a 348 bp gene fragment whose QEA profile indicated significant induction in Cold-Challenged mice. Initial identification of this DNA as corresponding to murine EST AA985996 was confirmed by sequencing. Real-time RT-PCR using primers/probes specific to this sequence validated the marked 2-fold cold-induction of the gene in the BAT of Cold-Challenged mice. Using murine EST AA985996 as the template for a contig analysis, and using mouse ESTs from the public database (SeqExtend Program, Genentech, Inc.), a putative murine full-length gene encoding a protein with high homology to the putative human protein CGI-69 (86% identical/98% similar) was discovered, thus confirming its identity as the mouse ortholog. Analysis of the CGI-69 protein structure indicated the presence of 4 mitochondrial carrier domains, 6 potential transmembrane spanning regions, a likely mitochondrial localization (NNPSL algorithm, The Sanger Centre, Hinxton, UK), and 3 regions with reasonable homologies to putative mitochondrial energy transfer signature motifs present in known UCP homologs. The mitochondrial localization of *carboxy*- and *amino*-FLAG-tagged CGI-69 indicates that native CGI-69 is targeted to this organelle.

### Example 2 Human *CGI-69* variants

In this example, a novel splice variant of human CGI-69 was discovered (SEQ ID NO:3).

mRNA abundance was analyzed in total RNA samples treated with DNAse per manufacturer's instructions (GIBCO BRL, Grand Island, NY, USA). Real-time quantitative RT-PCR was employed as described previously (Yu, et al., 2000a; Yu, et al., 2000b), using species- and isoform-specific primers and probes recognizing CGI-69. The isoform specificity of the human primer/probe sets were tested against authentic plasmids containing said isoforms. The sequences of primers and probes (5'→3') are as follows:

**Human CGI-69$_L$ : fwdAGCGAGCTGATGCCTTCCT (SEQ ID NO:11);**
**probeCAGACTGTGGAGCTTCTCCTATACCAAATTGCC (SEQ ID NO:12);**
**revCCCTGTGGATTGGAGAGAGG (SEQ ID NO:13)**

All data were normalized using 18S mRNA abundance to account for loading differences, using commercially-available 18S primer/probe sets (PE Applied Biosystems, Foster City, CA, USA).

A variety of CGI-69 clones were isolated from human liver upon PCR amplification and cloning, one of which corresponded to the original AF151827 sequence in GenBank ("CGI-69"). Numerous clones derived from separate, independent PCR cloning efforts diverged from the GenBank sequence in that they encoded an 8 amino acid insert preceded by a W64L change: this "long version" isoform was termed "CGI-69$_L$." In addition, various additional clones encoded proteins with an additional change (F239L in CGI-69; F247L in CGI-69$_L$). CGI-69 transcript was detected in numerous tissues, with particularly strong abundance in testis and BAT of mice, and testis and kidney of humans. In humans, both the short form(s) and long form(s) of the gene were expressed at various ratios. Transcripts for CGI-69 were widely-detected in human tissues, with particularly high expression in testis and kidney. All values are expressed relative to liver CGI-69 mRNA abundance, and represent abundance of *total* CGI-69. The relative contribution of CGI-69$_L$ (% of *total* CGI-69 transcript) in humans was: 23% (skeletal muscle, SKM), ~40-45% (heart, stomach, lung, uterus), 59% (brain), ~72% (liver, spleen), 80% (kidney), and 91% (testis).

### Example 3 Alteration of $\Delta\psi_m$

This example demonstrates that overexpression of a CGI-69 fusion protein having a *carboxy* FLAG-tagged CGI-69 diminished $\Delta\psi_m$. The FLAG tag contains the negatively charged amino acid sequence DYKDDDDK (SEQ ID NO:17).

Transfections and measurements of $\Delta\psi_m$ were carried out using protocols described previously (Yu, et al., 2000b). 293 cells were co-transfected with pGreen Lantern-1 (green fluorescent protein, GFP; GIBCO BRL) along with pRK7 vector alone (control) or expression vectors containing human CGI-69, OGC, or UCP3. Approximately 24 hr later, treatment-related differences in $\Delta\psi_m$ were determined in green-fluorescent protein (GFP) positive cells by monitoring changes in the fluorescence intensity of the $\Delta\psi_m$-sensitive dye TMRE (tetramethylrhodamine ethyl ester; Molecular Probes, Eugene, OR, USA). The degree of diminution of the $\Delta\psi_m$ was assessed by the shift in the relative number of cells displaying lowered $\Delta\psi_m$. The transfection protocols employed herein resulted in at least a 30-fold overexpression of each gene as judged by real-time RT-PCR analysis of mRNA abundance.

Incubations with anti-FLAG and anti-cytochrome c oxidase antibodies, Cy3 and FITC-conjugated secondary antibodies, and visualization via confocal microscopy were performed as detailed elsewhere (Mao, et al., 1999).

Overexpression of *carboxy*-FLAG-tagged CGI-69 in 293 cells diminished $\Delta\psi_m$ to a similar magnitude as did human UCP3. Similar to untagged CGI-69, *amino*-FLAG-tagged CGI-69 had no effect on $\Delta\psi_m$, despite mitochondrial localization in MCF7 cells. Overexpression of human CGI-69 in 293 cells also did not influence $\Delta\psi_m$.

## REFERENCES

U.S. Patent No. 4166452. Apparatus for testing human responses to stimuli. 1979.
U.S. Patent No. 4485045. Synthetic phosphatidyl cholines useful in forming liposomes. 1984.
U.S. Patent No. 4544545. Liposomes containing modified cholesterol for organ targeting. 1985.
U.S. Patent No. 4,676,980. Target specific cross-linked heteroantibodies. 1987.
U.S. Patent No. 4816567. Recombinant immunoglobin preparations. 1989.
U.S. Patent No. 5013556. Liposomes with enhanced circulation time. 1991.
U.S. Patent No. 5545807. Production of antibodies from transgenic animals. 1996.
U.S. Patent No. 5545806. Ransgenic <sic> non-human animals for producing heterologous antibodies. 1996.
U.S. Patent No. 5569825. Transgenic non-human animals capable of producing heterologous antibodies of various isotypes. 1996.
U.S. Patent No. 5633425. Transgenic non-human animals capable of producing heterologous antibodies. 1997.
U.S. Patent No. 5661016. Transgenic non-human animals capable of producing heterologous antibodies of various isotypes. 1997.
U.S. Patent No. 5625126. Transgenic non-human animals for producing heterologous antibodies. 1997.
U.S. Patent No. 3,773,919. Polylactide-drug mixtures. 1973.
U.S. Patent No. 5,116,742. RNA ribozyme restriction endoribonucleases and methods. 1992.
U.S. Patent No. 4,987,071. RNA ribozyme polymerases, dephosphorylases, restriction endoribonucleases and methods. 1991.
US Patent No. 4,522,811. Serial injection of muramyldipeptides and liposomes enhances the anti-infective activity of muramyldipeptides Serial injection of muramyldipeptides and liposomes enhances the anti-infective activity of muramyldipeptides. 1985.
U.S. Patent No. 4,870,009. Method of obtaining gene product through the generation of transgenic animals. 1989.
US Patent No. 5;804,604. Tat-derived transport polypeptides and fusion proteins. 1998.
US Patent No. 5;223,409. Directed evolution of novel binding proteins. 1993.
US Patent No. 5;459,039. Methods for mapping genetic mutations. 1995.
US Patent No. 4;683,202. Process for amplifying nucleic acid sequences. 1987.
US Patent No. 4,683,195. Process for amplifying, detecting, and/or cloning nucleic acid sequences. 1987.
US Patent No. 5,328,470. Treatment of diseases by site-specific instillation of cells or site-specific transformation of cells and kits therefor. 1994.
US Patent No. 5,283,317. Intermediates for conjugation of polypeptides with high molecular weight polyalkylene glycols. 1994.
US Patent No. 5,272,057. Method of detecting a predisposition to cancer by the use of restriction fragment length polymorphism of the gene for human poly (ADP-ribose) polymerase. 1993.
US Patent No. 4904582. Novel amphiphilic nucleic acid conjugates. 1988.
US Patent No. 4,873,191. Genetic transformation of zygotes. 1989.
U.S. Patent No. 4,736,866. Transgenic non-human animals. 1988.
WO 90/10448. Covalent conjugates of lipid and oligonucleotide. 1990.
WO 90/13641. Stably transformed eucaryotic cells comprisng a foreign transcribable DNA under the control of a pol III promoter. 1990.
WO 91/00360. Bispecific reagents for AIDS therapy. 1991.
WO 91/04753. Conjugates of antisense oligonucleotides and therapeutic uses thereof. 1991.
WO 91/00357. New strain with filamentous fungi mutants, process for the production of recombinant proteins using said strain, and strains and proteins. 1991.
WO 91/06629. Oligonucleotide analogs with novel linkages. 1991.
WO 92/20373. Heteroconjugate antibodies for treatment of HIV infection. 1992.
WO 93/08829. Compositions that mediate killing of HIV-infected cells. 1993.
WO 94/11026. Therapeutic application of chimeric and radiolabeled antibodies to human B lymphocyte restricted differentiation antigen for treatment of B cells. 1994.
WO 96/27011. A method for making heteromultimeric polypeptides. 1996.
WO 97/33551. Compositions and methods for the diagnosis, prevention, and treatment of neoplastic cell growth and proliferation. 1997.
WO89/10134. Chimeric peptides for neuropeptide delivery through the blood-brain barrier. 1989.
WO 91/01140. HOMOLOGOUS RECOMBINATION FOR UNIVERSAL DONOR CELLS AND CHIMERIC MAMMALIAN HOSTS. 1991.
WO 90/11354. Process for the specific replacement of a copy of a gene present in the receiver genome via the integration of a gene. 1990.
WO94/16101. DNA SEQUENCING BY MASS SPECTROMETRY. 1994.
WO94/10300. INTERACTION TRAP SYSTEM FOR ISOLATING NOVEL PROTEINS. 1994.
WO 93/04169. GENE TARGETING IN ANIMAL CELLS USING ISOGENIC DNA CONSTRUCTS. 1993.
EPO 402226. Transformation vectors for yeast *Yarrowia*. 1990.
Abravaya, K., J.J. Carrino, S. Muldoon, and H.H. Lee. 1995. Detection of point mutations with a modified ligase chain reaction (Gap- LCR). *Nucleic Acids Res.* 23:675-82.
Adams, S.H. 2000. *J. Nutr.* 130: 711-714.
Alam, J., and J.L. Cook. 1990. Reporter genes: Application to the study of mammalian gene transcription. Anal. *Biochem*. 188:245-254.
Austin, C.P., and C.L. Cepko. 1990. Cellular migration patterns in the developing mouse cerebral cortex. *Development*. 110:713-732.
Ausubel, F.M., R. Brent, R.E. Kingston, D.D. Moore, *et al*. 1987. Current protocols in molecular biology. John Wiley & Sons, New York.
Barany, F. 1991. Genetic disease detection and DNA amplification using cloned thermostable ligase. *Proc Natl Acad Sci U S A*. 88:189-93.
Bartel, D.P., and J.W. Szostak. 1993. Isolation of new ribozymes from a large pool of random sequences [see comment]. *Science.* 261:1411-8.
Bartel, P., C.T. Chien, R. Sternglanz, and S. Fields. 1993. Elimination of false positives that arise in using the two-hybrid system. *Biotechniques.* 14:920-4.
Beal, P.A., and P.B. Dervan. 1991. Second structural motif for recognition of DNA by oligonucleotide- directed triple-helix formation. *Science*. 251:1360-3.
Bechtold, N., and G. Pelletier. 1998. In planta Agrobacterium-mediated transformation of adult Arabidopsis thaliana plants by vacuum infiltration. *Methods Mol Biol.* 82:259-66.
Becker, D.M., and L. Guarente. 1991. High-efficiency transformation of yeast by electroporation. *Methods Enzymol*. 194:182-187.
Beggs, J.D. 1978. Transformation of yeast by a replicating hybrid plasmid. *Nature.* 275:104-109.
Bently, D.R., and I. Dunham. 1995. Mapping human chromosomes. *Curr Opin Genet Dev.* 5:328-34.
Berger, J., J. Hauber, R. Hauber, R. Geiger, *et al*. 1988. Secreted placental alkaline phosphatase: A powerful new qunatitative indicator of gene expression in eukaryotic cells. *Gene.* 66:1-10.
Bodine, D.M., K.T. McDonagh, N.E. Seidel, and A.W. Nienhuis. 1991. Survival and retrovirus infection of murine hematopoietic stem cells in vitro: effects of 5-FU and method of infection. *Exp. Hematol*. 19:206-212.
Boerner, P., R. Lafond, W.Z. Lu, P. Brams, *et al*. 1991. Production of antigen-specific human monoclonal antibodies from in vitro-primed human splenocytes. *J Immunol*. 147:86-95.
Bradley. 1987. Teratocarcinomas and Embryonic Stem Cells: A Practical Approach. Oxford University Press, Inc., Oxford. 268 pp.
Bradley, A. 1991. Modifying the mammalian genome by gene targeting. *Curr Opin Biotechnol*. 2:823-9.
Brand, M.D., L.F. Chien, E.K. Ainscow, D.F.S. Rolfe, and R.K. Porter. 1994. The causes and functions of mitochondrial proton leak. *Biochim*. *Biophys. Acta* 1187: 132-139.
Bray, G.A. 1997. Progress in understanding the genetics of obesity. *J Nutr*. 127:940S-942S.
Brennan, M., P.F. Davison, and H. Paulus. 1985. Preparation of bispecific antibodies by chemical recombination of monoclonal immunoglobulin G1 fragments. *Science.* 229:81-3.
Capecchi, M.R. 1980. High efficiency transformation by direct microinjection of DNA into cultured mammalian cells. *Cell.* 22:479.
Capecchi, M.R. 1989. Altering the genome by homologous recombination. *Science.* 244:1288-92.
Carell, T., E.A. Wintner, and J. Rebek Jr. 1994a. A novel procedure for the synthesis of libraries containing small organic molecules. *Angewandte Chemie International Edition.* 33:2059-2061.
Carell, T., E.A. Wintner, and J. Rebek Jr. 1994b. A solution phase screening procedure for the isolation of active compounds from a molecular library. *Angewandte Chemie International Edition.* 33:2061-2064.
Caron, P.C., W. Laird, M.S. Co, N.M. Avdalovic, *et al*. 1992. Engineered humanized dimeric forms of IgG are more effective antibodies. *J Exp Med*. 176:1191-5.
Carter, P. 1986. Site-directed mutagenesis. *Biochem J.* 237:1-7.
Case, M.E., M. Schweizer, S.R. Kushner, and N.H. Giles. 1979. Efficient transformation of Neurospora crassa by utilizing hybrid plasmid DNA. *Proc Natl Acad Sci U S A*. 76:5259-63.
Cepko, C.L., B.E. Roberts, and R.E. Mulligan. 1984. Construction and applications of a highly transmissible murine retrovirus shuttle vector. *Cell*. 37:1053-1062.
Chalfie, M., Y. tu, G. Euskirchen, W.W. Ward, *et al*. 1994. Green fluorescent protein as a marker for gene expression. *Science.* 263:802-805.
Chaney, W.G., D.R. Howard, J.W. Pollard, S. Sallustio, *et al*. 1986. High-frequency transfection of CHO cells using Polybrene. *Somatic Cell Mol. Genet.* 12:237.
Chen, C., and H. Okayama. 1988. Calcium phosphate-mediated gene transfer: A highly efficient system for stably transforming cells with plasmid DNA. *BioTechniques.* 6:632-638.
Chen, S.H., H.D. Shine, J.C. Goodman, R.G. Grossman, *et al*. 1994. Gene therapy for brain tumors: regression of experimental gliomas by adenovirus-mediated gene transfer in vivo. *Proc Natl Acad Sci U S A*. 91:3054-7.
Cho, C.Y., E.J. Moran, S.R. Cherry, J.C. Stephans, *et al*. 1993. An unnatural biopolymer. *Science.* 261:1303-5.
Cohen, A.S., D.L. Smisek, and B.H. Wang. 1996. Emerging technologies for sequencing antisense oligonucleotides: capillary electrophoresis and mass spectrometry. *Adv Chromatogr*. 36:127-62.
Cohen, J.S. 1989. Oligodeoxynucleotides: Antisense inhibitors of gene expression. CRC Press, Boca Raton, FL. 255 pp.
Cohen, S.M.N., A.C.Y. Chang, and L Hsu. 1972. Nonchromosomal antibiotic resistance in bacteria: Genetic transformation of *Escherichia coli* by R-factor DNA. *Proc. Natl. Acad. Sci. USA.* 69:2110.
Cooney, M., G. Czernuszewicz, E.H. Postel, S.J. Flint, *et al*. 1988. Site-specific oligonucleotide binding represses transcription of the human c-myc gene in vitro. *Science.* 241:456-9.
Cotton, R.G. 1993. Current methods of mutation detection. *Mutat Res.* 285:125-44.
Cronin, M.T., R.V. Fucini, S.M. Kim, R.S. Masino, *et al*. 1996. Cystic fibrosis mutation detection by hybridization to light-generated DNA probe arrays. *Hum Mutat*. 7:244-55.
Cull, M.G., J.F. Miller, and P.J. Schatz. 1992. Screening for receptor ligands using large libraries of peptides linked to the C terminus of the lac repressor. *Proc Natl Acad Sci U S A.* 89:1865-9.
Cwirla, S.E., E.A. Peters, R.W. Barrett, and W.J. Dower. 1990. Peptides on phage: a vast library of peptides for identifying ligands. *Proc Natl Acad Sci U S A*. 87:6378-82.
de Boer, A.G. 1994. Drug absorption enhancement: Concepts, possibilities, limitations and trends. Harwood Academic Publishers, Langhorne, PA.
de Louvencourt, L., H. Fukuhara, H. Heslot, and M. Wesolowski. 1983. Transformation of Kluyveromyces lactis by killer plasmid DNA. *J Bacteriol*. 154:737-42.
de Wet, J.R., K.V. Wood, M. DeLuca, D.R. Helinski, *et al*. 1987. Sturcture and expression in mammalian cells. *Mol. Cell Biol*. 7:725-737.
Demerec, M., E.A. Adelberg, AJ. Clark, and P.E. Hartman. 1966. A proposal for a uniform nomenclature in bacterial genetics. *Genetics*. 54:61-76.
Devlin, J.J., L.C. Panganiban, and P.E. Devlin. 1990. Random peptide libraries: a source of specific protein binding molecules. *Science.* 249:404-6.
DeWitt, S.H., J.S. Kiely, CJ. Stankovic, M.C. Schroeder, *et al*. 1993. "Diversomers": an approach to nonpeptide, nonoligomeric chemical diversity. *Proc Natl Acad Sci U S A.* 90:6909-13.
Eichelbaum, M., and B. Evert. 1996. Influence of pharmacogenetics on drug disposition and response. *Clin Exp Pharmacol Physiol.* 23:983-5.
Ellington, A.D., and J.W. Szostak. 1990. In vitro selection of RNA molecules that bind specific ligands. *Nature.* 346:818-22.
Elroy-Stein, O., and B. Moss. 1990. Cytoplasmic expression system based on constitutive synthesis of bacteriophage T7 RNA polymerase in mammalian cells. *Proc. Natl. Acad. Sci. USA.* 87:6743-6747.
Eppstein, D.A., Y.V. Marsh, M. van der Pas, P.L. Felgner, *et al*. 1985. Biological activity of liposome-encapsulated murine interferon gamma is mediated by a cell membrane receptor. *Proc Natl Acad Sci U S A*. 82:3688-92.
Escudero, J., and B. Hohn. 1997. Transfer and integration of T-DNA without cell injury in the host plant. *Plant Cell.* 9:2135-2142.
Fekete, D.M., and C.L. Cepko. 1993. Retroviral infection coupled with tissue transplantation limits gene transfer in the chick embryo. *Proc. Natl. Acad. Sci. USA*. 90:2350-2354.
Felgner, P.L., T.R. Gadek, M. Holm, R. Roman, *et al.* 1987. Lipofectin: A highly efficient, lipid-mediated DNA/ transfection procedure. *Proc. Natl. Acad Sci. USA.* 84:7413-7417.
Felici, F., L. Castagnoli, A. Musacchio, R. Jappelli, *et al*. 1991. Selection of antibody ligands from a large library of oligopeptides expressed on a multivalent exposition vector. *J Mol Biol*. 222:301-10.

Fieck, A., D.L. Wyborski, and J.M. Short. 1992. Modifications of the E.coli Lac repressor for expression in eukaryotic cells: effects of nuclear signal sequences on protein activity and nuclear accumulation. *Nucleic Acids Res.* 20: 1785-91.
Finer, J.J., K.R. Finer, and T. Ponappa. 1999. Particle bombardment-mediated transformation. *Current Topics in microbiology and immunology. 240:59-80.*
Finn, P.J., N.J. Cribson, R. Fallon, A. Hamilton, *et al*. 1996. Synthesis and properties of DNA-PNA chimeric oligomers. *Nucleic Acids Res.* 24:3357-63.
Fishwild, D.M., S.L. O'Donnell, T. Bengoechea, D.V. Hudson, *et al*. 1996. High-avidity human IgG kappa monoclonal antibodies from a novel strain of minilocus transgenic mice [see comments]. *Nat Biotechnol.* 14:845-51.
Fleer, R., P. Yeh, N. Amellal, L Maury, *et al*. 1991. Stable multicopy vectors for high-level secretion of recombinant human serum albumin by Kluyveromyces yeasts. *Biotechnology (N Y).* 9:968-75.
Fodor, S.P., R.P. Rava, X.C. Huang, A.C. Pease, *et al*. 1993. Multiplexed biochemical assays with biological chips. *Nature.* 364:555-6.
Fromm, M., L.P. Taylor, and V. Walbot. 1985. Expression of genes transferred into monocot and dicot plant cells by electroporation. *Proc. Natl. Acad Sci. USA.* 82:5824-5828.
Fujita, T., H. Shubiya, T. Ohashi, K. Yamanishi, *et al*. 1986. Regulation of human interleukin-2 gene: Functional DNA sequences in the 5' flanking region for the gene expression in activated T lymphocytes. *Cell*. 46:401-407.
Gabizon, A., R. Shiota, and D. Papahadjopoulos. 1989. Pharmacokinetics and tissue distribution of doxorubicin encapsulated in stable liposomes with long circulation times. *J Natl Cancer Inst.* 81:1484-8.
Gallagher, S.R. 1992. GUS protocols: Using the GUS gene as a reporter of gene expression. Academic Press, San Diego, CA.
Gallop, M.A., R.W. Barrett, W.J. Dower, S.P. Fodor, *et al*. 1994. Applications of combinatorial technologies to drug discovery. 1. Background and peptide combinatorial libraries. *J Med Chem.* 37:1233-51.
Gasparini, P., A. Bonizzato, M. Dognini, and P.F. Pignatti. 1992. Restriction site generating-polymerase chain reaction (RG-PCR) for the probeless detection of hidden genetic variation: application to the study of some common cystic fibrosis mutations. *Mol Cell Probes.* 6:1-7.
Gautier, C., F. Morvan, B. Rayner, T. Huynh-Dinh, *et al*. 1987. Alpha-DNA. IV: Alpha-anomeric and beta-anomeric tetrathymidylates covalently linked to intercalating oxazolopyridocarbazole. Synthesis, physicochemical properties and poly (rA) binding. *Nucleic Acids Res.* 15:6625-41.
Gennaro, A.R. 2000. Remington: The science and practice of pharmacy. Lippincott, Williams & Wilkins, Philadelphia, PA.
Gibbs, R.A., P.N. Nguyen, and C.T. Caskey. 1989. Detection of single DNA base differences by competitive oligonucleotide priming. *Nucleic Acids Res.* 17:2437-48.
Gietz, R.D., R.A. Woods, P. Manivasakam, and R.H. Schiestl. 1998. Growth and transformation of *Saccharomyces cerevisiae. In* Cells: A laboratory manual. Vol. L D. Spector, R. Goldman, and L. Leinwand, editors. Cold Spring Harbor Press, Cold Spring Harbor, NY.
Goding, J.W. 1996. Monoclonal antibodies: Principles and Practice. Academic Press, San Diego. 492 pp.
Goldstein, S.A., and D.H. Elwyn. 1989. The effects of injury and sepsis on fuel utilization. *Annu Rev Nutr.* 9:445-73.
Gong, D.W., S. Monemdjou, O. Gavrilova, L.R. Leon, B. Marcus-Samuels, C.J. Chou, C. Everett, L.P. Kozak, C. Li, C. Deng, M.E. Harper, and M.L. Reitman. 2000. Lack of obesity and normal response to fasting and thyroid hormone in mice lacking uncoupling protein-3. *J. Biol. Chem*.275(21)16251-7.
Gorman, C.M., L.F. Moffat, and B.H. Howard. 1982. Recombinant genomes which express chloramphenicol acetyltransferase in mammalian cells. *Mol. Cell. Biol*. 2:1044-1051.
Graham, F.L., and A.J. van der Eb. 1973. A new technique for the assay of infectivity of human adenovirus 5 DNA. *Virology.* 52:456-.
Green, H., and M. Meuth. 1974. An established pre-adipose cell line and its differentiation in culture. *Cell.* 3:127-33.
Griffin, H.G., and A.M. Griffin. 1993. DNA sequencing. Recent innovations and future trends. *Appl Biochem Biotechnol.* 38:147-59.
Grompe, M., D.M. Muzny, and C.T. Caskey. 1989. Scanning detection of mutations in human ornithine transcarbamoylase by chemical mismatch cleavage. *Proc Natl Acad Sci U S A*. 86:5888-92.
Gruber, M., B.A. Schodin, E.R. Wilson, and D.M. Kranz. 1994. Efficient tumor cell lysis mediated by a bispecific single chain antibody expressed in Escherichia coli. *J Immunol*. 152:5368-74.
Guatelli, J.C., K.M. Whitfield, D.Y. Kwoh, KJ. Barringer, *et al*. 1990. Isothermal, in vitro amplification of nucleic acids by a multienzyme reaction modeled after retroviral replication. *Proc Natl Acad Sci U S A*. 87:1874-8.
Hanahan, D. 1983. Studies on transformation of *Escherichia coli* with plasmids. *J. Mol. Biol*. 166:557-580.
Hansen, G., and M.-D. Chilton. 1999. Lessons in gene transfer to plants by a gifted microbe. *Curr. Top. MicrobioL Immunol*. 240:21-57.
Hansen, G., and M.S. Wright. 1999. Recent advances in the transformation of plants. *Trends Plant Sci.* 4:226-231.

Harlow, E., and D. Lane. 1988. Antibodies: A laboratory manual. Cold Spring Harbor Laboratory Press, Cold Spring Harbor. 726 pp.

Harlow, E., and D. Lane. 1999. Using antibodies: A laboratory manual. Cold Spring Harbor Laboratory PRess, Cold Spring Harbor, New York.

Haseloff, J., and W.L. Gerlach. 1988. Simple RNA enzymes with new and highly specific endoribonuclease activities. *Nature.* 334:585-91.

Hayashi, K. 1992. PCR-SSCP: A method for detection of mutations. *Genetic and Analytical Techniques Applications*. 9:73-79.

Helene, C. 1991. The anti-gene strategy: control of gene expression by triplex-forming- oligonucleotides. *Anticancer Drug Des.* 6:569-84.

Helene, C., N.T. Thuong, and A. Harel-Bellan. 1992. Control of gene expression by triple helix-forming oligonucleotides. The antigene strategy. *Ann N Y Acad Sci.* 660:27-36.

Hill, J.O., and J.C. Peters. 1998. Environmental contributions to the obesity epidemic. *Science.* 280:1371-4.

Hinnen, A., J.B. Hicks, and G.R. Fink. 1978. Transformation of yeast. *Proc. Natl. Acad Sci. USA.* 75:1929-1933.

Hoffman, F. 1996. Laser microbeams for the manipulation of plant cells and subcellular structures. *Plant Sci.* 113: 1-11.

Hogan, B., Beddington, R., Costantini, F., Lacy, E. 1994. Manipulating the Mouse Embryo: A Laboratory Manual. Cold Spring Harbor Laboratory Press. 500 pp.

Holliger, P., T. Prospero, and G. Winter. 1993. "Diabodies": small bivalent and bispecific antibody fragments. *Proc Natl Acad Sci U S A.* 90:6444-8.

Hoogenboom, H.R., A.D. Griffiths, K.S. Johnson, DJ. Chiswell, *et al.* 1991. Multi-subunit proteins on the surface of filamentous phage: methodologies for displaying antibody (Fab) heavy and light chains. *Nucleic Acids Res.* 19: 4133-7.

Houghten, R.A., J.R. Appel, S.E. Blondelle, J.H. Cuervo, *et al.* 1992. The use of synthetic peptide combinatorial libraries for the identification of bioactive peptides. *Biotechniques.* 13:412-21.

Hsu, I.C., Q. Yang, M.W. Kahng, and J.F. Xu. 1994. Detection of DNA point mutations with DNA mismatch repair enzymes. *Carcinogenesis.* 15:1657-62.

Hudson, TJ., L.D. Stein, S.S. Gerety, J. Ma, *et al.* 1995. An STS-based map of the human genome. *Science.* 270: 1945-54.

Hwang, K.J., K.F. Luk, and P.L. Beaumier. 1980. Hepatic uptake and degradation of unilamellar sphingomyelin/ cholesterol liposomes: a kinetic study. *Proc Natl Acad Sci U S A.* 77:4030-4.

Hyrup, B., and P.E. Nielsen. 1996. Peptide nucleic acids (PNA): synthesis, properties and potential applications. *Bioorg Med Chem.* 4:5-23.

Inoue, H., Y. Hayase, A. Imura, S. Iwai, *et al.* 1987a. Synthesis and hybridization studies on two complementary nona(2'-O- methyl)ribonucleotides. *Nucleic Acids Res.* 15:6131-48.

Inoue, H., Y. Hayase, S. Iwai, and E. Ohtsuka. 1987b. Sequence-dependent hydrolysis of RNA using modified oligonucleotide splints and RNase H. *FEBS Lett.* 215:327-30.

Ishiura, M., S. Hirose, T. Uchida, Y. Hamada, *et al.* 1982. Phage particle-mediated gene transfer to cultured mammalian cells. *Molecular and Cellular Biology.* 2:607-616.

Ito, H., Y. Fukuda, K. Murata, and A. Kimura. 1983. Transformation of intact yeast cells treated with alkali cations. *J. Bacteriol.* 153:163-168.

Iwabuchi, K., B. Li, P. Bartel, and S. Fields. 1993. Use of the two-hybrid system to identify the domain of p53 involved in oligomerization. *Oncogene.* 8:1693-6.

Jayasena, S.D. 1999. Aptamers: an emerging class of molecules that rival antibodies in diagnostics. *Clin Chem.* 45:1628-50.

Jones, P.T., P.H. Dear, J. Foote, M.S. Neuberger, *et al.* 1986. Replacing the complementarity-determining regions in a human antibody with those from a mouse. *Nature.* 321:522-5.

Kaufman, RJ. 1990. Vectors used for expression in mammalian cells. *Methods Enzymol.* 185:487-511.

Kaufman, RJ., P. Murtha, D.E. Ingolia, C.-Y. Yeung, *et al.* 1986. Selection and amplification of heterologous genes encoding adenosine deaminase in mammalian cells. *Proc. Natl. Acad. Sci. USA.* 83:3136-3140.

Kawai, S., and M. Nishizawa. 1984. New procedure for DNA transfection with polycation and dimethyl sulfoxide. *Mol. Cell. Biol.* 4:1172.

Keen, J., D. Lester, C. Inglehearn, A. Curtis, *et al.* 1991. Rapid detection of single base mismatches as heteroduplexes on Hydrolink gels. *Trends Genet.* 7:5.

Kelly, J.M., and M.J. Hynes. 1985. Transformation of Aspergillus niger by the amdS gene of Aspergillus nidulans. *Embo J.* 4:475-9.

Kinney, J., J. Duke, Jr., C. Long, and R. Gump. 1970. Tissue fuel and weight loss after injury. *J. Clin. Path.* 23 (suppl. 4):65-72.

Kissebah, A.H., G.E. Sonnenberg, J. Myklebust, M. Goldstein, *et al*. 2000. Quantitative trait loci on chromosomes 3 and 17 influence phenotypes of the metabolic syndrome. *Proc Natl Acad Sci U S A*. 97:14478-83.

Kostelny, S.A., M.S. Cole, and J.Y. Tso. 1992. Formation of a bispecific antibody by the use of leucine zippers. *J Immunol*. 148:1547-53.

Kozal, M.J., N. Shah, N. Shen, R. Yang, *et al.* 1996. Extensive polymorphisms observed in HIV-1 clade B protease gene using high-density oligonucleotide arrays. *Nat Med.* 2:753-9.

Kozbor, D., P. Tripputi, J.C. Roder, and C.M. Croce. 1984. A human hybrid myeloma for production of human monoclonal antibodies. *J Immunol*. 133:3001-5.

Kriegler, M. 1990. Gene transfer and expression: A laboratory manual. Stockton Press, New York. 242 pp.

Kwoh, D.Y., G.R. Davis, K.M. Whitfield, H.L. Chappelle, *et al*. 1989. Transcription-based amplification system and detection of amplified human immunodeficiency virus type 1 with a bead-based sandwich hybridization format. *Proc Natl Acad Sci U S A*. 86:1173-7.

Lakso, M., B. Sauer, B. Mosinger, E.J. Lee, *et al*. 1992. Targeted oncogene activation by site-specific recombination in transgenic mice. *Proc Natl Acad Sci U S A*. 89:6232-6.

Lam, K.S. 1997. Application of combinatorial library methods in cancer research and drug discovery. *Anticancer Drug Design*. 12:145-167.

Lam, K.S., S.E. Salmon, E.M. Hersh, VJ. Hruby, *et al*. 1991. General method for rapid synthesis of multicomponent peptide mixtures. *Nature.* 354:82-84.

Landegren, U., R. Kaiser, J. Sanders, and L. Hood. 1988. A ligase-mediated gene detection technique. *Science.* 241:1077-80.

Leduc, N., and e. al. 1996. Isolated maize zygotes mimic *in vivo* embryogenic development and express microinjected genes when cultured *in vitro. Dev. Biol.* 10:190-203.

Lee, J.S., D.A. Johnson, and A.R. Morgan. 1979. Complexes formed by (pyrimidine)n . (purine)n DNAs on lowering the pH are three-stranded. *Nucleic Acids Res.* 6:3073-91.

Lee, V.H.L. 1990. Peptide and protein drug delivery. Marcel Dekker, New York, NY.

Lemaitre, M., B. Bayard, and B. Lebleu. 1987. Specific antiviral activity of a poly(L-lysine)-conjugated oligodeoxyribonucleotide sequence complementary to vesicular stomatitis virus N protein mRNA initiation site. *Proc Natl Acad Sci U S A*. 84:648-52.

Lemischka, I.R., D.H. Raulet, and R.C. Mulligan. 1986. Developmental potential and dynamic behavior of hematopoietic stem cells. *Cell*. 45:917-927.

Letsinger, R.L., G.R. Zhang, D.K. Sun, T. Ikeuchi, *et al*. 1989. Cholesteryl-conjugated oligonucleotides: synthesis, properties, and activity as inhibitors of replication of human immunodeficiency virus in cell culture. *Proc Natl Acad Sci U S A*. 86:6553-6.

Li, E., T.H. Bestor, and R. Jaenisch. 1992. Targeted mutation of the DNA methyltransferase gene results in embryonic lethality. *Cell.* 69:915-26.

Linder, M.W., R.A. Prough, and R. Valdes. 1997. Pharmacogenetics: a laboratory tool for optimizing therapeutic efficiency. *Clin Chem.* 43:254-66.

Littlefield, J.W. 1964. Selection of hybrids from matings of fibroblasts in vitro and their presumed recombinants. *Science.* 145:709-710.

Lizardi, P.M., C.E. Guerra, H. Lomeli, L Tussie-Luna, *et al.* 1988. Exponential amplification of recombinant-RNA hybridization probes. *Biotechnology*. 6:1197-1202.

Lonberg, N., and D. Huszar. 1995. Human antibodies from transgenic mice. *Int Rev Immunol.* 13:65-93.

Lonberg, N., L.D. Taylor, F.A. Harding, M. Trounstine, *et al*. 1994. Antigen-specific human antibodies from mice comprising four distinct genetic modifications [see comments]. *Nature.* 368:856-9.

Lopata, M.A., D.W. Cleveland, and B. Sollner-Webb. 1984. High-level expression of a chloramphenicol acetyltransferase gene by DEAEdextran-mediated DNA traansfection couled with a dimethylsulfoxide or glycerol shock treatment. *Nucleic Acids Research.* 12:5707.

Luckow, V.A. 1991. Cloning and expression of heterologous genes in insect cells with baculovirus vectors. *In* Recombinant DNA technology and applications. A. Prokop, R.K. Bajpai, and C. Ho, editors. McGraw-Hill, New York. 97-152.

Madura, K., RJ. Dohmen, and A. Varshavsky. 1993. N-recognin/Ubc2 interactions in the N-end rule pathway. *J Biol Chem.* 268:12046-54.

Maher, L.J. 1992. DNA triple-helix formation: an approach to artificial gene repressors? *Bioessays.* 14:807-15.

Mandel, M., and A. Higa. 1970. Calcium-dependent bacteriophage DNA infection. *J. Mol. biol.* 53:159-162.

Mao, W., X.X. Yu, A. Zhong, W. Li, J. Brush, S.W. Sherwood, S.H. Adams, and G. Pan. 1999. UCP4, a novel brain-specific mitochondrial protein that reduces membrane potential in mammalian cells. *FEBS Letters.* 443: 326-330.

Marasco, W.A., W.A. Haseltine, and S.Y. Chen. 1993. Design, intracellular. expression, and activity of a human anti-human immunodeficiency virus type 1 gp120 single-chain antibody. *Proc Natl Acad Sci U S A*. 90:7889-93.

Marks, J.D., A.D. Griffiths, M. Malmqvist, T.P. Clackson, *et al*. 1992. By-passing immunization: building high affinity human antibodies by chain shuffling. *Biotechnology (N Y).* 10:779-83.
Marks, J.D., H.R. Hoogenboom, T.P. Bonnert, J. *McCafferty, et al. 1991.* By-passing immunization. Human antibodies from V-gene libraries displayed on phage. *J Mol Biol.* 222:581-97.
Martin, F.J., and D. Papahadjopoulos. 1982. Irreversible coupling of immunoglobulin fragments to preformed vesicles. An improved method for liposome targeting. *J Biol Chem*. 257:286-8.
Maxam, A.M., and W. Gilbert. 1977. A new method for sequencing DNA. *Proc Natl Acad Sci U S A*. 74:560-4.
Miller, A.D., and C. Buttimore. 1986. Redesign of retrovirus packaging cell lines to avoid recombination leading to helper virus production. *Mol. Cell biol*. 6:2895-2902.
Miller, L.K. 1988. Baculoviruses as gene expression vectors. *Annu. Rev. Microbiol*. 42:177-199.
Milstein, C., and A.C. Cuello. 1983. Hybrid hybridomas and their use in immunohistochemistry. *Nature.* 305:537-40.
Mitchell, B.D., S.A. Cole, A.G. Comuzzie, L. Almasy, *et al*. 1999. A quantitative trait locus influencing BMI maps to the region of the beta-3 adrenergic receptor. *Diabetes*. 48:1863-7.
Morrison, S.L., L. Wims, S. Wallick, L. Tan, et *al*. 1987. Genetically engineered antibody molecules and their application. Ann *N Y Acad Sci*. 507:187-98.
Munson, P.J., and D. Rodbard. 1980. Ligand: a versatile computerized approach for characterization of ligand-binding systems. *Anal Biochem.* 107:220-39.
Must, A., J. Spadano, E.H. Coakley, A.E. Field, *et al*. 1999. The disease burden associated with overweight and obesity. *Jama*. 282:1523-9.
Myers, R.M., Z. Larin, and T. Maniatis. 1985. Detection of single base substitutions by ribonuclease cleavage at mismatches in RNA:DNA duplexes. *Science.* 230:1242-6.
Naeve, C.W., G.A. Buck, R.L. Niece, R.T. Pon, *et al*. 1995. Accuracy of automated DNA sequencing: a multi-laboratory comparison of sequencing results. *Biotechniques.* 19:448-53.
Nakazawa, H., D. English, P.L. Randell, K. Nakazawa, *et al*. 1994. UV and skin cancer: specific p53 gene mutation in normal skin as a biologically relevant exposure measurement. *Proc Natl Acad Sci U S A*. 91:360-4.
Neumann, E., M. Schaefer-Ridder, Y. Wang, and P.H. Hofschneider. 1982. Gene transfer into mouse lyoma cells by electroporation in high electric fields. *EMBO J.* 1:841-845.
Nicholls, D.G. and R.M. Locke. 1984. Thermogenic mechanisms in brown fat. *Physiol. Rev*. 64: 1-64.
O'Gorman, S., D.T. Fox, and G.M. Wahl. 1991. Recombinase-mediated gene activation and site-specific integration in mammalian cells. *Science.* 251:1351-5.
Okano, H., J. Aruga, T. Nakagawa, C. Shiota, *et al*. 1991. Myelin basic protein gene and the function of antisense RNA in its repression in myelin-deficient mutant mouse. *J Neurochem*. 56:560-7.
O'Reilly, D.R., L.K. Miller, and V.A. Luckow. 1992. Baculovirus expression vectors. W.H. Freeman and Company, New York.
Orita, M., H. Iwahana, H. Kanazawa, K. Hayashi, *et al*. 1989. Detection of polymorphisms of human DNA by gel electrophoresis as single-strand conformation polymorphisms. *Proc Natl Acad Sci U S A*. 86:2766-70.
Ou-Lee, T.M., R. Turgeon, and R. Wu. 1986. Uptake and expression of a foreign gene linked to either a plant virus or *Drosophila* promoter in protoplasts of rice, wheat and sorghum. *Proc. Natl. Acad. Sci. USA*. 83:6815-6819.
Palmer, TD., R.A. Hock, W.R.A. osborne, and A.D. Miller. 1987. Efficient retrovirus-mediated transfer and expression of a human adenosine deaminase gene in diploid skin fibroblasts from an adenosie-deficient human. *Proc. Natl. Acad. Sci. USA*. 84:1055-1059.
Pear, W., G. Nolan, M. Scott, and D. Baltimore. 1993. Production of high-titer helper-free retroviruses by transient transfection. *Proc. Natl. Acad Sci. USA*. 90:8392-8396.
Perry-O'Keefe, H., X.W. Yao, J.M. Coull, M. Fuchs, *et al*. 1996. Peptide nucleic acid pre-gel hybridization: an alternative to southern hybridization. *Proc Natl Acad Sci U S A*. 93:14670-5.
Petersen, K.H., D.K. Jensen, M. Egholm, O. Buchardt, *et al*. 1976. A PNA-DNA linker synthesis of N-((4,4'-(dimethoxytrityloxy)ehtyl)-N-(thymin-1-ylacetyl)glycine. *Biorganic and Medicianl Chemistry Letters.* 5:1119-1124.
Potter, H. 1988. Electroporation in biology: Methods, applications,, and instrumentation. *Analytical Biochemistry*. 174:361-373.
Potter, H., L Weir, and P. Leder. 1984. Enhancer-dependent expression of human kappa immunoglobulin genes introduced into mouse pre-B lymphocytes by electroporation. *Proc. Natl. Acad Sci. USA.* 81:7161-7165.
Presta, L.G. 1992. Antibody engineering. *Curr Opin Biotechnol.* 3:394-8.
Prosser, J. 1993. Detecting single-base mutations. *Trends Biotechnol.* 11:238-46.
Rassoulzadegan, M., B. Binetruy, and F. Cuzin. 1982. High frequency of gene transfer after fusion between bacteria and eukaryotic cells. *Nature.* 295:257.
Reisfeld, R.A., and S. Sell. 1985. Monoclonal antibodies and cancer therapy: Proceedings of the Roche-UCLA symposium held in Park City, Utah, January 26-February 2,1985. Alan R. Liss, New York. 609 pp.
Rhodes, C.A., D.A. Pierce, I.J. Mettler, D. Mascarenhas, *et al*. 1988. Genetically transformed maize plants from protoplasts. *Science.* 240:204-207.

Ricquier, D., L Casteilla, and F. Bouillaud. 1991. Molecular studies of the uncoupling protein. *FASEB J.* 5: 2237-2242.

Riechmann, L., M. Clark, H. Waldmann, and G. Winter. 1988. Reshaping human antibodies for therapy. *Nature*. 332:323-7.

Rolfe, D.F.S., AJ. Hulbert, and M.D. Brand. 1994. Characteristics of mitochondrial proton leak and control of oxidative phosphorylation in the major oxygen-consuming tissues of the rat. *Biochim. Biophys. Acta* 1118: 405-416.

Rolfe, D.F.S., J.M.B. Newman, J.A. Buckingham, M.G. Clark, and M.D. Brand. 1999. Contribution of mitochondrial proton leak to respiration rate in working skeletal muscle and liver and to SMR. Am. *J. Physiol.* 276: C692-C699.

Rose, J.K., L. Buonocore, and M. Whitt. 1991. A new cationic liposome reagent mediating nearly quantitative transfection of animal cells. *BioTechniques.* 10:520-525.

Rossi, J.J. 1994. Practical ribozymes. Making ribozymes work in cells. *Curr Biol.* 4:469-71.

Rossiter, BJ., and C.T. Caskey. 1990. Molecular scanning methods of mutation detection. *J Biol Chem*. 265: 12753-6.

Saiki, R.K., T.L. Bugawan, G.T. Horn, K.B. Mullis, *et al*. 1986. Analysis of enzymatically amplified beta-globin and HLA-DQ alpha DNA with allele-specific oligonucleotide probes. *Nature.* 324:163-6.

Saiki, R.K., P.S: Walsh, C.H. Levenson, and H.A. Erlich. 1989. Genetic analysis of amplified DNA with immobilized sequence-specific oligonucleotide probes. *Proc Natl Acad Sci U S A.* 86:6230-4.

Saleeba, J.A., and R.G. Cotton. 1993. Chemical cleavage of mismatch to detect mutations. *Methods Enzymol*. 217:286-95.

Sambrook, J. 1989. Molecular cloning: a laboratory manual. Cold Spring Harbor Laboratory, Cold Spring Harbor.

Sandri-Goldin, R.M., A.L. Goldin, J.C. Glorioso, and M. Levine. 1981. High-frequency transfer of cloned herpes simjplex virus type I sequences to mammalian cells by protoplast fusion. *Mol. Cell. Biol.* 1:7453-752.

Sanger, F., S. Nicklen, and A.R. Coulson. 1977. DNA sequencing with chain-terminating inhibitors. *Proc Natl Acad Sci U S A.* 74:5463-7.

Saunders, J.A., B.F. Matthews, and P.D. Miller. 1989. Plant gene transfer using electrofusion and electroporation. In Electroporation and electrofusion in cell biology. E. Neumann, A.E. Sowers, and C.A. Jordan, editors. Plenum Press, New York. 343-354.

Schade, R., C. Staak, C. Hendriksen, M. Erhard, *et al*. 1996. The production of avian (egg yold) antibodies: IgY. The report and recommendations of ECVAM workshop. *Alternatives to Laboratory Animals (ATLA)*. 24:925-934.

Schaffner, W. 1980. Direct transfer of cloned genes from bacteria to mammalian cells. *Proc. Natl. Acad. Sci. USA.* 77:2163.

Schook, L.B. 1987. Monoclonal antibody production techniques and applications. Marcel Dekker, Inc., New York. 336 pp.

Schwartz, D.C., and A. Samad. 1997. Optical mapping approaches to molecular genomics. *Curr Opin Biotechnol.* 8:70-4.

Scott, J.K., and G.P. Smith. 1990. Searching for peptide ligands with an epitope library. *Science.* 249:386-90.

Selden, R.F., K. Burke-Howie, M.E. Rowe, H.M. Goodman, *et al.* 1986. Human growth hormone as a reporter gene in regulation studies employing transient gene expression. *Molecular and Cellular Biololgy.* 6:3173-3179.

Shalaby, M.R., H.M. Shepard, L. Presta, M.L. *Rodrigues, et al*. 1992. Development of humanized bispecific antibodies reactive with cytotoxic lymphocytes and tumor cells overexpressing the HER2 protooncogene. *J Exp Med*. 175:217-25.

Shigekawa, K., and WJ. Dower. 1988. Electroporation of eukaryotes and prokaryotes: A general approach to the introduction of macomolecules into cells. *BioTechniques.* 6:742-751.

Shillito, R. 1999. Methods of genetic transformations: Blectroporation and polyethylene glycol treatment. *In* Molecular improvement of cereal crop. L Vasil, editor. Kluwer, Dordrecht, The Netherlands. 9-20.

Shilo, B.Z., and R.A. Weinberg. 1981. DNA sequences homologous to vertebrate oncogenes are conserved in Drosophila melanogaster. *Proc Natl Acad Sci U S A.* 78:6789-92.

Shimkets, R.A., D.G. Lowe, J.T. Tai, P. Sehl, H. Jin, R. Yang, P.F. Predki, B.E. Rothberg, M.T. Murtha, M.E. Roth, S.G. Shenoy, A. Windemuth, J.W. Simpson, J.F. Simons, M.P. Daley, S.A. Gold, M.P. McKenna, K. Hillan, G.T. Went, and J.M. Rothberg. 1999. Gene expression analysis by transcript profiling coupled to a gene database query. *Nature Biotech*. 17(8): 798-803.

Shopes, B. 1992. A genetically engineered human IgG mutant with enhanced cytolytic activity. *J Immunol*. 148: 2918-22.

Simonsen, C.C., and A.D. Levinson. 1983. Isolation and expression of an altered mouse dihydrofolate reductase cDNA. *Proc. Natl. Acad Sci. USA.* 80:2495-2499.

Skulachev, V.P. 1996. Role of uncoupled and non-coupled oxidations in maintenance of safely low levels of oxygen and its one-electron reductants. *Quarterly Rev. Biophys.* 29(2): 169-202.

Southern, P.J., and P. Berg. 1982. Transformation of mammalian cells to antibiotic resistanced with a bacterial gene under control of the SV40 early region promoter. *J. Mol. Appl. Gen*. 1:327-341.

Sreekrishna, K., R.H. Potenz, J.A. Cruze, W.R. McCombie, *et al*. 1988. High level expression of heterologous proteins in methylotrophic yeast Pichia pastoris. *J Basic Microbiol.* 28:265-78.

Stein, C.A., and J.S. Cohen. 1988. Oligodeoxynucleotides as inhibitors of gene expression: a review. *Cancer Res.* 48:2659-68.

Stevenson, G.T., A. Pindar, and C.J. Slade. 1989. A chimeric antibody with dual Fc regions (bisFabFc) prepared by manipulations at the IgG hinge. *Anticancer Drug Des.* 3:219-30.

Stewart, E.A., K.B. McKusick, A. Aggarwal, E. Bajorek, *et al*. 1997. An STS-based radiation hybrid map of the human genome. *Genome Res.* 7:422-33.

Suresh, M.R., A.C. Cuello, and C. Milstein. 1986. Bispecific monoclonal antibodies from hybrid hybridomas. *Methods Enzymol*. 121:210-28.

Thomas, K.R., and M.R. Capecchi. 1987. Site-directed mutagenesis by gene targeting in mouse embryo-derived stem cells. *Cell.* 51:503-12.

Thompson, J.A., and e. al. 1995. Maize transformation utilizing silicon carbide whiskers: A review. *Euphytica*. 85: 75-80.

Tilburn, J., C. Scazzocchio, G.G. Taylor, J.H. Zabicky-Zissman, *et al*. 1983. Transformation by integration in Aspergillus nidulans. Gene. 26:205-21.

Tisdale, MJ. 1997. Cancer cachexia: metabolic alterations and clinical manifestations. *Nutrition.* 13:1-7.

Touraev, A., and e. al. 1997. Plant male germ line transformation. *Plant J.* 12:949-956.

Traunecker, A., F. Oliveri, and K. Karjalainen. 1991. Myeloma based expression system for production of large mammalian proteins. *Trends Biotechnol.* 9:109-13.

Trick, H.N., and e. al. 1997. Recent advances in soybean transformation. *Plant Tissue Cult. Biotechnol*. 3:9-26.

Tuerk, C., and L. Gold. 1990. Systematic evolution of ligands by exponential enrichment: RNA ligands to bacteriophage T4 DNA polymerase. *Science.* 249:505-10.

Turner, D.L., E.Y. Snyder, and C.L. Cepko. 1990. Lineage-independent determinationh of cell type in the embryonic mouse retina. *Neuron.* 4:833-845.

Tutt, A., G.T. Stevenson, and MJ. Glennie. 1991. Trispecific F(ab')3 derivatives that use cooperative signaling via the TCR/CD3 complex and CD2 to activate and redirect resting cytotoxic T cells. *J Immunol*. 147:60-9.

van der Krol, A.R., J.N. Mol, and A.R. Stuitje. 1988a. Modulation of eukaryotic gene expression by complementary RNA or DNA sequences. *Biotechniques.* 6:958-76.

van der Krol, A.R., J.N. Mol, and A.R. Stuitje. 1988b. Modulation of eukaryotic gene expression by complementary RNA or DNA sequences. *Biotechniques.* 6:958-76.

Verhoeyen, M., C. Milstein, and G. Winter. 1988. Reshaping human antibodies: grafting an antilysozyme activity. *Science.* 239:1534-6.

Vidal-Puig, A.J., D. Grujic, C.Y. Zhang, T. Hagen, O. Boss, Y. Ido, A. Szczepanik, J. Wade, V. Mootha, R. Cortright, D.M. Muoio, and B.B. Lowell. 2000. Energy metabolism uncoupling protein 3 gene knockout mice. *J. Biol. Chem*. 275(21)16258-66.

Vitetta, E.S., R.J. Fulton, R.D. May, M. Till, *et al*. 1987. Redesigning nature's poisons to create anti-tumor reagents. *Science.* 238:1098-104.

Wells, J.A., M. Vasser, and D.B. Powers. 1985. Cassette mutagenesis: an efficient method for generation of multiple mutations at defined sites. *Gene.* 34:315-23.

Whitt, M.A., L. Buonocore, J.K. Rose, V. Ciccarone, *et al*. 1990. TransfectACE reagent promotes transient transfection frequencies greater than 90%. *Focus.* 13:8-12.

Wigler, M., A. Pellicer, S. Silversttein, and R. Axel. 1978. Biochemical transfer of single-copy eucaryotic genes using total cellular DNA as donor. *Cell*. 14:725.

Williams, D.A., I.R. Lemischka, D.G. Nathan, and R.C. Mulligan. 1984. Introduction of a new genetic material into pluripotent haematopoietic stem cells of the mouse. *Nature.* 310:476-480.

Wilmut, L, A.E. Schnieke, J. McWhir, AJ. Kind, *et al*. 1997. Viable offspring derived from fetal and adult mammalian cells. *Nature*. 385:810-3.

Wolff, E.A., GJ. Schreiber, W.L. Cosand, and H.V. Raff. 1993. Monoclonal antibody homodimers: enhanced antitumor activity in nude mice. *Cancer Res.* 53:2560-5.

Wong, T.K., and E. Neumann. 1982. Electric field mediated gene transfer. *Biochemical and Biophysical Research Communications*. 107:584-587.

Wyborski, D.L., L.C. DuCoeur, and J.M. Short. 1996. Parameters affecting the use of the lac repressor system in eukaryotic cells and transgenic animals. *Environ Mol Mutagen.* 28:447-58.

Wyborski, D.L., and J.M. Short. 1991. Analysis of inducers of the E.coli lac repressor system in mammalian cells and whole animals. *Nucleic Acids Res.* 19:4647-53.

Yelton, M.M., J.E. Hamer, and W.E. Timberlake. 1984. Transformation of Aspergillus nidulans by using a trpC plasmid. *Proc Natl Acad Sci U S A*. 81:1470-4.

Yu, X.X., J.L. Barger, B.B. Boyer, M.D. Brand, G. Pan, and S.H. Adams. 2000a. Impact of endotoxin on UCP homolog mRNA abundance, thermoregulation, and mitochondrial proton leak kinetics. *Am. J. Physiol*., 279(2): E433-46.

Yu, X.X., W. Mao, A. Zhong, P. Schow, J. Brush, S.W. Sherwood, S.H. Adams, and G. Pan. 2000b. Characterization of novel UCP5/BMCP1 isoforms and differential regulation of UCP4 and UCP5 expression through dietary or temperature manipulation. *FASEB J* 14(11):1611-8.

Zervos, A.S., J. Gyuris, and R. Brent. 1993. Mxi1, a protein that specifically interacts with Max to bind Myc-Max recognition sites. *Cell.* 72:223-32.

Zhou, G., and e. al. 1983. Introduction of exogenous DNA into cotton embryos. *Methods Enzymol*. 101:433-481.

Zoller, M.J., and M. Smith. 1987. Oligonucleotide-directed mutagenesis: a simple method using two oligonucleotide primers and a single-stranded DNA template. *Methods Enzymol*. 154:329-50.

Zon, G. 1988. Oligonucleotide analogues as potential chemotherapeutic agents. *Pharm Res*. 5:539-49.

Zuckermann, R.N., E.J. Martin, D.C. Spellmeyer, G.B. Stauber, *et al*. 1994. Discovery of nanomolar ligands for 7-transmembrane G-protein-coupled receptors from a diverse N-(substituted)glycine peptoid library. *J Med Chem*. 37:2678-85.

## Claims

1. An isolated polynucleotide comprising:-

   (a) a nucleic acid sequence having at least 80% sequence identity to a sequence of SEQ ID NO:1, which nucleic acid sequence encodes a polypeptide, and
   (b) a nucleic acid sequence encoding a heterologous polypeptide expressed at the C-terminus of the polypeptide of (a);

   wherein said isolated polynucleotide encodes a polypeptide having uncoupling activity; or a complement of said isolated polynucleotide.

2. The polynucleotide of Claim 1, wherein the nucleic acid sequence of (a) is SEQ ID NO:1.

3. The polynucleotide of Claim 1, wherein the nucleic acid sequence of (a) has a sequence consisting of nucleotide 14 to nucleotide 1093 of SEQ ID NO:1.

4. The polynucleotide of Claim 1, wherein the nucleic acid sequence of (a) encodes a polypeptide comprising an amino acid sequence having at least 80% sequence identity to a sequence of SEQ ID NO:3, wherein the polypeptide comprises at least one biological activity of the polypeptide of SEQ ID NO:3.

5. The polypeptide of Claim 4, wherein the biological activity comprises mitochondrial localization.

6. A vector comprising the polynucleotide of any of Claims 1-5.

7. The vector of Claim 6, wherein the polynucleotide is operably linked to a promoter.

8. An isolated host cell comprising the vector of Claim 6 or Claim 7.

9. The cell of Claim 8, wherein the cell is mammalian.

10. An isolated polypeptide comprising:-

    (a) an amino acid sequence having at least 80% sequence identity to a sequence of SEQ ID NO:3, and
    (b) a heterologous polypeptide fused to the carboxy-terminus of the amino acid sequence of (a);

    wherein the polypeptide has uncoupling activity.

11. The polypeptide of Claim 10, wherein the polypeptide comprises a biological activity of the polypeptide of SEQ ID NO:3.

12. The polypeptide of Claim 10 or Claim 11, wherein the amino acid sequence has at least 99% sequence identity to the sequence of SEQ ID NO:3.

13. The polypeptide of any of Claims 10-12, wherein the heterologous fusion polypeptide functions as an uncoupling protein.

14. The polypeptide of Claim 13, wherein the heterologous polypeptide is negatively charged.

**15.** The polypeptide of any of Claims 10-14, wherein the heterologous polypeptide is FLAG-tag, β-glucoronidase, green fluorescent protein, blue fluorescent protein, chloramphenicol acetyl transferase, β-galactosidase, secreted alkaline phosphatase or Tat, or a negatively charged fragment thereof.

**16.** The polypeptide of Claims 10-15, wherein the heterologous polypeptide comprises an amino acid sequence of SEQ ID NO:17.

**17.** An isolated cell expressing the polypeptide of any of Claims 10-16.

**18.** The cell of Claim 17, wherein the cell is a 293 cell.

**19.** An in vitro method for determining whether a compound up-regulates or down-regulates expression of a gene encoding the polypeptide of any of Claims 10 to 16 in a cell or cell-free extract, comprising:-

(a) contacting the cell with the compound, wherein the cell expresses the polypeptide of any of Claims 10-16; and
(b) detecting expression of the gene.

**20.** The method of Claim 19, wherein detecting expression of the gene comprises detecting mRNA encoding the polypeptide of any of Claims 10-16.

**21.** The method of Claim 19, wherein detecting expression of the gene comprises detecting the polypeptide.

**22.** A transgenic non-human animal, comprising a transgene encoding the polypeptide of any of Claims 10-16.

**23.** A transgenic non-human animal, comprising a transgene comprising:-

(a) a nucleic acid sequence having at least 80% sequence identity to a sequence of SEQ ID NO:1, which nucleic acid sequence encodes a polypeptide, wherein the polypeptide has uncoupling activity; and
(b) a nucleic acid sequence encoding a heterologous polypeptide expressed at the C-terminus of the polypeptide of (a).

**24.** The transgenic non-human animal of Claim 22 or 23, wherein the non-human animal is a mouse.

**25.** An *in vitro* method of measuring agonist or antagonist activity of a compound on an activity of the polypeptide of any of Claims 10-16, comprising:

(a) contacting a composition of the polypeptide of any of Claims 10-16 with the compound; and
(b) determining a change in uncoupling activity.

**26.** The method of Claim 25 wherein the composition is a cell or cell-free extract.

**27.** An *in vitro* method for detecting the presence or absence of $CGI\text{-}69_L$ in a biological sample, comprising:

(a) contacting the biological sample with a $CGI\text{-}69_L$ primer or probe capable of detecting a polynucleotide encoding a polypeptide comprising an amino acid sequence having at least 98% sequence identity to SEQ ID NO:3, wherein said primer or probe is capable of detecting $CGI\text{-}69_L$ and is not capable of detecting CGI-69; and
(b) comparing the presence or absence of the polynucleotide with a control biological sample, wherein a change in amount of polynucleotide is indicative of metabolic disease or risk of metabolic disease.

**28.** The method of Claim 27, wherein the biological sample is blood or adipose tissue.

**29.** The method of Claim 27, wherein the primer or probe that detects the polynucleotide encoding the polypeptide is a labelled nucleic acid probe that can hybridize to the polynucleotide or its complement under stringent conditions.

**30.** The method of Claim 27, wherein the polypeptide comprises a sequence of SEQ ID NO:3.

31. The method of Claim 27, wherein the probe or primer that detects the polynucleotide is a primer that hybridizes to the polynucleotide or its complement under stringent conditions.

32. The method of Claim 27, wherein the polypeptide is localized in the mitochondria.

33. The method of Claim 29 or 31, wherein the probe or primer comprises a nucleic acid sequence of SEQ ID NO:11, SEQ ID NO:12 or SEQ ID NO:13.

## Patentansprüche

1. Isoliertes Polynucleotid, umfassend:

   (a) eine Nucleinsäuresequenz mit mindestens 80 %-iger Sequenzidentität zu einer Sequenz der SEQ ID NR: 1, welche Nucleinsäuresequenz ein Polypeptid kodiert, und
   (b) eine Nucleinsäuresequenz, kodierend ein heterologes Polypeptid, exprimiert am C-Terminus des Polypeptids aus (a);

   worin das isolierte Polynucleotid ein Polypeptid mit einer entkoppelnden Aktivität kodiert, oder ein Komplement des isolierten Polynucleotids.

2. Polynucleotid nach Anspruch 1, worin die Nucleinsäuresequenz aus (a) die SEQ ID NR:1 ist.

3. Polynucleotid nach Anspruch 1, worin die Nucleinsäuresequenz aus (a) eine Sequenz aufweist, die aus dem Nucleotid 14 bis Nucleotid 1093 der SEQ ID NR:1 besteht.

4. Polynucleotid nach Anspruch 1, worin die Nucleinsäuresequenz aus (a) ein Polypeptid kodiert, umfassend eine Aminosäuresequenz mit mindestens 80 % Sequenzidentität zu einer Sequenz der SEQ ID NR:3, worin das Polypeptid mindestens eine biologische Aktivität des Polypeptids der SEQ ID NR:3 umfasst.

5. Polynucleotid nach Anspruch 4, worin die biologische Aktivität die Lokalisation in Mitochondrien umfasst.

6. Vektor, umfassend das Polynucleotid nach einem der Ansprüche 1 bis 5.

7. Vektor nach Anspruch 6, worin das Polynucleotid operativ mit einem Promotor verbunden ist.

8. Isolierte Wirtszelle, umfassend den Vektor nach Anspruch 6 oder Anspruch 7.

9. Zelle nach Anspruch 8, worin die Zelle eine Säugerzelle ist.

10. Isoliertes Polypeptid, umfassend:

    (a) eine Aminosäuresequenz mit mindestens 80 %-iger Sequenzidentität zu einer Sequenz nach SEQ ID NR: 3, und
    (b) ein heterologes Polypeptid, fusioniert an den Carboxy-Terminus der Aminosäuresequenz aus (a);

    worin das Polypeptid eine entkoppelnde Aktivität aufweist.

11. Polypeptid nach Anspruch 10, worin das Polypeptid eine biologische Aktivität des Polypeptids aus SEQ ID NR:3 umfasst.

12. Polypeptid nach Anspruch 10 oder Anspruch 11, worin die Aminosäuresequenz eine mindestens 99 %-ige Sequenzidentität zur Sequenz der SEQ ID NR:3 aufweist.

13. Polypeptid nach einem der Ansprüche 10 bis 12, worin das heterologe Fusionspolypeptid als ein entkoppelndes Protein fungiert.

14. Polypeptid nach Anspruch 13, worin das heterologe Polypeptid negativ geladen ist.

**15.** Polypeptid nach einem der Ansprüche 10 bis 14, worin das heterologe Polypeptid FLAG-tag, β-Glucuronidase, das grün fluoreszierende Protein, das blau fluoreszierende Protein, die Chloramphenicol-Acetyl-Transferase, die β-Galactosidase, eine sekretierte alkaline Phosphatase oder Tat oder ein negativ geladenes Fragment davon darstellt.

**16.** Polypeptid nach den Ansprüchen 10 bis 15, worin das heterologe Polypeptid eine Aminosäuresequenz der SEQ ID NR:17 umfasst.

**17.** Isolierte Zelle, welche das Polypeptid nach einem der Ansprüche 10-16 exprimiert.

**18.** Zelle nach Anspruch 17, worin die Zelle eine 293-Zelle darstellt.

**19.** In-vitro-Verfahren zum Bestimmen, ob eine Verbindung die Expression eines Genes auf- oder abreguliert, welches das Polypeptid nach einem der Ansprüche 10 bis 16 kodiert, in einer Zelle oder zellfreiem Extrakt, umfassend:

(a) das In-Kontakt-bringen der Zelle mit der Verbindung, wobei die Zelle das Polypeptid nach einem der Ansprüche 10-16 exprimiert; und
(b) Detektieren der Expression des Gens.

**20.** Verfahren nach Anspruch 19, worin das Detektieren der Expression des Gens das Detektieren von mRNA umfasst, welche das Polypeptid nach einem der Ansprüche 10-16 kodiert.

**21.** Verfahren nach Anspruch 19, worin das Detektieren der Expression des Gens das Detektieren des Polypeptids umfasst.

**22.** Transgenes, nicht menschliches Tier, umfassend ein Transgen, kodierend das Polypeptid nach einem der Ansprüche 10-16.

**23.** Transgenes, nicht menschliches Tier, umfassend ein Transgen, umfassend:

(a) eine Nucleinsäuresequenz mit mindestens 80 %-iger Sequenzidentität zu einer Sequenz der SEQ ID NR: 1, welche Nucleinsäuresequenz ein Polypeptid kodiert, worin das Polypeptid eine entkoppelnde Aktivität aufweist; und
(b) eine Nucleinsäuresequenz, kodierend ein heterologes Polypeptid, exprimiert am C-Terminus des Polypeptids aus (a).

**24.** Transgenes, nicht menschliches Tier nach Anspruch 22 oder 23, worin das nicht menschliche Tier eine Maus ist.

**25.** In-vitro-Verfahren zum Messen der Agonist- oder Antagonistaktivität einer Verbindung auf eine Aktivität des Polypeptids nach einem der Ansprüche 10-16, umfassend

(a) das In-Kontakt-bringen einer Zusammensetzung des Polypeptids nach einem der Ansprüche 10-16 mit der Verbindung; und
(b) das Bestimmen der Änderung hinsichtlich der Entkopplungsaktivität.

**26.** Verfahren nach Anspruch 25, worin die Zusammensetzung eine Zelle oder ein zellfreier Extrakt ist.

**27.** *In-vitro*-Verfahren zum Detektieren der Anwesenheit oder Abwesenheit von CGI-$69_L$ in einer biologischen Probe, umfassend:

(a) das In-Kontakt-bringen der biologischen Probe mit einem CGI-$69_L$-Primer oder einer Sonde, der oder die ein Polynucleotid kodieren kann, kodierend ein Polypeptid, umfassend eine Aminosäuresequenz mit mindestens 98 %-iger Sequenzidentität zur SEQ ID NR:3, worin der Primer oder die Sonde CGI-$69_L$ detektieren kann und CGI-69 nicht detektieren kann; und
(b) das Vergleichen der Anwesenheit oder Abwesenheit des Polynucleotids mit einer biologischen Kontrollprobe, worin eine Änderung der Menge an Polynucleotid eine metabolische Erkrankung oder das Risiko einer metabolischen Erkrankung anzeigt.

**28.** Verfahren nach Anspruch 27, worin die biologische Probe Blut oder Fettgewebe ist.

**29.** Verfahren nach Anspruch 27, worin der Primer oder die Sonde, der oder die das Polypeptid kodierende Polynucleotid detektiert, eine markierte Nucleinsäuresonde ist, die mit dem Polynucleotid oder seinem Komplement unter Stringenzbedingungen hybridisieren kann.

**30.** Verfahren nach Anspruch 27, worin das Polypeptid eine Sequenz der SEQ ID NR:3 umfasst.

**31.** Verfahren nach Anspruch 27, worin die Sonde oder der Primer, die bzw. der das Polynucleotid detektiert, ein Primer ist, der mit dem Polynucleotid oder seinem Komplement unter Stringenzbedingungen hybridisiert.

**32.** Verfahren nach Anspruch 27, worin das Polypeptid in den Mitochondrien lokalisiert ist.

**33.** Verfahren nach Anspruch 29 oder 31, worin die Sonde oder der Primer eine Nucleinsäuresequenz der SEQ ID NR:11, SEQ ID NR:12 oder SEQ ID NR:13 umfasst.

**Revendications**

**1.** Polynucléotide isolé comprenant :

(a) une séquence d'acide nucléique ayant une identité de séquence d'au moins 80 % avec une séquence de SEQ ID N° : 1, laquelle séquence d'acide nucléique code pour un polypeptide, et
(b) une séquence d'acide nucléique codant pour un polypeptide hétérologue exprimé à l'extrémité C-terminale du polypeptide de (a) ;

dans lequel ledit polynucléotide isolé code pour un polypeptide ayant une activité de découplage ; ou un complément dudit polynucléotide isolé.

**2.** Polynucléotide selon la revendication 1, dans lequel la séquence d'acide nucléique de (a) est SEQ ID N° : 1.

**3.** Polynucléotide selon la revendication 1, dans lequel la séquence d'acide nucléique de (a) a une séquence constituée par le nucléotide 14 jusqu'au nucléotide 1093 de SEQ ID N° : 1.

**4.** Polynucléotide selon la revendication 1, dans lequel la séquence d'acide nucléique de (a) code pour un polypeptide comprenant une séquence d'acides aminés ayant une identité de séquence d'au moins 80 % avec une séquence de SEQ ID N° : 3, dans lequel le polypeptide comprend au moins une activité biologique du polypeptide de SEQ ID N° : 3.

**5.** Polypeptide selon la revendication 4, dans lequel l'activité biologique comprend la localisation mitochondriale.

**6.** Vecteur comprenant le polynucléotide selon l'une quelconque des revendications 1 à 5.

**7.** Vecteur selon la revendication 6, dans lequel le polynucléotide est lié de manière opérationnelle à un promoteur.

**8.** Cellule hôte isolée comprenant le vecteur selon la revendication 6 ou la revendication 7.

**9.** Cellule selon la revendication 8, dans laquelle la cellule est une cellule mammifère.

**10.** Polypeptide isolé comprenant :

(a) une séquence d'acides aminés ayant une identité de séquence d'au moins 80 % avec une séquence de SEQ ID N° : 3, et
(b) un polypeptide hétérologue fusionné à l'extrémité carboxy terminale de la séquence d'acides aminés de (a) ;

dans lequel le polypeptide a une activité de découplage.

**11.** Polypeptide selon la revendication 10, dans lequel le polypeptide comprend une activité biologique du polypeptide de SEQ ID N° : 3.

**12.** Polypeptide selon la revendication 10 ou la revendication 11, dans lequel la séquence d'acides aminés présente une identité de séquence d'au moins 99 % avec la séquence de SEQ ID N° : 3.

**13.** Polypeptide selon l'une quelconque des revendications 10 à 12, dans lequel le polypeptide de fusion hétérologue fonctionne comme une protéine de découplage.

**14.** Polypeptide selon la revendication 13, dans lequel le polypeptide hétérologue a une charge négative.

**15.** Polypeptide selon l'une quelconque des revendications 10 à 14, dans lequel le polypeptide hétérologue est FLAG-tag, la β-glucoronidase, la protéine à fluorescence verte, la protéine à fluorescence bleue, la chloramphénicol acétyl transférase, la β-galactosidase, la phosphatase alcaline sécrétée ou Tat, ou un fragment à charge négative de ceux-ci.

**16.** Polypeptide selon les revendications 10 à 15, dans lequel le polypeptide hétérologue comprend une séquence d'acides aminés de SEQ ID N° : 17.

**17.** Cellule isolée exprimant le polypeptide selon l'une quelconque des revendications 10 à 16.

**18.** Cellule selon la revendication 17, dans laquelle la cellule est une cellule 293.

**19.** Procédé *in vitro* destiné à déterminer si un composé régule à la hausse ou à la baisse l'expression d'un gène codant pour le polypeptide selon l'une quelconque des revendications 10 à 16 dans un extrait cellulaire ou acellulaire comprenant :

(a) la mise en contact de la cellule et du composé, dans lequel la cellule exprime le polypeptide selon l'une quelconque des revendications 10 à 16 ; et
(b) la détection de l'expression du gène.

**20.** Procédé selon la revendication 19, dans lequel la détection de l'expression du gène comprend la détection de l'ARNm codant pour le polypeptide selon l'une quelconque des revendications 10 à 16.

**21.** Procédé selon la revendication 19, dans lequel la détection de l'expression du gène comprend la détection du polypeptide.

**22.** Animal transgénique non humain, comprenant un transgène codant pour le polypeptide selon l'une quelconque des revendications 10 à 16.

**23.** Animal transgénique non humain, comprenant un transgène comprenant :

(a) une séquence d'acide nucléique ayant une identité de séquence d'au moins 80 % avec une séquence de SEQ ID N° : 1, laquelle séquence d'acide nucléique code pour un polypeptide, dans lequel le polypeptide a une activité de découplage ; et
(b) une séquence d'acide nucléique codant pour un polypeptide hétérologue exprimé à l'extrémité C-terminale du polypeptide de (a).

**24.** Animal transgénique non humain selon la revendication 22 ou 23, dans lequel l'animal non humain est une souris.

**25.** Procédé *in vitro* de mesure de l'activité agoniste ou antagoniste d'un composé sur une activité du polypeptide selon l'une quelconque des revendications 10 à 16, comprenant :

(a) la mise en contact d'une composition du polypeptide selon l'une quelconque des revendications 10 à 16 avec le composé ; et
(b) la détermination d'une modification de l'activité de découplage.

**26.** Procédé selon la revendication 25, dans lequel la composition est un extrait cellulaire ou acellulaire.

**27.** Procédé *in vitro* de détection de la présence ou de l'absence de CGI-$69_L$ dans un échantillon biologique, comprenant :

(a) la mise en contact de l'échantillon biologique avec une amorce ou une sonde de CGI-$69_L$ capable de détecter un polynucléotide codant pour un polypeptide comprenant une séquence d'acides aminés ayant une identité de séquence d'au moins 98 % avec SEQ ID N° : 3, dans lequel ladite amorce ou sonde est capable de détecter CGI-$69_L$ et n'est pas capable de détecter CGI-69 ; et
(b) la comparaison de la présence ou de l'absence du polynucléotide avec un échantillon biologique de contrôle, dans lequel une modification de la quantité de polynucléotide est un indicateur d'une maladie métabolique ou d'un risque de maladie métabolique.

**28.** Procédé selon la revendication 27, dans lequel l'échantillon biologique est du sang ou du tissu adipeux.

**29.** Procédé selon la revendication 27, dans lequel l'amorce ou la sonde qui détecte le polynucléotide codant pour le polypeptide est une sonde d'acide nucléique marquée qui peut s'hybrider au polynucléotide ou à son complément dans des conditions stringentes.

**30.** Procédé selon la revendication 27, dans lequel le polypeptide comprend une séquence de SEQ ID N° : 3.

**31.** Procédé selon la revendication 27, dans lequel la sonde ou l'amorce qui détecte le polynucléotide est une amorce qui s'hybride au polynucléotide ou à son complément dans des conditions stringentes.

**32.** Procédé selon la revendication 27, dans lequel le polypeptide est localisé dans les mitochondries.

**33.** Procédé selon la revendication 29 ou 31, dans lequel la sonde ou l'amorce comprend une séquence d'acide nucléique de SEQ ID N° : 11, SEQ ID N° : 12 ou SEQ ID N° : 13.

```
Human_CGI-69    MADQDPAGISPLQQMVASGTGAVVTSLFMTPLDVVKVRLQSQRPSMASELMPSSRLWSLS
Human_CGI-69L   MADQDPAGISPLQQMVASGTGAVVTSLFMTPLDVVKVRLQSQRPSMASELMPSSRLWSLS
Mouse_CGI-69    --MIRTLGALAVQQMVASGAGAVVTSLFMTPLDVVKVRLQSQRPSATSELTTPSRFWSLS

Human_CGI-69    YTKW--------KCLLYCNGVLEPLYLCPNGARCATWFQDPTRFTGTMDAFVKIVRHEGT
Human_CGI-69L   YTKLPSSLQSTGKCLLYCNGVLEPLYLCPNGARCATWFQDPTRFTGTMDAFVKIVRHEGT
Mouse_CGI-69    YTKSSSALQSPGKCLLYCNGVLEPLYLCPNGTRCATWFQDPTRFTGTIDAFVKIVRHEGT

Human_CGI-69    RTLWSGLPATLVMTVPATAIYFTAYDQLKAFLCGRALTSDLYAPMVAGALARLGTVTVIS
Human_CGI-69L   RTLWSGLPATLVMTVPATAIYFTAYDQLKAFLCGRALTSDLYAPMVAGALARLGTVTVIS
Mouse_CGI-69    RTLWSGLPATLVMTVPATAIYFTAYDQLKAFLCGQSLTSDLYAPMVAGALARMGTVTVVS

Human_CGI-69    PLELMRTKLQAQHVSYRELGACVRTAVAQGGWRSLWLGWGPTALRDVPFSALYWFNYELV
Human_CGI-69L   PLELMRTKLQAQHVSYRELGACVRTAVAQGGWRSLWLGWGPTALRDVPFSALYWFNYELV
Mouse_CGI-69    PLELVRTKLQAQHVSYRELASSVQAAVTQGGWRSLWLGWGPTALRDVPFSALYWFNYELV

Human_CGI-69    KSWLNGFRPKDQTSVGMSFVAGGISGTVAAVLTLPFDVVKTQRQVALGAMEAVRVNPLHV
Human_CGI-69L   KSWLNGLRPKDQTSVGMSFVAGGISGTVAAVLTLPFDVVKTQRQVALGAMEAVRVNPLHV
Mouse_CGI-69    KSWLSGLRPKDQTSVGISFVAGGISGMVAATLTLPFDVVKTQRQMSLGAVEAVRVKPPRV

Human_CGI-69    DSTWLLLRRIRAESGTKGLFAGFLPRIIKAAPSCAIMISTYEFGKSFFQRLNQDRLLGG
Human_CGI-69L   DSTWLLLRRIRAESGTKGLFAGFLPRIIKAAPSCAIMISTYEFGKSFFQRLNQDRLLGG
Mouse_CGI-69    DSTWLLLRRIRAESGTRGLFAGFLPRIIKAAPSCAIMISTYEFGKSFFQRLNQEQPLGR
```

FIG. 1